(19) Europäisches Patentamt
European Patent Office
Office européen des brevets

(11) EP 2 625 190 B1

(12) # EUROPÄISCHE PATENTSCHRIFT

(45) Veröffentlichungstag und Bekanntmachung des Hinweises auf die Patenterteilung:
**14.07.2021 Patentblatt 2021/28**

(21) Anmeldenummer: **11819158.4**

(22) Anmeldetag: **06.10.2011**

(51) Int Cl.:
*C07J 41/00* (2006.01) *A61K 31/56* (2006.01)
*A61P 31/04* (2006.01) *A61P 31/10* (2006.01)
*A61P 31/12* (2006.01) *A61P 33/00* (2006.01)

(86) Internationale Anmeldenummer:
**PCT/DE2011/001829**

(87) Internationale Veröffentlichungsnummer:
**WO 2012/051988 (26.04.2012 Gazette 2012/17)**

(54) **DERIVATE VON STEROIDBENZYLAMINEN MIT ANTIPARASITÄRER, ANTIBAKTERIELLER, ANTIMYKOTISCHER UND/ODER ANTIVIRALER WIRKUNG**

DERIVATIVES OF STEROID BENZYLAMINES, HAVING AN ANTIPARASITIC, ANTIBACTERIAL, ANTIMYCOTIC AND/OR ANTIVIRAL ACTION

DÉRIVÉS DE BENZYLAMINES STÉROÏDES À ACTION ANTIPARASITAIRE, ANTIBACTÉRIENNE, ANTIFONGIQUE ET/OU ANTIVIRALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.10.2010 DE 102010047714**

(43) Veröffentlichungstag der Anmeldung:
**14.08.2013 Patentblatt 2013/33**

(73) Patentinhaber: **Justus-Liebig-Universität Giessen
35390 Giessen (DE)**

(72) Erfinder:
- **BECKER, Katja
35435 Wettenberg (DE)**
- **KRIEG, Reimar
07745 Jena (DE)**
- **SCHÖNECKER, Bruno
07743 Jena (DE)**

(74) Vertreter: **Arth, Hans-Lothar
ABK Patent Attorneys
Jasminweg 9
14052 Berlin (DE)**

(56) Entgegenhaltungen:
**WO-A2-87/02367 DE-A1- 19 633 206**

- **A. M. BELLINI ET AL: "Antimicrobial Activity of Basic Cholane Derivatives Part IX", ARCHIV DER PHARMAZIE, Bd. 323, Nr. 4, 1. Januar 1990 (1990-01-01), Seiten 201-205, XP55024553, ISSN: 0365-6233, DOI: 10.1002/ardp.19903230404**
- **A. M. BELLINI ET AL: "Antimicrobial Activity of Basic Cholane Derivatives, VIII", ARCHIV DER PHARMAZIE, Bd. 322, Nr. 12, 1. Januar 1989 (1989-01-01), Seiten 879-883, XP55024550, ISSN: 0365-6233, DOI: 10.1002/ardp.19893221208**
- **SALMI CHANAZ ET AL: "Antimicrobial activities of 3-amino- and polyaminosterol analogues of squalamine and trodusquemine", JOURNAL OF ENZYME INHIBITION AND MEDICINAL CHEMISTRY, TAYLOR, READING, GB, Bd. 23, Nr. 6, 1. Dezember 2008 (2008-12-01), Seiten 860-865, XP009130732, ISSN: 1475-6366, DOI: 10.1080/14756360701809910**
- **DUBS M ET AL: "Reactions of the four diastereomeric 16-amino-17-hydroxy-3-methoxy-estra-1,3,5(10)-trienes with aromatic ortho-hydroxy and heteroaromatic alpha-aldehydes and with 1,3-dicarbonyl compounds-molecular structures of condensation products and of copper(II) complexes", STEROIDS, ELSEVIER SCIENCE PUBLISHERS, NEW YORK, NY, US, Bd. 65, Nr. 6, 1. Juni 2000 (2000-06-01), Seiten 305-318, XP004198895, ISSN: 0039-128X, DOI: 10.1016/S0039-128X(00)00092-1**
- **None**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 625 190 B1

## Beschreibung

Die Erfindung betrifft von Steroiden abgeleitete Verbindungen der allgemeinen Formel (I); ihre Verwendung in der Medizin und zur Prophylaxe und / oder zur Behandlung von Infektionserkrankungen. Des Weiteren sind pharmazeutische Zusammensetzungen enthaltend mindestens eine erfindungsgemäße Verbindung beschrieben. Ein weiterer Teil der Erfindung betrifft die Synthese der Verbindungen der Formel (I).

Eine Infektionskrankheit, umgangssprachlich "Infekt" oder "ansteckende Krankheit", ist eine durch Erreger hervorgerufene Erkrankung. Sie ist aber nicht einer Infektion gleichzusetzen, da nicht jede Infektion notwendigerweise zu einer Erkrankung führt. Infektionskrankheiten zeigen ein breites Spektrum an zeitlichen Verläufen und Symptomen. Diese sind oftmals für den Erreger spezifisch. Sie können akut in wenigen Tagen entstehen oder sich über Wochen, Monate, manchmal Jahre hinweg langsam entwickeln. Es gibt lokal beschränkte und auch systemische Infektionskrankheiten. Ausschlaggebend für den Verlauf und die Prognose einer Infektionskrankheit ist unter anderem die Fähigkeit des Immunsystems, den Erreger zu eliminieren. Die Gefährlichkeit einer Infektionskrankheit ist aber auch abhängig von der so genannten Virulenz des Erregers. Ob und in welcher Schwere eine Infektion auch zu einer Infektionskrankheit führt, hängt neben vielen anderen Faktoren zudem von der Zahl der aufgenommenen Erreger (minimale Infektionsdosis) ab.

Das Immunsystem dient als biologisches Abwehrsystem das Gewebeschädigungen durch Krankheitserreger verhindert. Es zerstört fehlerhaft gewordene körpereigene Zellen und ist außerdem in der Lage,. in den Körper eingedrungene Mikroorganismen, wie Bakterien, Viren und andere Parasiten oder fremde Substanzen abzutöten und zu entfernen. Das Immunsystem ist ein komplexes Netzwerk aus verschiedenen Organen, Zelltypen und Molekülen. Trotz großer Impfprogramme bleiben virale Infektionen wie die Grippe, aber auch Infektionen hervorgerufen durch Human Immunodeficiency Virus ("HIV")-, Human Papilloma Virus (HPV Typ 16 oder 18), humane Cytomegalovirus ("HCMV") oder die menschlichen Hepatitis B oder C-Viren ("HBV", Typ B; "HCV", Typ C), eine häufige Ursache von lebensbedrohlichen Erkrankungen in der ganzen Welt und gelten insbesondere bei Menschen mit einer Immunschwäche als bedeutende Ursache von Krankheit und Tod. Obwohl gezeigt werden konnte, dass eine antivirale Chemotherapie mit Verbindungen wie Amantadin und Rimantadin die Dauer der Symptome klinischer Infektionen (z.B. Influenza-Infektion) vermindern kann, wurden schwere Nebenwirkungen und die rasche Entstehung von resistenten Varianten beschrieben. Momentan werden neue Klassen von antiviralen Wirkstoffen entwickelt, welche insbesondere auf virale Proteine wie z.B. die Influenza-Neuraminidase abzielen. Allerdings stellt die Fähigkeit der Viren, die Zielproteine rasch zu mutieren, ein Hindernis dar für eine effektive Behandlung mit Wirkstoffen, die selektiv die Funktion von spezifischen, viralen Polypeptiden hemmen. Somit besteht Bedarf für neue therapeutische Strategien zur Vorbeugung und Behandlung von viralen Infektionen.

Zusätzlich gibt es einen Bedarf an neuen Therapien für die Vorbeugung und Behandlung von bakteriellen Infektionen, vor allem bakterielle Infektionen, welche durch mehrere resistente Bakterien verursacht werden. Derzeit werden bakterielle Infektionen mit verschiedenen Antibiotika behandelt. Obwohl Antibiotika bei der Behandlung von verschiedenen bakteriellen Infektionen sehr wirksam sein können, gibt es eine Reihe von Einschränkungen, die Wirksamkeit und Sicherheit von Antibiotika betreffend. Zum Beispiel haben einige Personen eine allergische Reaktion auf bestimmte Antibiotika und andere Personen leiden an schweren Nebenwirkungen. Darüber hinaus entwickeln sich unter der Verwendung von Antibiotika zur Behandlung von bakteriellen Infektionen zunehmend Antibiotikaresistente Bakterienstämme. Daher besteht auch hier weiterhin ein Bedarf an neu entwickelten Substanzen zur Behandlung von bakteriellen Erkrankungen.

Parasitismus ist eine Art symbiotische Beziehung zwischen Organismen verschiedener Arten, bei denen ein Organismus, der Parasit, in oder auf einem Wirtsorganismus lebt und seine Nahrung auf Kosten des Wirtes erhält. Im Allgemeinen sind die Parasiten sehr viel kleiner als Ihre Wirte, zeigen einen hohen Grad an Spezialisierung für Ihren Wirt und dessen Besiedelung und reproduzieren sich schneller und in größerer Zahl als ihre Wirte. Klassische Beispiele des Parasitismus sind Wechselwirkungen zwischen Wirbeltieren als Wirten und diversen Parasiten wie einzellige Protozoen, vielzelligen Würmern (Helminthen) und Arthropoda. Parasitäre Infektionen beim Menschen sind Infektionen durch Protozoen bzw. Protista und Wurminfektionen. Einige Parasiten übertragen Krankheitserreger auf den Menschen, die zum Teil tödliche Krankheiten (Parasitosen) verursachen. Eine Auflistung parasitärer Erkrankungen ist weiter unten beschrieben. Auch auf viele Bakterien und Pilze trifft die Definition Parasit zu; sie werden aber aufgrund ihrer medizinischen Bedeutung und auch ihres teilweise nur fakultativen Parasitismus in eigenen Fachgebieten, der Bakteriologie und Mykologie innerhalb der Mikrobiologie behandelt und werden daher auch in dieser Anmeldung getrennt beschrieben.

Parasiten reduzieren die Fitness des Wirtes durch eine allgemeine Pathologie, einen Lebensmittel-Wettbewerb, Organschäden oder der Änderung des Verhaltens. Oftmals kann Parasitenbefall von geringer Intensität eher harmlos verlaufen, d.h. ohne einen großen Schaden anzurichten und verursacht keine andauern Symptome. Mittlere und schwere Infektionen können jedoch die Ursache von schwereren Krankheitsbildern sein, wodurch sowohl die Morbidität als auch Mortalität steigt.

Der Einzeller *Plasmodium falciparum,* ein Parasit, der zu den Apicomplexa gehört, ist der Erreger der Malaria

tropica. Malaria, auch Sumpffieber oder Wechselfieber genannt. Plasmodien werden durch den Biss weiblicher Stechmücken übertragen, wobei die *Anopheles gambiae* das prominenteste Beispiel ist. Nach einer anfänglichen Phase in der Leber leben und vermehren sich diese Parasiten in den roten Blutkörperchen. Die Lyse der roten Blutkörperchen entlässt alle zwei Tage frische Parasiten ins Blut und führt zu den, für Malaria charakteristischen Fieberschüben. Derzeit gibt es rund 3 Milliarden Menschen in 108 Ländern mit einem Risiko für eine Malaria-Infektion. Mit 240 Millionen Malaria-Fällen jährlich, verursacht *P. falciparum* bis zu einer Million Todesfälle, wobei Afrika mit mehr als 90% am schwersten davon betroffen ist. Im Jahr 2004 wurde *P. falciparum* weltweit zu den führenden Todesursachen, welche durch nur einen einzigen Erreger verursacht wurden. Der Beginn der Chloroquin-Resistenz der Erreger markierte den Beginn eines neuen Kapitels in der Geschichte der Malaria in Südostasien und von 1973 musste Chloroquin durch die Kombination von Sulphadoxin und Pyrimethamin (SP) ersetzt werden. Ab 1985 wurde zunehmend Mefloquin eingesetzt. Die rasche Entwicklung von Resitenzen gegen die verwendeten Wirkstoffe hatte die Einführung von weiteren Wirkstoffen zur Folge. Die Therapieschemata für die Prävention und Behandlung von Chloroquin-resistenten *P. falciparum* sind mit höheren Kosten und Nebenwirkungen im Vergleich zu Chloroquin assoziiert. Daher sind dringend Maßnahmen erforderlich, um neue wirksame, erschwingliche und alternative Therapien gegen Malaria und zu entwickeln.

Schistosomatidae sind Trematoden, welche in den Blutgefässen von Wirbeltieren leben. Als Zwischenwirt dient eine Wasserschnecke. Die Schistosomosen oder Bilharziosen sind wichtige Tropenkrankheiten in über 70 Ländern. Neben S. mansoni haben S. haematobium (nur in Afrika) und S. japonicum (nur in Asien) grosse Bedeutung. Die Weltgesundheitsbehörde (WHO) schätzt dass heute rund 200 Millionen Menschen mit Schistosomen infiziert sind. Die Eier können in Stuhl (bei S. haematobium im Urin) und im Blut nachgewiesen werden. Neben der direkten Schädigung von Blutgefässen und Darmepithel durch migrierende Eier spielen immunologische Prozesse bei der Krankheitsentstehung eine Rolle. Im Besonderen sind dies Immunreaktionen gegen Ei-Antigene. Wurmeier, welche den Weg in den Darm nicht finden, lagern sich in der Leber (und andern Organen) ab und werden von Wirtszellen abgekapselt (Granulombildung). Das führt zu einer Organschädigung, meist zu einer Leberfibrose.

Eine deutsche Patentanmeldung DE 19633206 offenbart dass einige Steroidaminderivate, vor allem die Ferrocenylamino-, Ferrocenylimmnosteroide, breite antikrobielle Aktivität gegen grampositive und gramnegative Bakterien zeigen.

Der vorliegenden Erfindung liegt daher die Aufgabe zugrunde, neue antiparasitäre, antibakterielle, antimykotische und/oder antivirale Wirkstoffe zur Verfügung zu stellen.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

Die Erfindung betrifft Verbindungen der allgemeinen Formel (I)

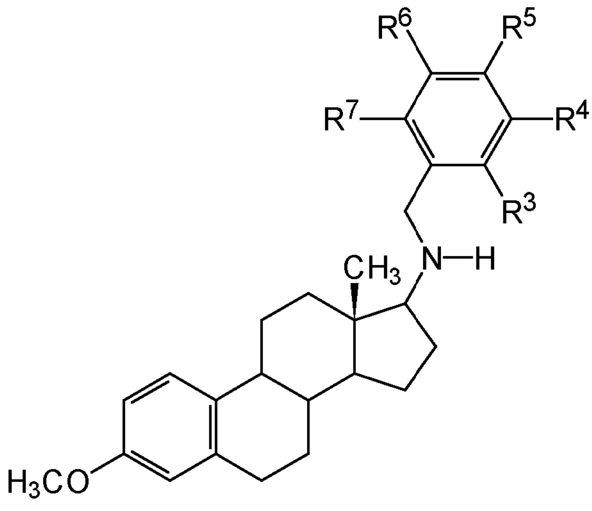

(I)

worin $R^3$, $R^4$, $R^5$, $R^6$ und $R^7$ unabhängig voneinander folgende Reste bedeuten: -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, -O-CO-Ph, -O-CO-$CH_3$, -O-CO-$C_2H_5$, -O-CO-$C_3H_7$, -O-CO-cyclo-$C_3H_5$, -O-CO-$CH(CH_3)_2$, -O-CO-$C(CH_3)_3$, -O-CO-para-$C_6H_4$-$OR^9$, $-CONH_2$, $-CONHCH_3$, $-CONHC_2H_5$,

-CONHC$_3$H$_7$, -CONH-cyclo-C$_3$H$_5$, -CONH[CH(CH$_3$)$_2$], -CONH[C(CH$_3$)$_3$], -CON(CH$_3$)$_2$, -CON(C$_2$H$_5$)$_2$, -CON(C$_3$H$_7$)$_2$, -CON(cyclo-C$_3$H$_5$)$_2$, -CON[CH(CH$_3$)$_2$]$_2$, -CON[C(CH$_3$)$_3$]$_2$, -NHCOCH$_3$, -NHCOC$_2$H$_5$, -NHCOC$_3$H$_7$, -NHCO-cyclo-C$_3$H$_5$, -NHCO-CH(CH$_3$)$_2$, -NHCO-C(CH$_3$)$_3$, -NHCO-OCH$_3$, -NHCO-OC$_2$H$_5$, -NHCO-OC$_3$H$_7$, -NHCO-O-cyclo-C$_3$H$_5$, -NHCO-OCH(CH$_3$)$_2$, -NHCO-OC(CH$_3$)$_3$, -NH$_2$, -NHCH$_3$, -NHC$_2$H$_5$, -NHC$_3$H$_7$, -NH-cyclo-C$_3$H$_5$, -NHCH(CH$_3$)$_2$, -NHC(CH$_3$)$_3$, -N(CH$_3$)$_2$, -N(C$_2$H$_5$)$_2$, -N(C$_3$H$_7$)$_2$, -N(cyclo-C$_3$H$_5$)$_2$, -N[CH(CH$_3$)$_2$]$_2$, -N[C(CH$_3$)$_3$]$_2$, -SOCH$_3$, -SOC$_2$H$_5$, -SOC$_3$H$_7$, -SO-cyclo-C$_3$H$_5$, -SOCH(CH$_3$)$_2$, -SOC(CH$_3$)$_3$, -SO$_2$CH$_3$, -SO$_2$C$_2$H$_5$, -SO$_2$C$_3$H$_7$, -SO$_2$-cyclo-C$_3$H$_5$, -SO$_2$CH(CH$_3$)$_2$, -SO$_2$C(CH$_3$)$_3$, -SO$_3$H, -SO$_3$CH$_3$, -SO$_3$C$_2$H$_5$, -SO$_3$C$_3$H$_7$, -SO$_3$-cyclo-C$_3$H$_5$, -SO$_3$CH(CH$_3$)$_2$, -SO$_3$C(CH$_3$)$_3$, -SO$_2$NH$_2$, -OCF$_3$, -OC$_2$F$_5$, -O-COOCH$_3$, -O-CO0C$_2$H$_5$, -O-COOC$_3$H$_7$, -O-COO-cyclo-C$_3$H$_5$, -O-COOCH(CH$_3$)$_2$, -O-COOC(CH$_3$)$_3$, -NH-CO-NH$_2$, -NH-CO-NHCH$_3$, -NH-CO-NHC$_2$H$_5$, -NH-CO-NHC$_3$H$_7$, -NH-CO-NH-cyclo-C$_3$H$_5$, -NH-CO-NH[CH(CH$_3$)$_2$], -NH-CO-NH[C(CH$_3$)$_3$], -NH-CO-N(CH$_3$)$_2$, -NH-CO-N(C2H5)$_2$, -NH-CO-N(C3H$_7$)$_2$, -NH-CO-N(cyclo-C$_3$H$_5$)$_2$, -NH-CO-N[CH(CH$_3$)$_2$]$_2$, -NH-CO-N[C(CH$_3$)$_3$]$_2$, -NH-CS-NH$_2$, -NH-CS-NHCH$_3$, -NH-CS-NHC$_2$H$_5$, -NH-CS-NHC$_3$H$_7$, -NH-CS-NH-cyclo-C$_3$H$_5$, -NH-CS-NH[CH(CH$_3$)$_2$], -NH-CS-NH[C(CH$_3$)$_3$], -NH-CS-N(CH$_3$)$_2$, -NH-CS-N(C$_2$H$_5$)$_2$, -NH-CS-N(C$_3$H$_7$)$_2$, -NH-CS-N(cyclo-C$_3$H$_5$)$_2$, -NH-CS-N[CH(CH$_3$)$_2$]$_2$, -NH-CS-N[C(CH$_3$)$_3$]$_2$, -NH-C(=NH)-NH$_2$, -NH-C(=NH)-NHCH$_3$, -NH-C(=NH)-NHC$_2$H$_5$, -NH-C(=NH)-NHC$_3$H$_7$, -NH-C(=NH)-NH-cyclo-C$_3$H$_5$, -NH-C(=NH)-NH[CH(CH$_3$)$_2$], -NH-C(=NH)-NH[C(CH$_3$)$_3$], -NH-C(=NH)-N(CH$_3$)$_2$, -NH-C(=NH)-N(C$_2$H$_5$)$_2$, -NH-C(=NH)-N(C$_3$H$_7$)$_2$, -NH-C(=NH)-N(cyclo-C$_3$H$_5$)$_2$, -NH-C(=NH)-N[CH(CH$_3$)$_2$]$_2$, -NH-C(=NH)-N[C(CH$_3$)$_3$]$_2$, -O-CO-NH$_2$, -O-CO-NHCH$_3$, -O-CO-NHC$_2$H$_5$, -O-CO-NHC$_3$H$_7$, -O-CO-NH-cyclo-C$_3$H$_5$, -O-CO-NH[CH(CH$_3$)$_2$], -O-CO-NH[C(CH$_3$)$_3$], -O-CO-N(CH$_3$)$_2$, -O-CO-N(C$_2$H$_5$)$_2$, -O-CO-N(C$_3$H$_7$)$_2$, -O-CO-N(cyclo-C$_3$H$_5$)$_2$, -O-CO-N[CH(CH$_3$)$_2$]$_2$, -O-CO-N[C(CH$_3$)$_3$]$_2$, -O-CO-OCH$_3$, -O-CO-0C$_2$H$_5$, -O-CO-OC$_3$H$_7$, -O-CO-O-cyclo-C$_3$H$_5$, -O-CO-OCH(CH$_3$)$_2$, -O-CO-OC(CH$_3$)$_3$, -CH$_2$F, -CHF$_2$, -CF$_3$, -CH$_2$Cl, -CH$_2$Br, -CH$_2$I, -CH$_2$-CH$_2$F, -CH$_2$-CHF$_2$, -CH$_2$-CF$_3$, -CH$_2$-CH$_2$Cl, -CH$_2$-CH$_2$Br, -CH$_2$-CH$_2$I, cyclo-C$_3$H$_5$, cyclo-C$_4$H$_7$, cyclo-C$_5$H$_9$, cyclo-C$_6$H$_{11}$, cyclo-C$_7$H$_{13}$, cyclo-C$_8$H$_{15}$, -Ph, -CH$_2$-Ph, -CPh$_3$, -CH$_3$, -C$_2$H$_5$, -C$_3$H$_7$, -CH(CH$_3$)$_2$, -C$_4$H$_9$, -CH$_2$-CH(CH$_3$)$_2$, -CH(CH$_3$)-C$_2$H$_5$, -C(CH$_3$)$_3$, -C$_5$H$_{11}$, -CH(CH$_3$)-C$_3$H$_7$, -CH$_2$-CH(CH$_3$)-C$_2$H$_5$, -CH(CH$_3$)-CH(CH$_3$)$_2$, -C(CH$_3$)$_2$-C$_2$H$_5$, -CH$_2$-C(CH$_3$)$_3$, -CH(C$_2$H$_5$)$_2$, -C$_2$H$_4$-CH(CH$_3$)$_2$, -C$_6$H$_{13}$, -C$_7$H$_{15}$, -C$_8$H$_{17}$, -C$_3$H$_6$-CH(CH$_3$)$_2$, -C$_2$H$_4$-CH(CH$_3$)-C$_2$H$_5$, -CH(CH$_3$)-C$_4$H$_9$, -CH$_2$-CH(CH$_3$)-C$_3$H$_7$, -CH(CH$_3$)-CH$_2$-CH(CH$_3$)$_2$, -CH(CH$_3$)-CH(CH$_3$)-C$_2$H$_5$, -CH$_2$-CH(CH$_3$)-CH(CH$_3$)$_2$, -CH$_2$-C(CH$_3$)$_2$-C$_2$H$_5$, -C(CH$_3$)$_2$-C$_3$H$_7$, -C(CH$_3$)$_2$-CH(CH$_3$)$_2$, -C$_2$H$_4$-C(CH$_3$)$_3$, -CH(CH$_3$)-C(CH$_3$)$_3$, -CH=CH$_2$, -CH$_2$-CH=CH$_2$, -C(CH$_3$)=CH$_2$, -CH=CH-CH$_3$, -C$_2$H$_4$-CH=CH$_2$, -CH$_2$-CH=CH-CH$_3$, -CH=CH-C$_2$H$_5$, -CH$_2$-C(CH$_3$)=CH$_2$, -CH(CH$_3$)-CH=CH, -CH=C(CH$_3$)$_2$, -C(CH$_3$)=CH-CH$_3$, -CH=CH-CH=CH$_2$, -C$_3$H$_6$-CH=CH$_2$, -C$_2$H$_4$-CH=CH-CH$_3$, -CH$_2$-CH=CH-C$_2$H$_5$, -CH=CH-C$_3$H$_7$, -CH$_2$-CH=CH-CH=CH$_2$, -CH=CH-CH=CH-CH$_3$, -CH=CH-CH$_2$-CH=CH$_2$, -C(CH$_3$)=CH-CH=CH$_2$, -CH=C(CH$_3$)-CH=CH$_2$, -CH=CH-C(CH$_3$)=CH$_2$, -C$_2$H$_4$-C(CH$_3$)=CH$_2$, -CH$_2$-CH(CH$_3$)-CH=CH$_2$, -CH(CH$_3$)-CH$_2$-CH=CH$_2$, -CH$_2$-CH=C(CH$_3$)$_2$, -CH$_2$-C(CH$_3$)=CH-CH$_3$, -CH(CH$_3$)-CH=CH-CH$_3$, -CH=CH-CH(CH$_3$)$_2$, -CH=C(CH$_3$)-C$_2$H$_5$, -C(CH$_3$)=CH-C$_2$H$_5$, -C(CH$_3$)=C(CH$_3$)$_2$, -C(CH$_3$)$_2$-CH=CH$_2$, -CH(CH$_3$)-C(CH$_3$)=CH$_2$, -C(CH$_3$)=CH-CH=CH$_2$, -CH=C(CH$_3$)-CH=CH$_2$, -CH=CH-C(CH$_3$)=CH$_2$, -C$_4$H$_8$-CH=CH$_2$, -C$_3$H$_6$-CH=CH-CH$_3$, -C$_2$H$_4$-CH=CH-C$_2$H$_5$, -CH$_2$-CH=CH-C$_3$H$_7$, -CH=CH-C$_4$H$_9$, -C$_3$H$_6$-C(CH$_3$)=CH$_2$, -C$_2$H$_4$-CH(CH$_3$)-CH=CH$_2$, -CH$_2$-CH(CH$_3$)-CH$_2$-CH=CH$_2$,-CH(CH$_3$)-C$_2$H$_4$-CH=CH$_2$, -C$_2$H$_4$-CH=C(CH$_3$)$_2$, -C$_2$H$_4$-C(CH$_3$)=CH-CH$_3$, -CH$_2$-CH(CH$_3$)-CH=CH-CH$_3$, -CH(CH$_3$)-CH$_2$-CH=CH-CH$_3$, -CH$_2$-CH=CH-CH(CH$_3$)$_2$, -CH$_2$-CH=C(CH$_3$)-C$_2$H$_5$, -CH$_2$-C(CH$_3$)=CH-C$_2$H$_5$, -CH(CH$_3$)-CH=CH-C$_2$H$_5$, -CH=CH-CH$_2$-CH(CH$_3$)$_2$, -CH=CH-CH(CH$_3$)-C$_2$H$_5$, -CH=C(CH$_3$)-C$_3$H$_7$, -C(CH$_3$)=CH-C$_3$H$_7$, -CH$_2$-CH(CH$_3$)-C(CH$_3$)=CH$_2$, -CH(CH$_3$)-CH$_2$-C(CH$_3$)=CH$_2$, -CH(CH$_3$)-CH(CH$_3$)-CH=CH$_2$, -CH$_2$-C(CH$_3$)$_2$-CH=CH$_2$, -C(CH$_3$)$_2$-CH$_2$-CH=CH$_2$, -CH$_2$-C(CH$_3$)=C(CH$_3$)$_2$, -CH(CH$_3$)-CH=C(CH$_3$)$_2$, -C(CH$_3$)$_2$-CH=CH-CH$_3$, -CH(CH$_3$)-C(CH$_3$)=CH-CH$_3$, -CH=C(CH$_3$)-CH(CH$_3$)$_2$, -C(CH$_3$)=CH-CH(CH$_3$)$_2$, -C(CH$_3$)=C(CH$_3$)-C$_2$H$_5$, -CH=CH-C(CH$_3$)$_3$, -C(CH$_3$)$_2$-C(CH$_3$)=CH$_2$, -CH(C$_2$H$_5$)-C(CH$_3$)=CH$_2$, -C(CH$_3$)(C$_2$H$_5$)-CH=CH$_2$, -CH(CH$_3$)-C(C$_2$H$_5$)=CH$_2$, -CH$_2$-C(C$_3$H$_7$)=CH$_2$, -CH$_2$-C(C$_2$H$_5$)=CH-CH$_3$, -CH(C$_2$H$_5$)-CH=CH-CH$_3$, -C(C$_4$H$_9$)=CH$_2$, -C(C$_3$H$_7$)=CH-CH$_3$, -C(C$_2$H$_5$)=CH-C$_2$H$_5$, -C(C$_2$H$_5$)=C(CH$_3$)$_2$, -C[C(CH$_3$)$_3$]=CH$_2$, -C[CH(CH$_3$)(C$_2$H$_5$)]=CH$_2$, -C[CH$_2$-CH(CH$_3$)$_2$]=CH$_2$, -C$_2$H$_4$-CH=CH-CH=CH$_2$, -CH=CH-CH=C(CH$_3$)$_2$, -CH$_2$-CH=CH-CH$_2$-CH=CH$_2$, -CH=CH-C$_2$H$_4$-CH=CH$_2$, -CH$_2$-CH=CH-CH=CH-CH$_3$, -CH=CH-CH$_2$-CH=CH-CH$_3$, -CH=CH-CH=CH-C$_2$H$_5$, -CH$_2$-CH=CH-C(CH$_3$)=CH$_2$, -CH$_2$-CH=C(CH$_3$)-CH=CH$_2$, -CH$_2$-C(CH$_3$)=CH-CH=CH$_2$, -CH(CH$_3$)-CH=CH-CH=CH$_2$, -CH=CH-CH$_2$-C(CH$_3$)=CH$_2$, -CH=CH-CH(CH$_3$)-CH=CH$_2$, -CH=C(CH$_3$)-CH$_2$-CH=CH$_2$, -C(CH$_3$)=CH-CH$_2$-CH=CH$_2$, -CH=CH-C(CH$_3$)=CH-CH$_3$, -CH=C(CH$_3$)-CH=CH-CH$_3$, -C(CH$_3$)=CH-CH=CH-CH$_3$, -CH=C(CH$_3$)-C(CH$_3$)=CH$_2$, -C(CH$_3$)=CH-C(CH$_3$)=CH$_2$, -C(CH$_3$)=C(CH$_3$)-CH=CH$_2$, -CH=CH-CH=CH-CH=CH$_2$, -C≡CH, -C≡C-CH$_3$, -CH$_2$-C≡CH, -C$_2$H$_4$-C≡CH, -CH$_2$-C≡C-CH$_3$, -C≡C-C$_2$H$_5$, -C$_3$H$_6$-C≡CH, -C$_2$H$_4$-C≡C-CH$_3$,-CH$_2$-C≡C-C$_2$H$_5$, -C≡C-C$_3$H$_7$, -CH(CH$_3$)-C≡CH, -CH$_2$-CH(CH$_3$)-C≡CH, -CH(CH$_3$)-CH$_2$-C≡CH, -CH(CH$_3$)-C≡C-CH$_3$, -C$_4$H$_8$-C≡CH, -C$_3$H$_6$-C≡C-CH$_3$, -C$_2$H$_4$-C≡C-C$_2$H$_5$, -CH$_2$-C≡C-C$_3$H$_7$, -C≡C-C$_4$H$_g$, -C$_2$H$_4$-CH(CH$_3$)-C≡CH, -CH$_2$-CH(CH$_3$)-CH$_2$-C≡CH, -CH(CH$_3$)-C$_2$H$_4$-C≡CH, -CH$_2$-CH(CH$_3$)-C≡C-CH$_3$, -CH(CH$_3$)-CH$_2$-C≡C-CH$_3$, -CH(CH$_3$)-C≡C-C$_2$H$_5$, -CH$_2$-C≡C-CH(CH$_3$)$_2$, -C≡C-CH(CH$_3$)-C$_2$H$_5$, -C≡C-CH$_2$-CH(CH$_3$)$_2$, -C≡C-C(CH$_3$)$_3$, -CH(C$_2$H$_5$)-C≡C-CH$_3$, -C(CH$_3$)$_2$-C≡C-CH$_3$, -CH(C$_2$H$_5$)-CH$_2$-C≡CH, -CH$_2$-CH(C$_2$H$_5$)-C≡CH, -C(CH$_3$)$_2$-CH$_2$-C≡CH, -CH$_2$-C(CH$_3$)$_2$-C≡CH, -CH(CH$_3$)-CH(CH$_3$)-C≡CH, -CH(C$_3$H$_7$)-C≡CH, -C(CH$_3$)(C$_2$H$_5$)-C≡CH, -C=C-C=CH, -CH$_2$-C≡C-C≡CH, -C≡C-C≡C-CH$_3$, -CH(C≡CH)$_2$, -C$_2$H$_4$-C≡C-C≡CH, -CH$_2$-C≡C-CH$_2$-C≡CH, -C≡C-C$_2$H$_4$-C≡CH, -CH$_2$-C≡C-C≡C-CH$_3$, -C≡C-CH$_2$-C≡C-CH$_3$, -C≡C-C≡C-C$_2$H$_5$, -C≡C-CH(CH$_3$)-C≡CH, -CH(CH$_3$)-C≡C-C≡CH, -CH(C≡CH)-CH$_2$-C≡CH, -C(C≡CH)$_2$-CH$_3$, -CH$_2$-CH(C≡CH)$_2$, -CH(C≡CH)-C≡C-CH$_3$; wobei jeweils zwei benachbarte Substituenten R$^3$ und R$^4$ oder R$^4$ und R$^5$

oder $R^5$ und $R^6$ oder $R^6$ und $R^7$ über -CH=CH-CH=CH-Gruppe miteinander verbunden sind, um einen kondensierten aromatischen Ring zu bilden;
sowie deren Metallkomplexe, Salze, Enantiomere, Enantiomerengemische, Diastereomere, Diastereomerengemische, Tautomere, Hydrate, Solvate und Racemate der vorgenannten Verbindungen. Bei den erfindungsgemäßen Verbindungen ist zudem bevorzugt, wenn einer der Reste $R^3$, $R^5$ oder $R^7$eine Hydroxygruppe ist. Des Weiteren ist bevorzugt, wenn die erfindungsgemäßen Verbindungen keine $-C(CH_3)_3$ -Gruppe an den Positionen $R^3$ - $R^7$ tragen.

Die Verbindungen der Formel (I) entsprechend der vorliegenden Erfindung können selbst oder in Form eines pharmakologisch wirksamen Salzes verabreicht werden. Da die Verbindungen der allgemeinen Formel (I) sowohl basische Eigenschaften als auch saure Eigenschaften besitzen können, können Salze dieser Verbindungen nach gängigen Methoden hergestellt werden.

Geeignete Beispiele dieser Salze der Verbindungen der Formel (I) umschließen Säureadditionssalze, Alkalimetallsalze sowie Salze mit Aminen. So können Alkalimetallsalze wie das Natriumsalz, das Kaliumsalz, das Lithiumsalz oder das Magnesiumsalz, das Calciumsalz, Alkylammoniumsalze oder Aminosäurensalze, z.B. mit basischen Aminosäuren wie Lysin, genannt werden. Als Säuren, welche ein Säureadditionssalz der Verbindung der Formel (I) bilden, können die folgenden genannt werden: Schwefelsäure, Sulfonsäuren, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Das Grundgerüst der Steroide ist das Gonan, welches ein Cyclopentanoperhydrophenanthren-Ringsystem darstellt (Ausnahme: Vitamin D). Gonan besteht aus siebzehn Kohlenstoffatomen, welche vier Ringe bilden: drei Cyclohexanringe (bezeichnet als Ringe A, B und C in der Abbildung Steroidgrundgerüst unten) und einen Cyclopentanring (D-Ring). Bevorzugt sind Verbindungen der allgemeinen Formel (I), wobei der Steroidrest am D-Ring substituiert ist. Die Steroide unterscheiden sich durch die funktionellen Gruppen, die an den Ringen binden und durch die Oxidationsstufe der Ringe. Es werden viele verschiedene Steroide von Pflanzen, Tieren und Pilze synthetisiert. Alle Steroide werden in Zellen entweder aus Lanosterin (Tiere und Pilze) oder aus Cycloartenol (Pflanzen) hergestellt. Steroide haben eine starre Molekülgestalt, in der Regel einen relativ hohen Schmelzpunkt und lassen sich gut kristallisieren. Durch die asymmetrischen C-Atome an den Ringverknüpfungen sind zahlreiche Struktur-Isomere möglich. Nach allgemeiner Konvention dient die Position der Methylgruppe am Kohlenstoffatom 10, welche bei natürlich vorkommenden Steroiden zumeist oberhalb der gedachten Steroidebene (β-Seite) liegt, als Bezugspunkt für die systematische Namensgebung der räumlichen Konfiguration von Substituenten am Gonangerüst, die an gesättigten Ringsystemen prinzipiell axial oder äquatorial sein Können: Mehr in Richtung β-Seite weisende (zumeist äquatoriale) Substitutienten werden mit dem Index β bezeichnet, während mehr in Richtung Steroidunterseite weisende (zumeist axial stehende) Substituenten mit dem Index α gekennzeichnet werden.

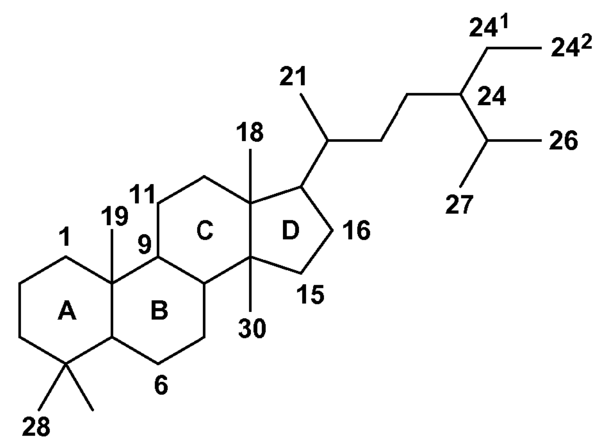

Die folgenden beiden Formeln beschreiben eine Form des Gonangrundgerüstes:

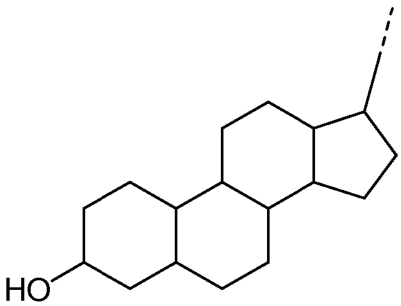

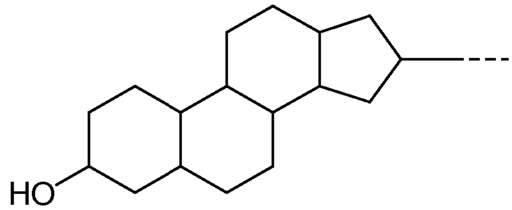

Gonan ist der systematische Name für das Kohlenwasserstoff-Grundgerüst der Steroide (Perhydrocyclopenta[a]penanthren) ohne anguläre Methyl-Gruppen an C-10 und C-13. Sterole (oder auch Sterine) sind Steoridalkohole. Sie tragen an C-3 eine β-ständige Hydroxygruppe und besitzen eine oder mehrere Doppelbindungen in Ring B, weitere Sauerstoff-Funktionen wie Carbonyl- und Carboxy-Gruppen fehlen.

Das wichtigste tierische Sterol ist das Cholesterol (Cholesterin). In Pflanzen und Mikroorganismen findet man eine größere Vielfalt ähnlicher Sterole, z.B. Ergosterol oder Stigmasterol. Aus Cholesterol werden in der Leber Gallensäuren (z.B. Cholsäure) gebildet. Charakteristisch ist die um drei C-Atome verkürzte Seitenkette, deren letztes C-Atoms zur Carboxy-Gruppe oxidiert wird. Die Doppelbindung in Ring B ist reduziert und am Steroidkern befinden sich eine bis drei α-ständige HydroxyGruppen. Steroidhormone werden bei Säugern ebenfalls aus Cholesterol gebildet.

Steroidhormoneklasse mit Schlüsselfunktion für den menschlichen Organismus lassen sich folgenden sechs Steroid-Grundkörpern zuordnen: Progesteron, Cortisol, Aldosteron, Testosteron, Estradiol und Calcitriol. Diese Steroide tragen mit Ausnahme des Calcitriols entweder gar keine oder nur eine kurze (max. 2 C-Atome) Seitenkette. Charakteristisch ist eine Oxo-Gruppe an C-3 und die dazu konjugierte Doppelbindung an C-4/C-5 in Ring A. Unterschiede finden sich in den Ringen A, B, C und D. Estradiol ist in Ring A aromatisch. Calcitriol weicht von den übrigen Steroidhormonen der Säuger ab. Es enthält noch das ganze C-Gerüst des Cholesterols, ist aber durch eine Öffnung des Ringes B zu einem Secosteroid (Steroid mit geöffnetem Ring) abgewandelt.

Außerdem bevorzugt sind Verbindungen der allgemeinen Formel (I), worin $R^3$ -OH, $-OCH_3$, $-OC_2H_5$, $-OC_3H_7$, -O-cyclo-$C_3H_5$, $-OCH(CH_3)_2$, $-OC(CH_3)_3$,$-OC_4H_9$, -OPh, $-OCH_2$-Ph, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$ oder $-N[C(CH_3)_3]_2$ bedeutet.

Folgende Verbindungen der allgemeinen Formel (I) sind besonders bevorzugt:

| | Formel | Nomenklatur |
|---|---|---|
| 17 | 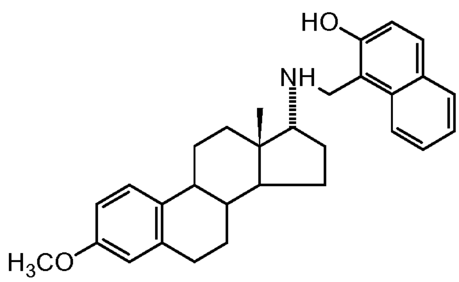 | 17α-[(2-Hydroxynaphth-1-yl)-methylamino]-3-methoxy-estra-1,3,5(10)-trien |
| 18 | 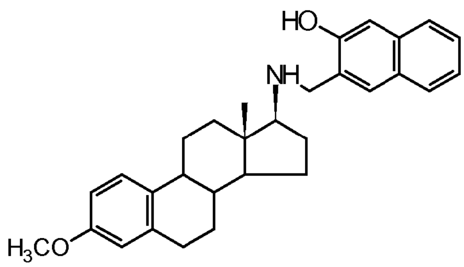 | 17β-[(3-Hydroxynaphth-2-yl)-methylamino]-3-methoxy-estra-1,3,5(10)-trien |

(fortgesetzt)

| | Formel | Nomenklatur |
|---|---|---|
| 19 | 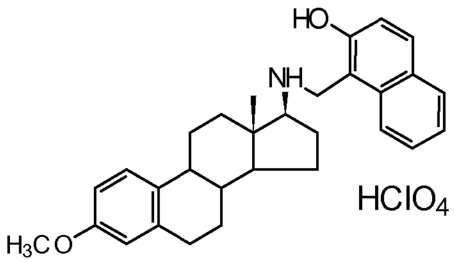 | 17β-[(2-Hydroxynaphth-1-yl)-methylamino]-3-methoxy-estra-1,3,5(10)-trien Hydroperchlorat |
| 41 | 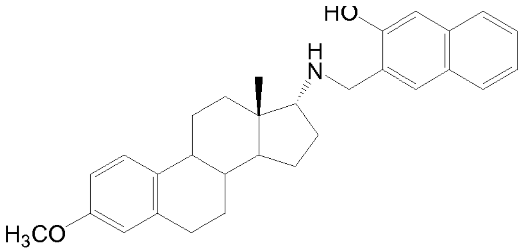 | 17α-[(3-Hydroxy-naphth-2-yl)-methylamino]-3-methoxy-estra-1,3,5(10)-trien |

Die Referenzverbidnungen sind folgend:

| | Formel | Nomenklatur |
|---|---|---|
| 1a | 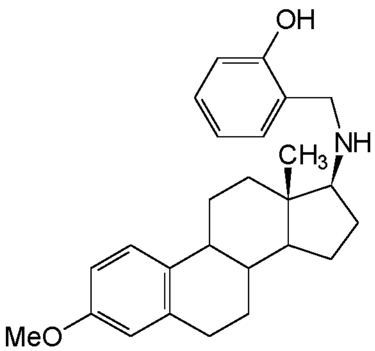 | 17β-(2-Hydroxyphenyl-methylamino)-3-methoxy-estra-1,3,5(10)-trien |
| 2 | 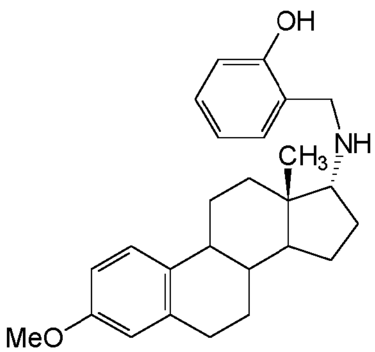 | 17α-(2-Hydroxyphenyl-methylamino)-3-methoxy-estra-1,3,5(10)-trien |
| 3 | 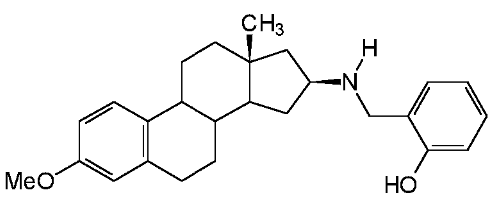 | 16β-(2-Hydroxyphenyl- methylamino)-3-methoxy-estra-1,3,5(10)-trien |

(fortgesetzt)

| | Formel | Nomenklatur |
|---|---|---|
| 4 | 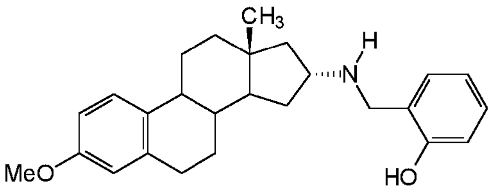 | 16α-(2-Hydroxyphenyl-methylamino)-3-methoxy-estra-1,3,5(10)-trien |
| 5 | 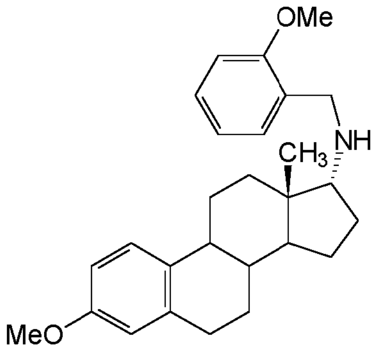 | 17α-(2-Methoxyphenyl-methylamino)-3-methoxy-estra-1.3,5(10)-trien |
| 6 | 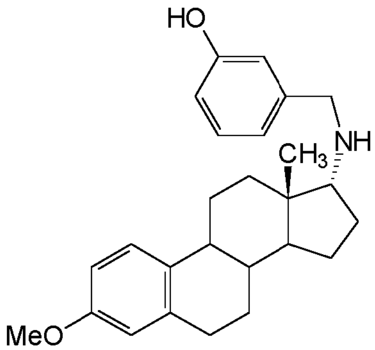 | 17α-(3-Hydroxyphenyl-methylamino)-3-methoxy-estra-1,3,5(10)-trien |
| 7 | 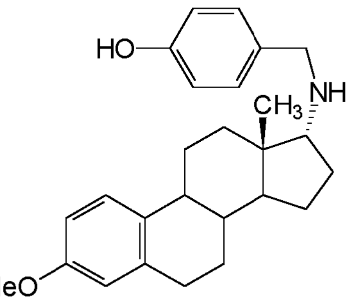 | 17α-(4-Hydroxyphenyl-methylamino)-3-methoxy-estra-1,3,5(10)-trien |
| 8 | 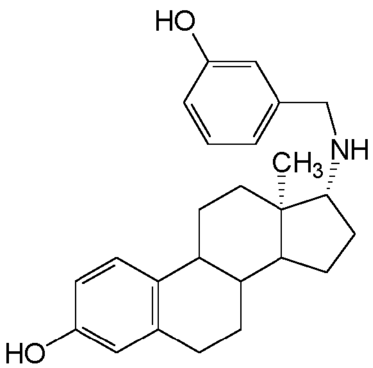 | 13α-3-Hydroxy-17α-(3-hydroxyphenyl-methylamino)-estra-1,3,5(10)-trien |

(fortgesetzt)

| | Formel | Nomenklatur |
|---|---|---|
| 9 | 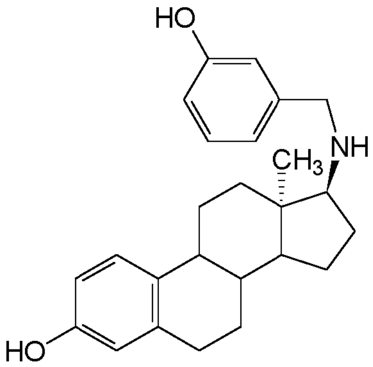 | 13α-3-Hydroxy-17β-(3-hydroxyphenyl-methylamino)-estra-1,3,5(10)-trien |
| 10 | 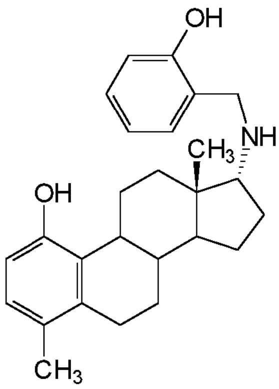 | 1-Hydroxy-17a-(2-hydroxyphenyl-methylamino)-4-methyl-estra-1,3,5(10)-trien |
| 11 | 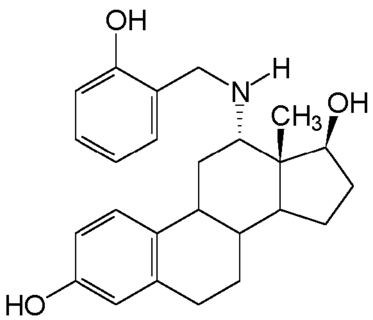 | 3,17β-Dihydroxy-12α-(2-hydroxyphenylmethyl-amino)estra-1,3,5(10)-trien |
| 12 | 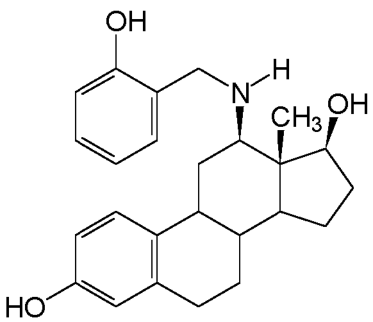 | 3,17β-Dihydroxy-12β-(2-hydroxyphenyl-methylamino)estra-1,3,5(10)-trien |

(fortgesetzt)

| | Formel | Nomenklatur |
|---|---|---|
| 13 | 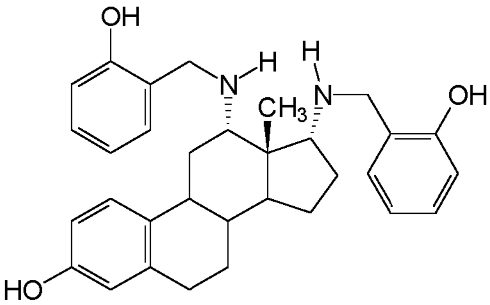 | 3-Hydroxy-12α,17α-bis(2-hydroxyphenyl-methylamino)-estra-1,3,5(10)-trien |
| 14 | 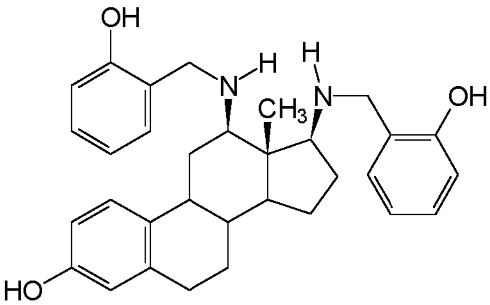 | 3-Hydroxy-12β,17β-bis(2-hydroxyphenyl-methylamino)-estra-1,3,5(10)-trien |
| 15 | 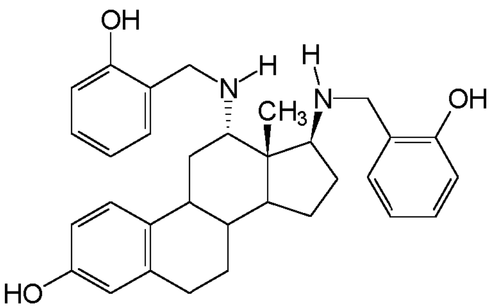 | 3-Hydroxy-12α,17β-bis(2-hydroxyphenyl-methylamino)-estra-1,3,5(10)-trien |
| 16 | 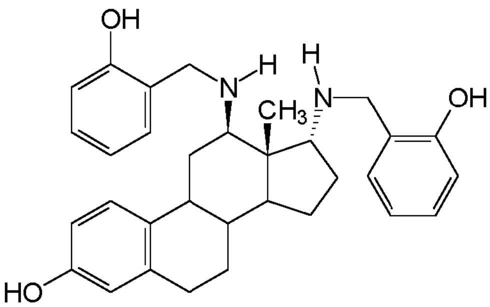 | 3-Hydroxy-12β,17α-bis(2-hydroxyphenyl-methylamino)-estra-1,3,5(10)-trien |
| 20 | 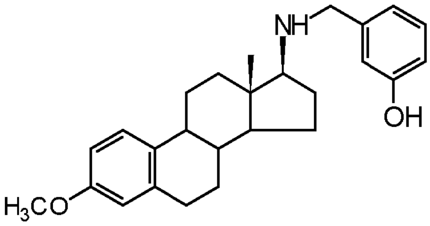 | 17β-(3-Hydroxyphenyl-methylamino)-3-methoxy-estra-1,3,5(10)-trien |

(fortgesetzt)

| | Formel | Nomenklatur |
|---|---|---|
| 21 | 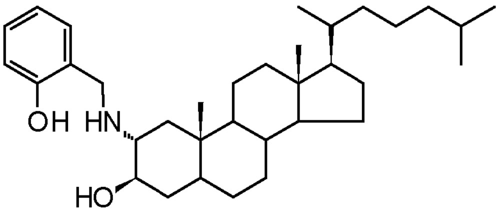 | 3β-Hydroxy-2α-(2-hydroxyphenyl-methylamino)-cholan |
| 22 | 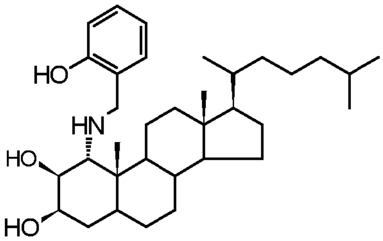 | 2β,3β-Dihydroxy-1α-(2-hydroxyphenyl-methylamino)-cholan |
| 23 | 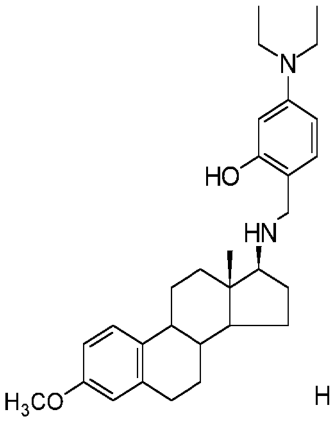 | 17β-[4-(N,N-diethylamino)-2-hydroxyphenyl-methylamino]-3-methoxy-estra-1,3,5(10)-trien |
| 24 | 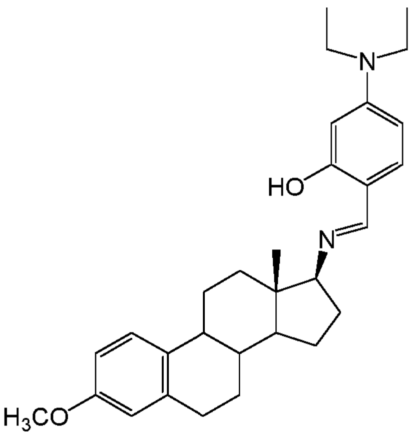 | 17β-(4-N,N-Diethylamino-2-hydroxyphenyl-carbaldimino)-3-methoxy-estra-1,3,5(10)-trien |
| 25 | 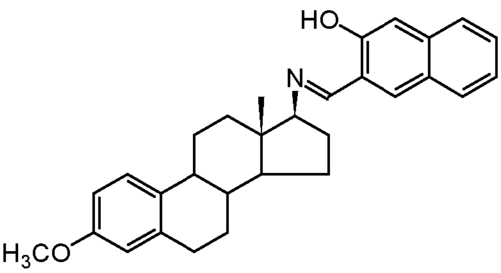 | 17β-(3-Hydroxy-naphth-2-carbaldimino)-3-methoxy-estra-1,3,5(10)-trien |

(fortgesetzt)

| | Formel | Nomenklatur |
|---|---|---|
| 26 | 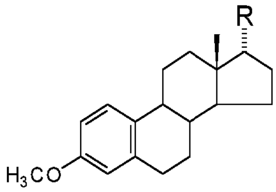 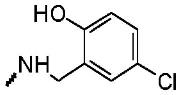 | 17α-(5-Chlor-2-hydroxyphenyl-methylamino)-3-methoxy-estra-1,3,5(10)-trien |
| 27 | 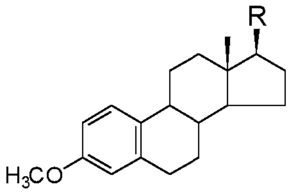 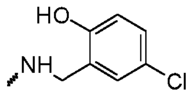 | 17β-(5-Chlor-2-hydroxyphenyl-methylamino)-3-methoxy-estra-1,3,5(10)-trien |
| 28 | 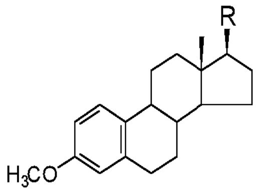 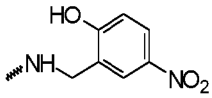 | 17β-(2-Hydroxy-5-nitrophenyl-methylamino)-3-methoxy-estra-1,3,5(10)-trien |
| 29 | 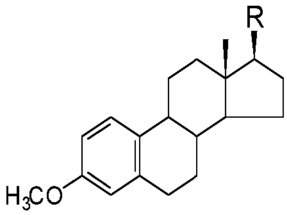 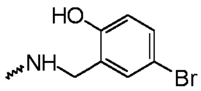 | 17β-(5-Brom-2-hydroxyphenyl-methylamino)-3-methoxy-estra-1,3,5(10)-trien |
| 30 | 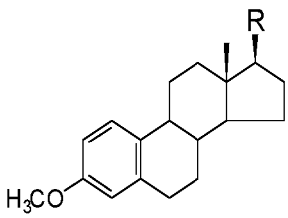 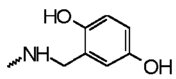 | 17β-(2,5-Dihydroxyphenyl-methylamino)-3-methoxy-estra-1,3,5(10)-trien |
| 31 | 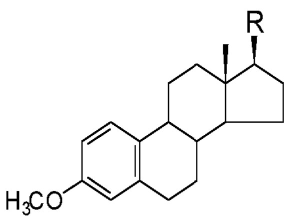 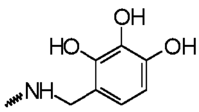 | 17β-(2,3,4-Trihydroxyphenyl-methylamino)-3-methoxy-estra-1,3,5(10)-trien |
| 32 | 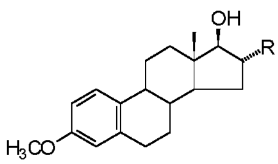 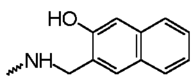 | 16α-[(3-Hydroxy-naphth-2-yl)-methylamino]-17β-hydroxy-3-methoxy-estra-1,3,5(10)-trien |

(fortgesetzt)

| | Formel | Nomenklatur |
|---|---|---|
| 33 | 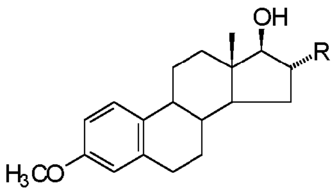 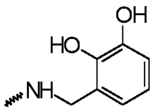 | 16α-(2,3-Dihydroxyphenyl-methylamino)-17β-hydroxy-3-methoxy-estra-1,3,5(10)-trien |
| 34 | 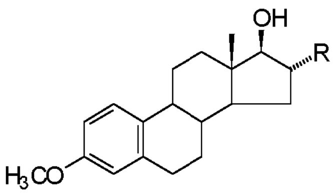 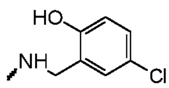 | 16α-(5-Chlor-2-hydroxyphenyl-methylamino)-17β-hydroxy-3-methoxy-estra-1,3,5(10)-trien |
| 35 | 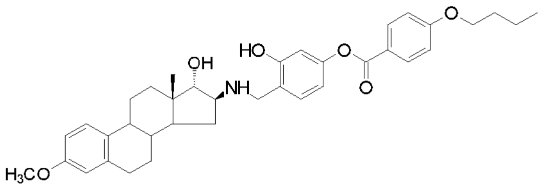 | 16β-{[2-Hydroxy-4-(4'-(4"-n-butoxyphenyl) carbonyloxy)-phenyl]-methylamino}-17α-hydroxy-3-methoxy-estra-1,3,5(10)-trien |
| 36 | 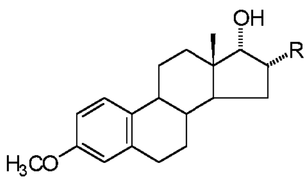 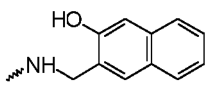 | 16α-[(3-Hydroxy-naphth-2-yl)-methylamino]-17α-hydroxy-3-methoxy-estra-1,3,5(10)-trien |
| 37 | 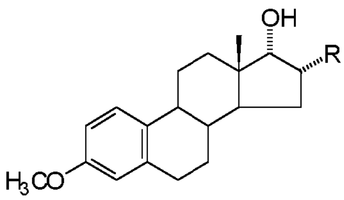 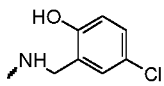 | 16α-(5-Chlor-2-hydroxyphenyl-methylamino)-17α-hydroxy-3-methoxy-estra-1,3,5(10)-trien |
| 38 | 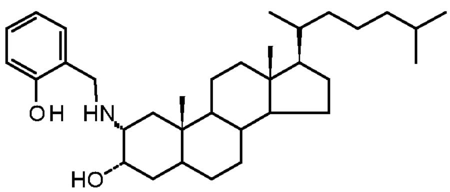 | 2α-(2-Hydroxyphenyl-methylamino)-3α-hydroxy-cholan |
| 39 | 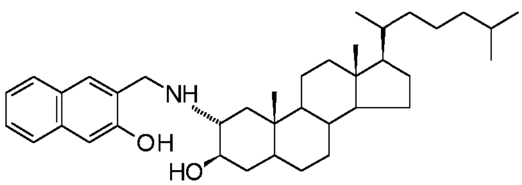 | 2α-[(3-Hydroxy-naphth-2-yl)-methylamino]-3β-hydroxy-cholan |

(fortgesetzt)

| | Formel | Nomenklatur |
|---|---|---|
| 40 | 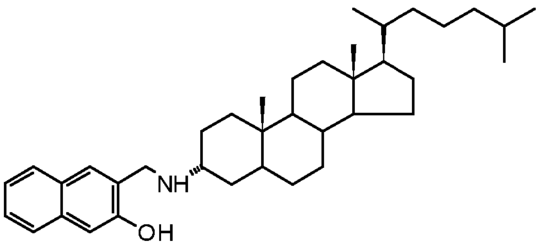 | 3α-[(3-Hydroxy-naphth-2-yl)-methylamino]-cholan |
| 42 | 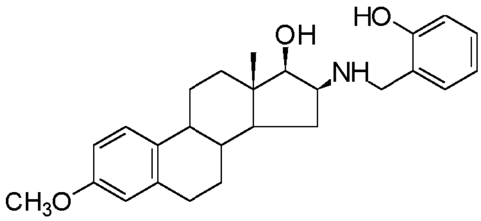 | 16β-(2-Hydroxyphenyl-methylamino)-17β-hydroxy-3-methoxy-estra-1,3,5(10)-trien |
| 43 | 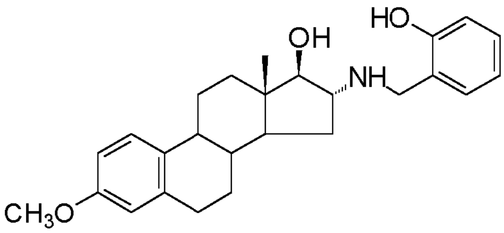 | 16α-(2-Hydroxyphenyl-methylamino)-17β-hydroxy-3-methoxy-estra-1,3,5(10)-trien |
| 44 | 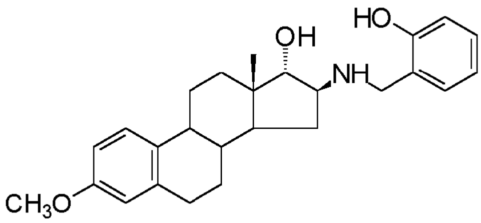 | 16β-(2-Hydroxyphenyl-methylamino)-17α-hydroxy-3-methoxy-estra-1,3,5(10)-trien |
| 45 | 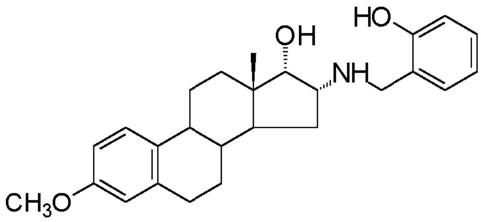 | 16α-(2-Hydroxyphenyl-methylamino)-17α-hydroxy-3-methoxy-estra-1,3,5(10)-trien |
| 46 | 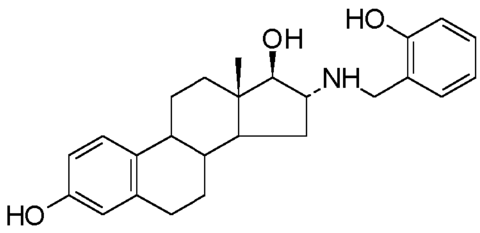 | 3,17β-Dihydroxy-16α-(2-hydroxyphenyl-methylamino)-estra-1,3,5(10)-trien |

(fortgesetzt)

| | Formel | Nomenklatur |
|---|---|---|
| 47 | 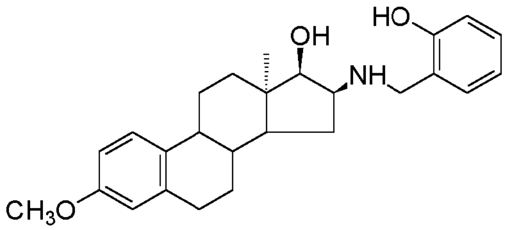 | 16β-(2-Hydroxyphenyl-methylamino)-17β-hydroxy-3-methoxy-13α-estra-1,3,5(10)-trien |
| 48 | 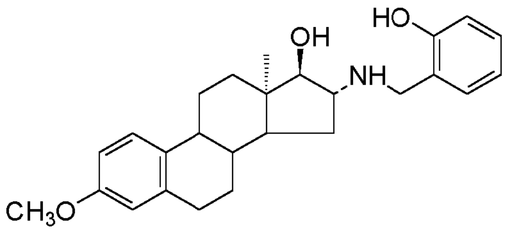 | 16α-(2-Hydroxyphenyl-methylamino)-17β-hydroxy-3-methoxy-13a-estra-1,3,5(10)-trien |
| 49 | 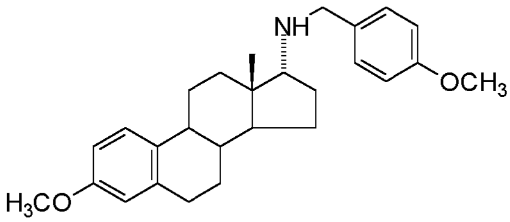 | 17α-(4-Methoxyphenyl-methylamino)-3-methoxy-estra-1,3,5(10)-trien |
| 50 | 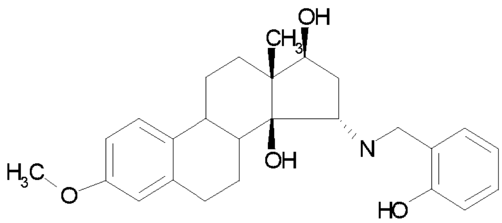 | 14β,17β-Diydroxy-15α-(2-hydroxyphenyl-methylamino)-estra-1,3,5(10)-trien |
| 51 | 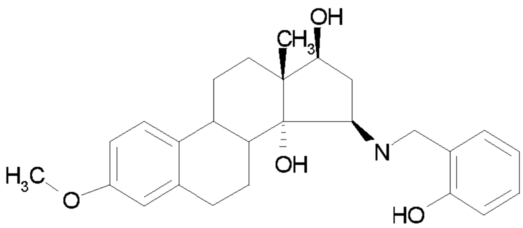 | 14α,17β-Diydroxy-15β-(2-hydroxyphenyl-methylamino)-estra-1,3,5(10)-trien |
| 52 | 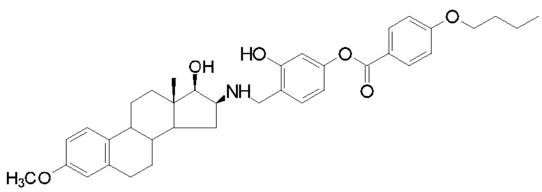 | 16β-{[2-Hydroxy-4-(4'-(4"-n-butoxy-phenyl) carbonyloxy)-phenyl]-methylamino}-17β-hydroxy-3-methoxy-estra-1,3,5(10)-trien |
| 53 | 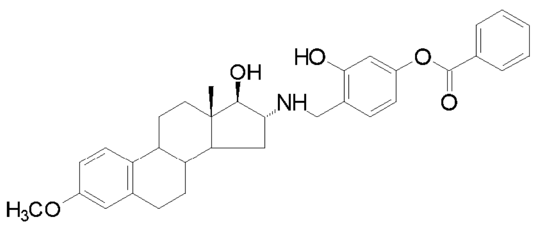 | 16α-{[2-Hydroxy-4-(4'-phenylcarbonyloxy)-phenyl]-methylamino}-17β-hydroxy-3-methoxy-estra-1,3,5(10)-trien |

(fortgesetzt)

| | Formel | Nomenklatur |
|---|---|---|
| 54 | 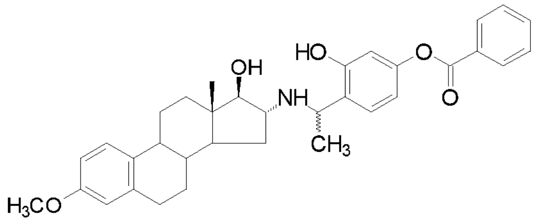 | 16α-{1-[2-Hydroxy-4-(4'-phenylcarbonyloxy)-phenyl]-eth-1-ylamino}-17β-hydroxy-3-methoxy-estra-1,3,5(10)-trien |
| 55 | 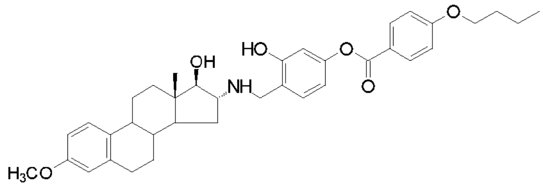 | 16α-{[2-Hydroxy-4-(4'-(4"-n-butoxyphenyl) carbonyloxy)-phenyl]-methylamino}-17β-hydroxy-3-methoxy-estra-1,3,5(10)-trien |
| Ref 1 | 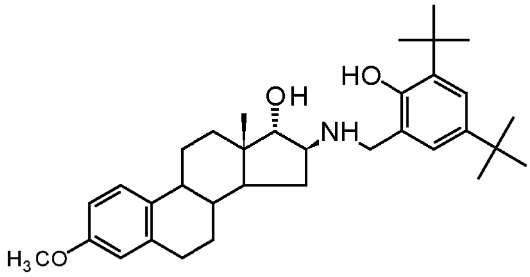 | 16β-(2-Hydroxy-3,5-di-tert-butylphenyl-methylamino)-17α-hydroxy-3-methoxy-estra-1,3,5 (10)-trien |

Ferner umfasst die vorliegende Erfindung Verbindungen gemäß der allgemeinen Formel (I) zur Verwendung in der Medizin und besonders bevorzugt zur Behandlung und/oder Prophylaxe von parasitärer, Infektionen und Erkrankungen.

Es konnte überraschend gezeigt werden, dass die Verbindungen der allgemeinen Formel (I) inhibierend auf die Vermehrung von Chloroquin-resistenten *P. falciparum* Stämmen wirken. Dies konnte sowohl *in vitro* als auch im Mausmodel also *in vivo* gezeigt werden.

Die hormonale Aktivität der Steroidderivate ist durch den eingeführten basischen Stickstoffrest und generell durch die von Steroidhormonen abweichenden Substituentenmuster, die bei Hormonen für deren spezifische Wechselwirkung und Bindung verantwortlich sind, weitestgehend reduziert worden. Dies führt zu geringen Nebenwirkungen. Es konnte von den Erfindern zudem gezeigt werden, dass die Zytotoxizität der Verbindungen der vorliegenden Erfindung sehr gering ist.

Ferner betrifft die vorliegende Erfindung pharmazeutische Zusammensetzungen, welche unter Verwendung mindestens einer erfindungsgemäßen Verbindung oder eines Salzes davon hergestellt wurden.

Neben mindestens einer Verbindung der allgemeinen Formel (I) enthalten die pharmazeutischen Zusammensetzungen einen pharmakologisch verträglichen Träger, Hilfsstoff und/oder Lösungsmittel.

Die pharmazeutischen Zusammensetzungen können in Form von Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen hergestellt und verabreicht werden. Zudem Umfassen die erfindungsgemäßen pharmazeutischen Zusammensetzungen Formulierungen wie Schichttabletten zur kontrollierten und/oder kontinuierlichen Freisetzung des Wirkstoffs sowie Mikroverkapselungen als spezielle Darreichungsform.

Derartige Formulierungen sind unter anderem für die Inhalation oder die intravenöse, intraperitoneale, intramuskuläre, subkutane, mucokutane, orale, rektale, transdermale, topikale, bukkale, intradermale, intragastrale, intrakutane, intranasale, intrabuccale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphat, Calciumsulfat, Talk, Mannitol, Ethylalcohol und dergleichen eingesetzt werden. Puder als auch Tabletten können zu 5 bis 95% aus einem derartigen Träger bestehen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen,beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierter Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, NatriumCarboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Wachse, NatriumCarboxymethylcellulose, Hydroxypropyl-methylcellulose, Polyethylenglycol, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Borsäure und Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Ferner betrifft die vorliegende Erfindung pharmazeutische Zusammensetzungen, welche des weiteren einen antiparasitären, antibakteriellen, antimykotischen und/oder antiviralen Wirkstoff enthalten.

Es ist bevorzugt, wenn die antiparasitären Wirkstoffe, welche in Kombination mit einer Verbindung der allgemeinen Formel (I) verwendet werden, aus der Gruppe umfassend bzw. bestehend aus: Tinidazol, Metronidazol, Melarsoprol, Eflornithin, Rifampin, Amphotericin B, Pentamidin, Natrium-Stiboglukonat, Meglumin, Antimoniat, N-Methyl-Glucaminantimonat, Fluconazol, Artesunat, Quinin, Quinidin, Chloroquin, Atovaquon-Proguanil, Artemether, Artemether-Lumefantrin, Mefloquin, Doxycyclin, Clindamycin, Paromomycin, Atovaquon, Nitazoxanid, Azithromycin, Fumagillin, Paromomycin, Diloxanid, Secnidazol, Ornidazol, lodoquinol, Diloxanid furoate, Clindamycin, Atovaquone, Azithromycin, Diminazen, Praziquantel, Oxamniquinin, Niclosamid, Albendazol, Mebendazole, Thiabendazol, Pyrantel, Diethylcarbamazine, Ivermectin, Selamectin, Doramectin, Abamectin, Mefloquin, Amodiaquin, Artesunat, Artemisinin, Sulfadoxin - Pyrimethamin, Proguanil, Pyrimethamin, Sulfadiazin, Sulfamethoxazol - Trimethoprim (Cotrimoxazol), Suramin, Eflornithin, Melarsoprol, Nifurtimox, Methylenblau, Levamisol und Ivermectin ausgewählt werden. Besonders bevorzugt ist eine Kombination mit Artesunat und/oder Artemisinin.

Es ist bevorzugt, wenn die antimykotischen Wirkstoffe, welche in Kombination mit einer Verbindung der Formel (I) verwendet werden, aus der Gruppe umfassend bzw. bestehend aus: Sertaconazol, Fluconazol, Butoconazol, Chlormidazol, Enilconazol, Fenticonazol, Sulconazol, Naftifidin, Clioquinol, lodoquinol, Rimoprogin, Griseofulvin, Terbinafin, Clotrimazol, Itraconazol, Tioconazol, Miconazol, Miconazolnitrat, Glyceroltriacetat, Tolnaftat, Pyrogallol, Econazol, Posaconazol, Isoconazol, Terconazol, Oxiconazol, Voriconazol, Amphotericin B, Nystatin, Natamycin, Caspofungin, Cilofungin, Flucytosin, Tolciclat, Sulbentin, Haloprogin, Ketoconazol, Ciclopirox, Amorolfin, Bifonazol, Bifonazol/Urea, butenafine/Urea, Urea (Harnstoff), Natriumpropionat, Natriumpyrithion, und Salicylsäure ausgesucht werden.

Es ist zudem bevorzugt, wenn die antibakteriellen Wirkstoffe, speziell Antibiotika, welche in Kombination mit einer Verbindung der Formel (I) verwendet werden, aus der Gruppe umfassend bzw. bestehend aus: Zellwandsynthesehemmern, wie Carbapeneme: Imipenem, Meropenem, Ertapenem, Cephalosporine, Monobactame, Aztreonam, Penicillin, Penicillin G (Benzylpenicillin), Penicillin V (Phenoxymethylpenicillin), Acylaminopenicillin, Piperacillin, Mezlocillin, Aminopenicilline: Ampicillin, Amoxicillin, Isoxazolylpenicilline, Flucloxacillin, Methicillin, Oxacillin, Clavulansäure, Sulbactam, Tazobactam, Sultamicillin, Fosfomycin, Glycopeptide, Teicoplanin, Vancomycin, Bacitracin, Colistin, Gramicidin, Polymyxin B, Tyrothricin; Hemmer der Proteinbiosynthese am Ribosom wie, Aminoglykoside, Amikacin, Gentamicin, Kanamycin, Neomycin, Netilmicin, Streptomycin, Tobramycin, Chloramphenicol, Fusidinsäure, Ketolid, Cethromycin, Narbomycin, Telithromycin, Lincosamid, Clindamycin, Lincomycin, Daptomycin, Streptogramin, Dalfopristin, Quinupristin, Azithromycin, Clarithromycin, Erythromycin, Roxithromycin, Oxazolidinone, Linezolid, Tetracyclin, Doxycyclin, Minocyclin, Oxytetracyclin, Tigecyclin; Gyrase-Hemmer (Hemmer der DNA-Replikation) wie Fluorchinolone, Norfloxacin, Ciprofloxacin, Enoxacin, Ofloxacin, Levofloxacin, Moxifloxacin, Nitroimidazol, Metronidazol, Tinidazol, Aminocumarin; Folsäureantagonisten wie Sulfonamide, Sulfadiazin, Sulfadoxin, Sulfamethoxazol, Sulfasalazin, Diaminopyrimidine, Pyrimethamin, Trimethoprim; Ansamycine (Hemmer der bakteriellen RNA-Polymerase) wie Rifampicin und Hemmer des Stoffwechselwegs wie Fosmidomycin ausgewählt werden.

Es ist ferner bevorzugt, wenn die antiviralen Wirkstoffe, welche in Kombination mit einer Verbindung der Formel (I) verwendet werden, aus der Gruppe umfassend bzw. bestehend aus: Aciclovir, Abacavir, Didanosin, Emtricitabin, Lamivudin, Stavudin, Tenofovir, Zidovudin, Efavirenz, Nevirapin, Atazanavir, Ritonavir, Lopinavir - Ritonavir, Indinavir, Saquinavir, Efavirenz - Emtricitabin - Tenofovir, Zidovudin - Lamivudin, Stavudin - Lamivudin - Nevirapin, Emtricitabin

- Tenofovir, Ribavirin, Zidovudin - Lamivudin - NevirapinSaquinavir, Efavirenz - Emtricitabin - Tenofovir, Emtricitabin - Tenofovir, Stavudin - Lamivudin - Nevirapin, Zidovudin - Lamivudin, Zidovudin - Lamivudin - Nevirapin und Ribavirin ausgesucht werden.

Die erfindungsgemäßen Verbindungen und die oben beschriebenen pharmazeutischen Zusammensetzungen werden zur Behandlung und/oder Prophylaxe von bzw. zur Herstellung einer pharmazeutischen Formulierung zur Behandlung und/oder Prophylaxe von einer Infektionskrankheit eingesetzt, das heißt einer parasitär verursachten Erkrankung.

Bevorzugt werden die erfindungsgemäßen Verbindungen und die oben beschriebenen pharmazeutischen Zusammensetzungen zur Behandlung und/oder Prophylaxe von bzw. zur Herstellung einer pharmazeutischen Formulierung zur Behandlung und/oder Prophylaxe von Erkrankungen verursacht von Sporozoen und weiteren parasitären Infektionen wie beispielsweise hervorgerufen durch: *Babesia, Balantidium, Besnoitia, Blastocystis, Coccidia, Cryptosporidium, Cytauxzoon, Cyclospora, Dientamoeba, Eimeria, Entamoeba, Enterocytozoon, Enzephalitozoon, Eperythrozoon, Giardia, Hammondia, Isospora, Leishmania, Microsporidia, Naegleria, Plasmodium, Pneumocystis, Schistosoma, Sarcocystis, Theileria, Trichinella, Toxoplasma, Trichomonas, Trypanosoma, Unicaria, Cestoda, Dipylidium, Dranunculus, Echinococcus, Fasciola, Fasciolopsis, Taenia, Ancylostoma, Ascaris, Brugia, Enterobius, Loa loa, Mansonella, Necator, Oncocerca, Strongyloides, Strongylus, Toxocara, Toxascaris, Trichuris* oder *Wucheria.* Besonders bevorzugt werden die erfindungsgemäßen Verbindungen zur Behandlung und/oder Prophylaxe von bzw. zur Herstellung einer pharmazeutischen Formulierung zur Behandlung und/oder Prophylaxe von Infektionen hervorgerufen durch Parasiten der Gattung Plasmodium, wie *Plasmodium falciparum, Plasmodium vivax, Plasmodium ovale, Plasmodium malariae* und *Plasmodium knowlesi,* sowie Mischinfektionen dieser Erreger.

Ebenfalls bevorzugt werden die erfindungsgemäßen Verbindungen zur Behandlung und/oder Prophylaxe von bzw. zur Herstellung einer pharmazeutischen Formulierung zur Behandlung und/oder Prophylaxe von Infektionen hervorgerufen durch Parasiten der Gattung Schistosoma, wie *S. mansoni S. haematobium S. japonicum S. intercalatum und S. mekongi,* sowie Mischinfektionen dieser Erreger.

Ebenfalls bevorzugt werden die erfindungsgemäßen Verbindungen zur Behandlung und/oder Prophylaxe von bzw. zur Herstellung einer pharmazeutischen Formulierung zur Behandlung und/oder Prophylaxe von Infektionen hervorgerufen durch Parasiten der Gattung *Trypanosma,* wie *Trypanosoma brucei* und *Trypanosoma cruzi.*

Bevorzugt werden die erfindungsgemäßen Verbindungen und die oben beschriebenen pharmazeutischen Zusammensetzungen zur Behandlung und/oder Prophylaxe von bzw. zur Herstellung einer pharmazeutischen Formulierung zur Behandlung und/oder Prophylaxe von parasitären Erkrankungen aus der Gruppe umfassend bzw. bestehend aus: Trypanosomiasis (afrikanische Schlafkrankheit), Chagas-Krankheiten, Echinococcosis, Amoebiasis (Amöbenruhr), Ancylostomiasis, Babesiose, Balantidiasis, Blastocystis Infektion, Schistosomose, Bilharziose (Schistosomiasis), Kokzidiosen, Kryptosporidiose, Dientamoebiasis, Microsporidiose, Zoonosen, Giardiasis, Isosporiasis, Leishmaniose, *Naegleria* infection, Malaria, Sarcosporidiose, Piroplasmosis, Trichinose, Toxoplasmose, Trochomoniasis, Ascariasis, Filariose, Taeniasis, Echinokokkose, Onchozerkose, Capillariasis Elephantiasis, Enterobiasis, Sarcosporidiose, Strongyloidiasis, Trichuriasis und Zystizerkose. Besonders bevorzugt werden die erfindungsgemäßen Verbindungen zur Behandlung und/oder Prophylaxe von bzw. zur Herstellung einer pharmazeutischen Formulierung zur Behandlung und/oder Prophylaxe von Malaria, insbesondere der Malaria tropica.

Die vorliegende Erfindung betrifft auch ein Verfahren zur Behandlung und Prophylaxe bei einem Patienten, umfassend die Verabreichung einer wirksamen Menge einer erfindungsgemäßen Verbindung der allgemeine Formel (I) ausreichend um besagte Infektionserkrankungen zu behandeln.

Des Weiteren bezieht sich die vorliegende Erfindung auf die Herstellung der erfindungsgemäßen Steroid-Hydroxyarylmethylamine. Die Synthese der Verbindungen der allgemeinen Formel (I) wird bevorzugt nach dem folgenden allgemeinen Schema durchgeführt:

## Schema 1

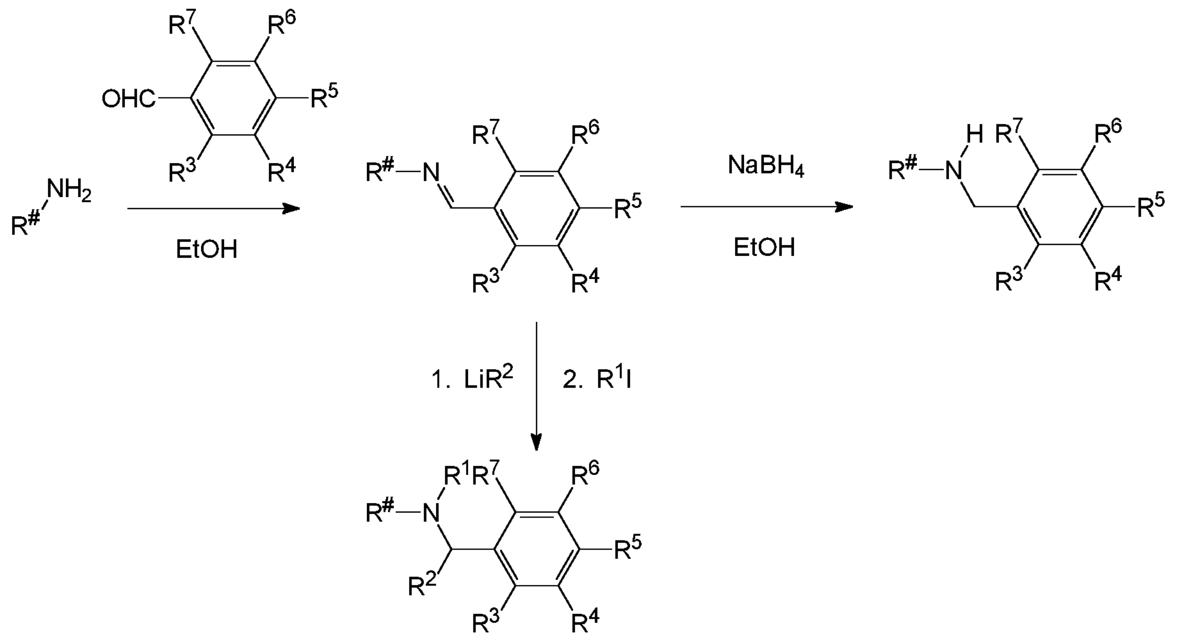


Das Aminogruppen-funktionalisierte Steroid R# wird mit einem entsprechend substituierten Benzaldehyd zu einem Imin umgesetzt, welches mittels Natriumborhydrid oder anderen geeigneten Reduktionsmitteln zum Amin reduziert wird. Alternativ findet im ersten Schritt die Addition eines Nucleophils und anschließend die eines Electrophils and die Imindoppelbindung statt.

Trägt der Steroidrest R# zwei oder mehr Aminogruppen, so kann eine doppelte oder mehrfache Funktionalisierung mit dem Arylmethylamino-Fragment erfolgen und man erhält Verbindungen wie die Referenzverbindungen 13 bis 16.

Alternativ sind die erfindungsgemäßen Steroidhydroxyarylmethylamine auch durch reduktive Aminierung von Steroidketonen oder Aldehyden mit entsprechenden Alternativ sind die erfindungsgemäßen Steroidhydroxyarylmethylamine auch durch reduktive Aminierung von Steroidketonen oder Aldehyden mit entsprechenden primären oder sekundären Arylmethylaminen zugänglich, z.B. mittels $NaBH(OAc_3)$, $NaBH_3CN$, Decaboran, Pyridin-$BH_3$-Addukt mit oder ohne Anwendung katalytischer Lewis-Säure-Zusätze wie beispielsweise $ZnCl_2$ oder $TiCl_4$.

**Figurenbeschreibung:**

Figur 1: je 8 Pärchen von adulten S. *mansoni* nach 48 Stunden Inkubation in reinem Medium (M199) und DMSO-Kontrolle

Figur 2: je 8 Pärchen von adulten S. *mansoni* nach 48 Stunden Inkubation mit 20 μM und 100 μM Verbindung 18

Figur 3: Isobologramme für Verbindung 18 mit Artemisinin und Artesunate Für die Diagramme wurden jeweils $FIC_{90}$-Werte verwendet.

Figur 4: Isobologramme für Verbindung 2 ($FIC_{90}$-Werte) und Verbindung 41 ($FIC_{50}$-Werte) mit Artemisinin

**Beispiele**

**Beispiel 1:** Synthese von 16-Amino-3-methoxy-estra-1,3,5(10)-trien-Vorstufen zu den Referenzverbindungen in Schema 1 ausgehend von kommerziell erhältlichem Estronmethylether

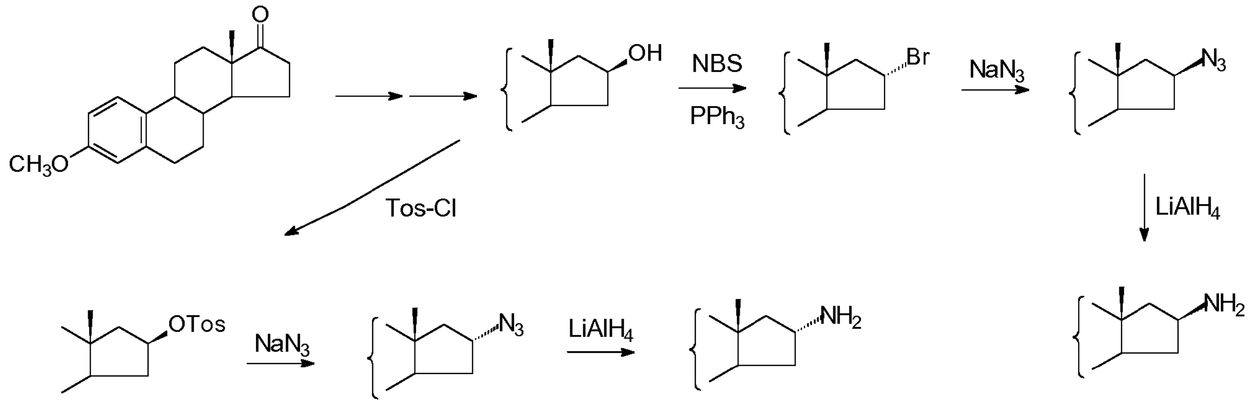


**Beispiel 2:** Synthese von 17-Amino-estra-3-methoxy-1,3,5(10)-trien-Vorstufen zu den erfindungsgemäßen Verbindungen in Schema 1 ausgehend von kommerziell erhältlichem Estronmethylether

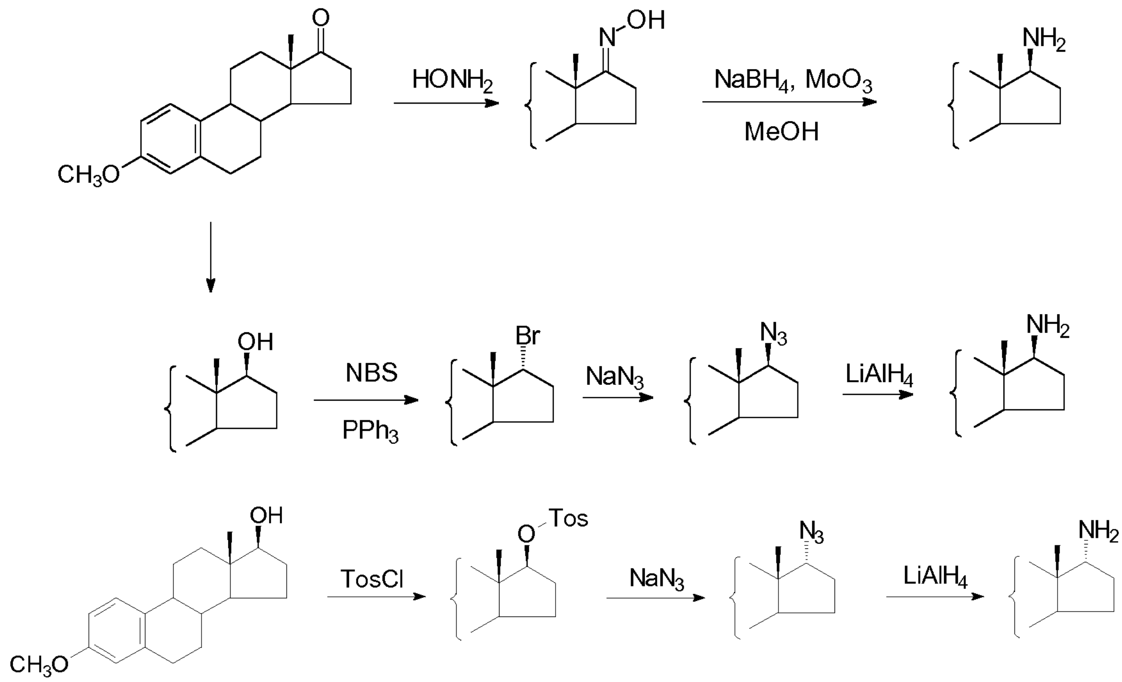


**Beispiel 3:** Synthese von 16-Amino-17-hydroxy-3-methoxy-estra-1,3,5(10)-trien-Vorstufen zu den Referenzverbindungen in Schema 1 ausgehend von literaturbekannten Steroid-Intermediaten

**3.1: 16α-Amino-Reihe**

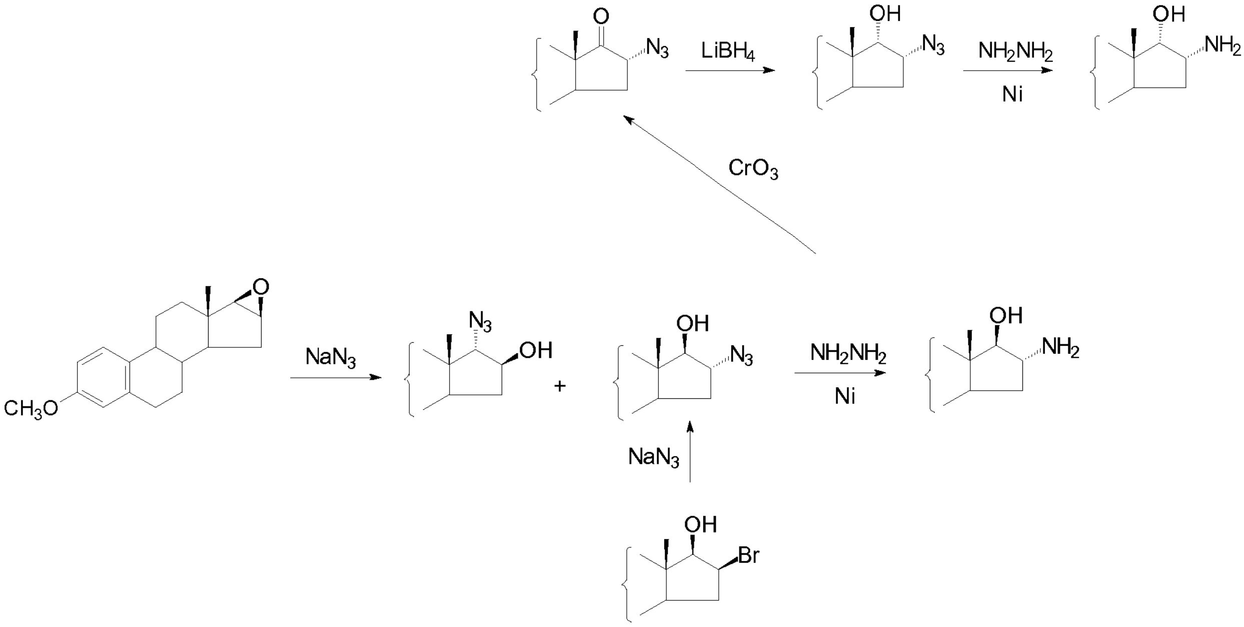


**3.2: 16β-Amino-Reihe**

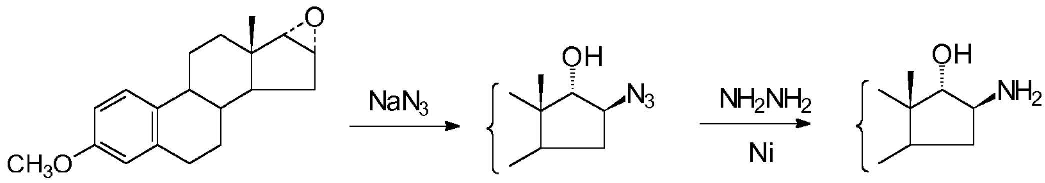


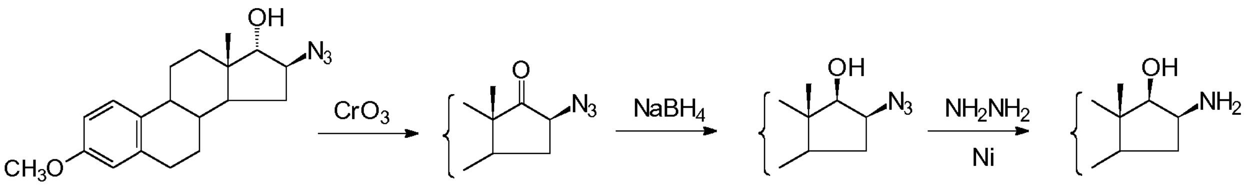


**Beispiel 4:** Synthese der Amino-Cholan-Vorstufen zu den Referenzverbindungen in Schema 1 ausgehend von literaturbekannten Steroid-Intermediaten

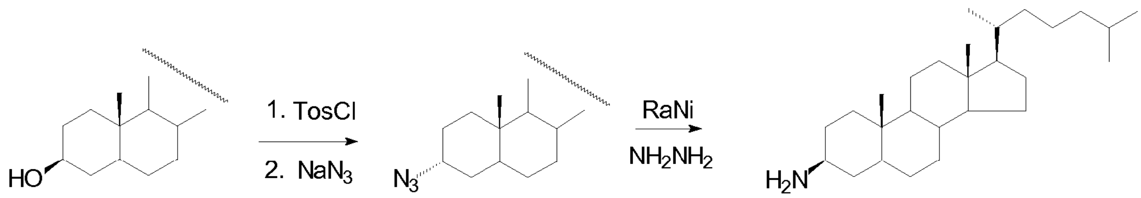

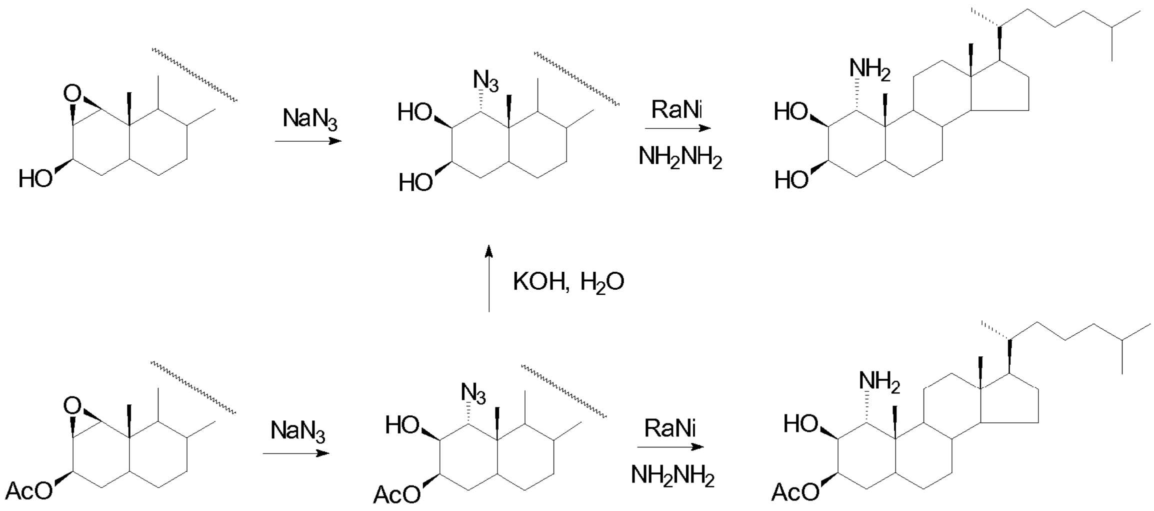


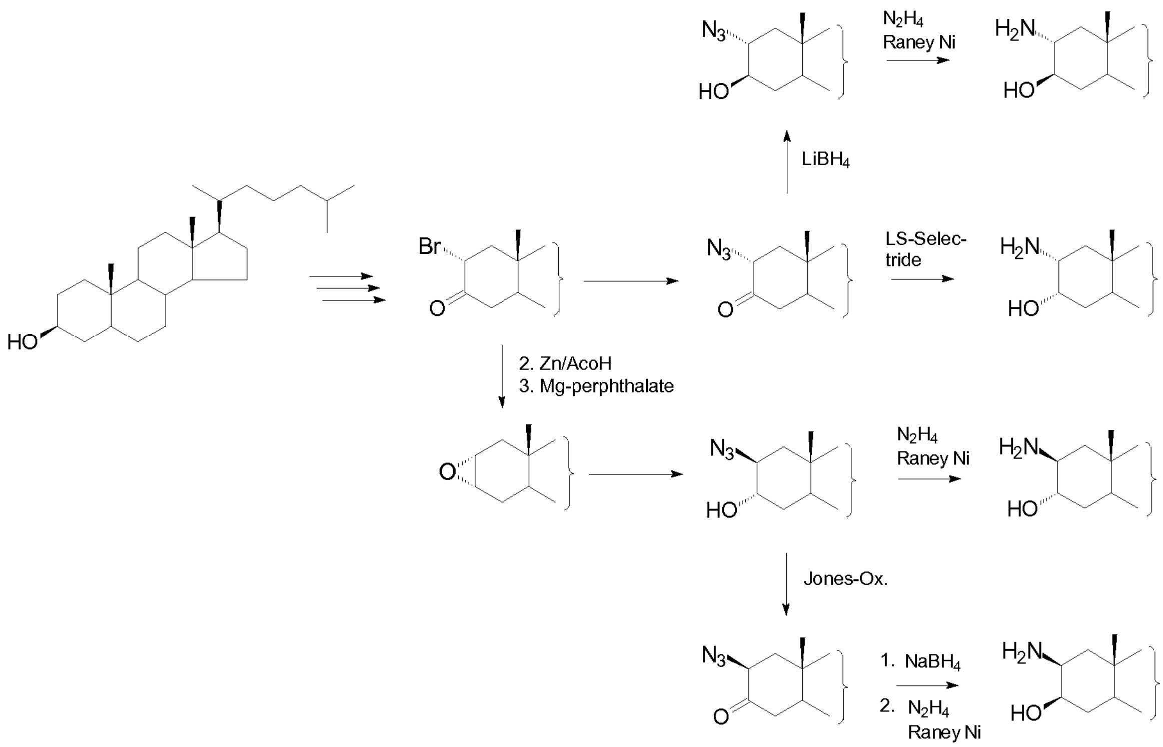


**Beispiel 5:** Synthese der 13-Epi-Estratrien-Vorstufen zu den Referenzverbindungen in Schema 1 ausgehend von literaturbekannten Steroid-Intermediaten

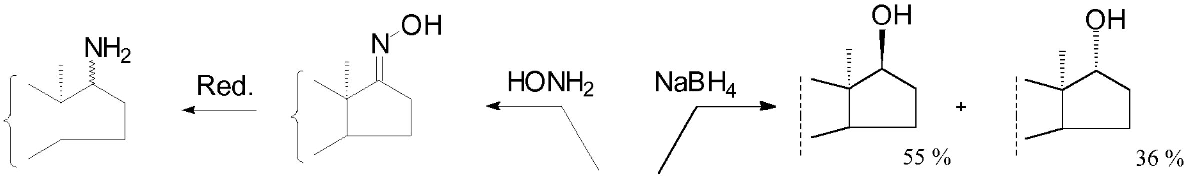

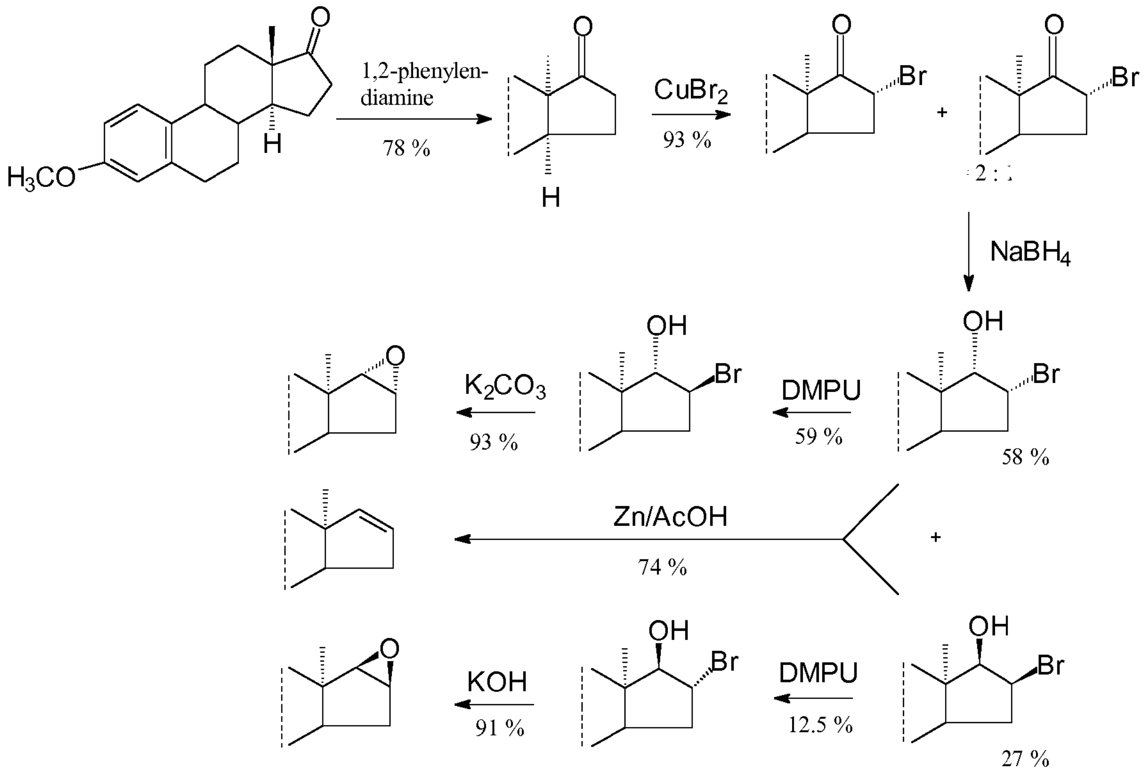
1,2-phenylen-
diamine
78 %
CuBr2
93 %
+
2 : 1
NaBH4
DMPU
59 %
K2CO3
93 %
58 %
Zn/AcOH
74 %
+
DMPU
12.5 %
KOH
91 %
27 %

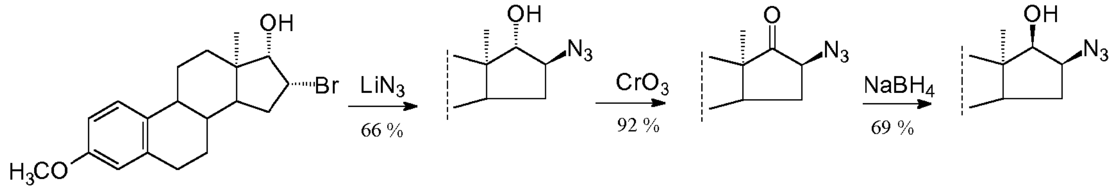
LiN3
66 %
CrO3
92 %
NaBH4
69 %

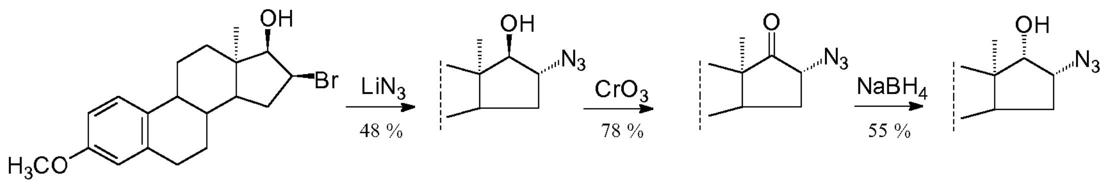
LiN3
48 %
CrO3
78 %
NaBH4
55 %

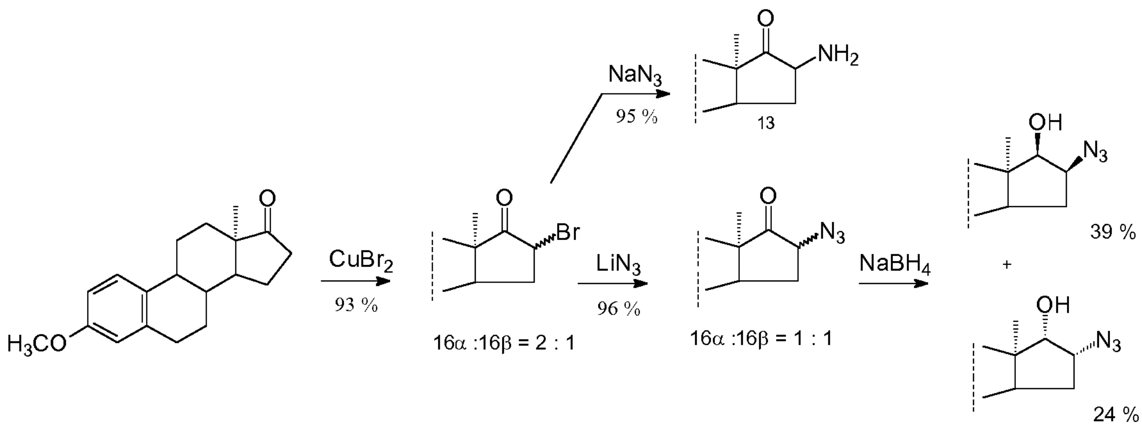
NaN3
95 %
13
CuBr2
93 %
16α :16β = 2 : 1
LiN3
96 %
16α :16β = 1 : 1
NaBH4
39 %
+
24 %

**Beispiel 6:** Bestimmung von $IC_{50}$-Werten an Plasmodium falciparum *in vitro*

Kultivierung von *Plasmodium falciparum zur Bestimmung der antiparasitären Wirkung der* Steroidbenzylamin-Derivate

Der Chloroquin-sensitive *P. falciparum* Stamm 3D7-Niederlande und der Chloroquin-sensitive *P. falciparum* Stamm K1 wurden nach international anerkannten und mehrfach publizierten Standards in kontinuierlichen Kultur gehalten, wie von unserer Gruppe u.a. in Akoachere et al. (In Vitro Assessment of Methylene Blue on Chloroquine-Sensitive and-Resistant Plasmodium falciparum Strains Reveals Synergistic Action with Artemisinins; Antimicrobial Agents and Chemotherapy, 2005, Vol. 49, 4592 - 4597) beschrieben. Die Parasiten wurden bei 1 bis 10% Parasitämie und 3,3% Hämatokrit in RPMI 1640 Kulturmedium (Gibco, Invitrogen), welches mit A+ Erythrozyten, 0,5% Rinderserumalbumin (Albumax, Invitrogen), 9 mM (0,16%) Glukose, 0,2 mM Hypoxanthin, 2,1 mM L-Glutamin und 22 μg/ml Gentamicin angereichert war, kultiviert. Alle Inkubationen wurden bei 37°C in einem Heraeus/Thermo Scientific Cytoperm 2-Inkubator unter konstanter Begasung mit 3% $O_2$, 3% $CO_2$ und 94% $N_2$ durchgeführt. Die Synchronisation der Parasiten in Kultur zum Ring-Stadium als Ausgangspopulation wurde durch wiederholte Behandlung der parasitierten Erythrozyten mit 5% (w/v) Sorbitol (jeweils 3 Minuten bei 37°C), gefolgt von Zentrifugation (3 Minuten bei 900 x g, Raumtemperatur) und 1 x Waschen mit dem oben genannten angereicherten RPMI-Medium durchgeführt. Nach nochmaliger Zentrifugation (3 Minuten, 900 x g) wurden die Parasiten in 10 ml Medium aufgenommen (Hämatokrit 5%). Die erhaltenen Parasiten im Ringstadium wurden für 48 h (1 Entwicklungszyklus) inkubiert und dann für die Testung der Steroidbenzylamin-Derivate eingesetzt.

Bestimmung von $IC_{50}$-Werten *in vitro*

Ein radioaktiver Sensitivitäts-Assay wurde eingesetzt, um die Empfindlichkeit von *P. falciparum* für verschiedene Verbindungen der allgemeinen Formel (I) zu bestimmen. Das Verfahren basiert auf der Inkorporation von radioaktivem $^3$H-Hypoxanthin, welches durch den Parasiten als Vorläufer für Purin-Desoxynukleotide für die DNA-Synthese aufgenommen wird. In 96-well Mikrotiterplatten für Suspensionskultur (Greiner) wurde eine Verdünnungsreihe (insgesamt 8 serielle Verdünnungen, um jeweils Faktor 2) der Ausgangskonzentration (50 ng/ml) der pharmakologisch aktiven Verbindung der Formel (I) durchgeführt. Die Parasiten wurden bei einer Parasitämie von 0,1% (>90% Ringstadien) und 1,25% Hämatokrit zunächst für 48 h in Hypoxanthin-freiem Medium (Gesamtvolumen 200 μl) mit den Hemmstoffen inkubiert. Danach wurden 0,5 μCi $^3$H-Hypoxanthin in jede Vertiefung zugegeben und die Platten wurden für weitere 24 Stunden inkubiert. Die Zellen jedes wells wurden dann auf einem Glasfaserfilter (Perkin-Elmer, Rodgau-Jügesheim, Deutschland) geerntet, mit destilliertem Wasser gewaschen und im Wärmeschrank (60-70°C, 1 h) getrocknet. Ihre Radioaktivität in counts per minute wurde auf einem Betacounter gemessen und war proportional zur Anzahl der vor der Ernte überlebensfähigen Parasiten auf der Glasfaserplatte. Alle $IC_{50}$-Werte wurden vierfach bestimmt. Bei jedem Ansatz wurde eine Chloroquin-Kontrolle mitgeführt.

Ergebnis

Die Referenzverbindung 1a hat eine in vitro Aktivität von $IC_{50}$ = 73 nM an dem Chloroquin-resistenten *Plasmodium falciparum Stamm* K1 und 50 nM in Bezug auf 3D7 (siehe Tabelle 1). Die Referenzverbindung hat eine geringe Zytotoxizität von $IC_{50}$ = 11 μM (SI = 217). Die Referenzverbindungen 3 und 4 weisen eine vergleichbare Aktivität auf. Interessanterweise war jedoch Referenzverbindung 2 an dem Stamm 3D7 mit einem $IC_{50}$ -Wert von 7.4 nM (SI = 1071) sehr viel wirksamer.

Tabelle 1: *In vitro* Aktivitäten

| **Verbindung** | **$IC_{50}$** | **$IC_{50}$** | **$IC_{50}$ [μg/ml]** | | |
|---|---|---|---|---|---|
| | **[nM]** | **[ng/ml]** | **Antiproliferative Aktivität** | | **Zytotoxizität** |
| | **P.f .(3D7)** | | **L929** | **K562** | **HeLa** |
| **1a** | 50 | 19.8 | 2.2 | 0.8 | 4.3 |
| **2** | 7.4 | 2.9 | 1.9 | 0.5 | 3.1 |
| **3** | 62 | 24.5 | 1.9 | 0.7 | 4.4 |
| **4** | 41.5 | 16.4 | 1.6 | 1.3 | 4.7 |
| **5** | 879 | 578 | - | - | - |

(fortgesetzt)

| Verbindung | $IC_{50}$ | $IC_{50}$ | $IC_{50}$ [$\mu$g/ml] | | |
|---|---|---|---|---|---|
| | [nM] | [ng/ml] | Antiproliferative Aktivität | | Zytotoxizität |
| | P.f .(3D7) | | L929 | K562 | HeLa |
| **6** | 440 | 172 | - | - | - |
| **7** | 115 | 45 | - | - | - |
| **17** | 72.8 | 32.1 | - | - | - |
| **18** | 7,0 | 3,1 | | | |
| **19** | 182 | 98.60 | - | - | - |
| **20** | 733 | 287 | - | - | - |
| **21** | 465 | 237 | - | - | - |
| **23** | 716.10 | 331.3 | - | - | - |
| **26** | 11.0 | 4.7 | | | |
| **27** | 62.1 | 26.4 | | | |
| **28** | 114 | 49.6 | | | |
| **29** | 42 | 19.8 | | | |
| **30** | 210 | 85.5 | | | |
| **31** | 625 | 265 | | | |
| **32** | 29.9 | 13.7 | | | |
| **33** | 919 | 389 | | | |
| **32** | 29.9 | 13.7 | | | |
| **33** | 919 | 389 | | | |
| **34** | 54.6 | 24.2 | | | |
| **36** | 42.1 | 19.3 | | | |
| **37** | 99.2 | 43.9 | | | |
| **38** | 208 | 106 | | | |
| **41** | 4,4 | 1,9 | | | |
| **42** | 227 | 91.3 | - | - | - |
| **43** | 58.5 | 23.9 | 2.0 | 1.2 | 7.7 |
| **44** | 675.5 | 275 | 2.5 | 0.85 | 3.4 |
| **45** | 101 | 41 | - | - | - |
| **46** | 998 | 393 | - | - | - |
| **47** | 140.5 | 57.3 | - | - | - |
| **48** | 83.3 | 34.0 | - | - | - |
| **Ref 1** | 1608 | 836 | | | |

**Beispiel 7:** Bestimmung von $IC_{50}$-Werten an *Plasmodium berghei* im Mausmodell (*in vivo*)

Um die Wirksamkeit der Steroidbenzylamin-Derivate *in vivo* zu testen, wurden die Referenzverbindungen 1 und 2 in einem Mausmodel unter *Plasmodium berghei*-Infektion untersucht (s. auch Peters und Robinson, Handbook of Animal Models in Infection; Chapter 92, Malaria, Academic Press, London, United Kingdom, 1999 und Sturm et al.

Compounds Structurally Related to Ellagic Acid Show Improved Antiplasmodial Activity, Antimicrobial Agents and Chemotherapy, 2009, Vol. 53, 622-630). Diese Experimente wurden am Schweizer Tropeninstitut in Basel unter der Leitung von Dr. Sergio Wittlin nach internationalen Standards durchgeführt. Einen Tag nach Infektion der Tiere mit *Plasmodium berghei* GFP MRA-865 wurden die Substanzen gelöst in Tween/Alkohol entweder subkutan oder oral mit jeweils einer Dosis pro Tag an den drei Folgetagen nach der Infektion appliziert. Subkutan wurden 3 x 10 mg/kg oder 3 x 30 mg/kg gegeben, oral 3 x 10 mg/kg, 3 x 30 mg/kg oder 3 x 100 mg/kg. An Tag 4 wurden Giemsa-gefärbte Blutausstriche der Mäuse auf ihre Parasitämie hin untersucht (Zählen parasitierter Erythrozyten unter dem Lichtmikroskop). Als weiterer Parameter wurde die Überlebensdauer bestimmt. Die infizierten aber unbehandelten Mäuse wurden aufgrund schwerster Symptomatik an Tag 4 getötet.

Ergebnis:

Referenzverbindungen 1 und 2 zeigten die in Tabelle 2 aufgeführten in vivo Aktivitäten. Die Substanzen wirkten sowohl nach subkutaner als auch nach oraler Applikation auf *Plasmodium berghei* Blutstadien in der Maus, was am deutlichen und Dosis-abhängigen Absinken der Parasitämie - im Vergleicht zu unbehandelten Kontrolltieren - zu erkennen war. Alle Dosierungen und Applikationswege führten des weiteren zu einer deutlichen Lebensverlängerung.

Tabelle 2: *In vivo* Aktivität (angegeben in % der Parasitämie unbehandelter Kontrollen (n=5)) der Referenzverbindungen 1 und 2 im Vergleich zu Chloroquin. (sk = subkutan; ip = intraperetoneal; MSD = mittlere Überlebensdauer).

| Verabreichung / Dosis | MSD (Tage) *Referenz-Verbindung* **1** | Aktivität (%) *Referenz-Verbindung* **1** | MSD (Tage) *Referenzverbindung* **2** | Aktivität (%) *Referenz-Verbindung* **2** | MSD (Tage) Kontrolle | MSD Chloroquin ip 3x10 mg/kg | Aktivität (%) Chloroquin |
|---|---|---|---|---|---|---|---|
| sk 3x10 mg/kg | 10.2 (n=3) | 47 | 7 (n=3) | 17.7 | 4 (euth.) | 13.6 | 99.6 |
| sk 3x30 mg/kg | 15 (n=3) | 94 | 14 (n=3) | 61.8 | 4 (euth.) | 13.6 | 99.6 |
| oral 3x10 mg/kg | nd | nd | 8.7 (n=3) | 46.8 | nd | nd | nd |
| oral 3x30 mg/kg | nd | nd | 13 (n=3) | 99.4 | nd | nd | nd |
| oral 3x100 mg/kg | 16.3 (n=3) | 99 | 15.33 (n=3) | 99.7 | 4 (euth.) | 13.6 | 99.6 |

**Beispiel 8:** Herstellung und Charakterisierung der Verbindungen

**Experimentelles - allgemeine Angaben zu den Ausführungsbeispielen**

Schmelzpunkte (korrigiert): Mikroheiztisch nach BOËTIUS. - EA: Foss-Heraeus CHN-O-RAPID oder Mikroanalyse nach Knobloch, Angaben von M in g/mol. - Massenspektren: AMD 402, EI. - IR: Shimadzu IR-470; Angaben für v in $cm^{-1}$. - UV (in Acetonitril): Specord M-40 (Carl Zeiss Jena). - Spezifische Drehwerte: Polamat A (Carl Zeiss Jena) oder PROPOL DIGITAL Automatik-Polarimeter (Dr. Kernchen), gemessen bei Raumtemp. in Chloroform (c =1), sofern nicht anders angegeben; Angaben für c in g $100^{-1}$ $ml^{-1}$. - $^1H$- und $^{13}C$-NMR: Bruker AC 200, WP 200 SY und AMX 400; in $CDCl_3$, TMS als interner Standard; $\delta$ in ppm; gemessen in $CDCl_3$ bei 250 MHz ($^1H$), sofern nicht anders angegeben. - DC: Merck-Aluminium-Fertigplatten Kieselgel 60 $F_{254}$ (KG) oder Fluka-Aluminium-Fertigplatten ($Al_2O_3$), Detektion im UV oder durch Besprühen mit einer Mischung von 80 ml konz. Schwefelsäure, 20 ml Ethanol, 200 mg Vanillin und anschließender Sichtbarmachung bei 170 °C; Laufmittel: Essigester/n-Hexan (1 : 2), soweit nicht anders angegeben - Die benutzten Lösungsmittel und flüssigen Reagenzien werden, soweit nicht kommerziell erhältlich, nach üblichen Methoden gereinigt und getrocknet. Die Kondensationen mit Arylaldehyden wird vorteilhafterweise unter Argon und Feuchtigkeitsausschluß durchgeführt. Rohprodukte werden bei ≈ 5 Torr mehrere Stunden über Phosphorpentoxid bei Raumtemperatur getrocknet.

Im Folgenden werden repräsentative Beispiele und Daten aufgeführt.

**Darstellung der Hydroxyarylmethylamino-Steroide - am Schema der Kondensation von Amino-Steroiden mit Carbonylverbindungen zu Azomthinen und deren anschließender Reduktion entsprechend Beispiel 1**

Allgemeine Arbeitsvorschrift zur Kondensation von Amino-Steroiden mit Carbonylverbindungen zu Azomethinen

1 mmol Steroid-Aminoalkohol wird in 15 ml abs. Methanol unter Erwärmen gelöst und mit 1.1 mmol der Carbonylverbindung versetzt. Es wird 30 min bei 60 °C gerührt und nach Abkühlen auf Raumtemperatur wird das ausgefallene Reaktionsprodukt abgesaugt, mit Methanol gewaschen, getrocknet und vorteilhafterweise direkt weiterverarbeitet. Nicht präzipitierende Reaktionsprodukte können auch ohne Aufarbeitung wie unten beschrieben mit $NaBH_4$ zu den entsprechenden Steroid-Arylaminen reduziert werden. Alternativ kann auch in Ethanol gearbeitet werden. Bei reaktiven Aldehyden wie z.B. Salicylaldehyd arbeitet man bei Raumtemperatur.

Allgemeine Arbeitsvorschrift - am Beispiel der Darstellung von 16β-(2-Hydroxyphenyl-carbaldimino)-estra-3, 17β-diol-3-methylether:

3 mmol (904.2 mg) 16ß-Amino-estra-3,17β-diol-3-methylether werden in 40 ml absolutem Methanol gelöst und unter Rühren bei Raumtemperatur mit 3.3 mmol (403 mg, 346 μl) frisch destilliertem Salicylaldehyd versetzt. Nach ca. 3 Stunden wird auf 50 % des Ausgangsvolumens eingeengt und tiefgekühlt. Nach absaugen, waschen mit Methanol und Trocknung werden 1.3 g (97 %) 16β-(2-Hydroxyphenylmethylenimino)-estra-3,17β-diol-3-methylether in Form eines leuchtend zitronengelben, feinkristallinen Produktes erhalten ($^1H$-NMR: praktisch einheitlich, kein Signal bei 5.6 ppm). Die Umkristallisation erfolgt durch Lösen in ca. 30 ml siedenden Essigester und anschließender Zugabe von 15 ml heißem Heptan: 1.0 g (75 %), leuchtend gelbe Nadeln oder Säulen vom Schmp. 221-224 °C. $C_{26}H_{31}NO_3$ (405,56), Ber: C 76.99, H 7.71, N 3.47, Gef: C 77.01, H 7.71, N 3.75, $[\alpha]_D$ + 47.4°.
$^1H$-NMR: 0.90 (s, 3H, 18-$H_3$), 1.13-2.31 (m, überl., 12H), 2.79 (m, 2H, 6-$H_2$), 3.66 (t, $^3J$ = 8.5 Hz, 1H, 17a-H), 3.70 (s, 3H, 3-MeO), 3.84 (m, 1H, 16αH), 6.55-7.25 (m, überl., 7H, 1-H, 2-H, 4-H und Phenyl-$H_4$), 8.30 (s, 1H, N=CH).
$^{13}C$-NMR ($CDCl_3$): 12.1 (C-18), 34.4 (C-15), 37.1 (C-12), 43.4 (C-13), 47.9 (C-14), 69.0 (C-16), 55.5 ($OCH_3$), 82.9 (C-17), 166.3 (N=CH).

Auf diese Weise werden beispielsweise erhalten:
17β-(4-N,N-Diethylamino-2-hydroxyphenyl-carbaldimino)-3-methoxy-estra-1,3,5(10)-trien (**24**):
erhalten durch Umsetzung von 17β-Amino-3-methoxy-estra-1,3,5(10)-trien) und 4-(N,N-Diethylamino)salicylaldehyd in abs. Ethanol.
$C_{30}H_{40}N_2O_2$ (460.65), F = 234.5-235.5 °C (aus EtOH), $R_f$ = 0.48.
$^1H$-NMR: 0.890 (s, 3H, 18-$H_3$), 1.190 (t, $^3J$ = 7.0 Hz, 6H, 2x$CH_3$-$CH_2$), 1.200-2.311 (m, überl., 13H), 2.862 (s, breit, 2H, 6-$H_2$), 3.173 (t, $^3J$ = 8.7 Hz, 1H, 17a-H), 3.373 (q, $^3J$ = 7.0 Hz, 4H, 2x$CH_3$-$CH_2$), 3.780 (s, 3H, 3-MeO), 6.084 (s, 1H, Phenyl-H), 6.138 (d, $^3J$ = 8.4 Hz, 1H, Phenyl-H), 6.628 (d, $^3J$ = 8.4 Hz, 1H, Phenyl-H), 6.643 (s, 1H, 4-H), 6.69 (d, $^3J$ = 8.6 Hz, 1H, 2-H), 7.204 (d, $^3J$ = 8.6 Hz, 1H, 1-H), 7.957 (s, 1H, CH=N), 14.260 (s, breit, 1H, OH).
$^{13}C$-NMR: 12.70, 12.75, 24.02, 25.60, 26.13, 27.61, 29.66, 29.84, 36.88, 38.86, 43.95, 44.49, 44.74, 52.23, 55.19, 76.87, 77.20, 98.65, 102.90, 108.36, 111.44, 113.80, 126.30, 132.58, 132.69, 137.94, 151.76, 157.45, 161.03, 168.04.

17β-(3-Hydroxy-naphth-2-carbaldimino)-3-methoxy-estra-1,3,5(10)-trien (**25**):

erhalten durch Umsetzung von 17β-Amino-3-methoxy-estra-1,3,5(10)-trien) und 3-Hydrox-naphth-2-carbaldehyd in abs. Ethanol.

$C_{30}H_{33}NO_2$ (339.59), F = 234.5-236.5 °C (aus EtOH/THF = 1:1), $R_f$ = 0.76. $^1$H-NMR (ausgewählte Daten): 0.960 (s, 3H, 18-$H_3$), 2.880 (s, breit, 2H, 6-$H_2$), 3.296 (t, $^3J$ = 8.6 Hz, 1H, 17a-H), 3.782 (s, 3H, 3-MeO), 6.661 (m, 1H, 4-H), 6.780 (d, $^3J$ = 7.5 Hz, 1H, 2-H), 7.264 (d, $^3J$ = 7.5 Hz, 1H, 1-H), 7.304-7.769 (m, ca. 6H, 6x Naphthyl-H), 8.530 (s, 1H, N=CH), 13.303 (s, breit, 1H, OH).

$^{13}$C-NMR: 13.11, 24.37, 26.13, 27.71, 29.85, 30.06, 36.98, 38.79, 43.94, 45.45, 52.53, 55.20, 80.09, 110.84, 111.47, 113.84, 121.30, 123.30, 126.31, 127.29, 127.92, 128.31, 132.51, 132.57, 133.75, 137.94, 157.11, 157.49, 163.14.

17α-(3-Hydroxy-naphth-2-carbaldimino)-3-methoxy-estra-1,3,5(10)-trien:

erhalten durch Umsetzung von 17α-Amino-3-methoxy-estra-1,3,5(10)-trien) und 3-Hydrox-naphth-2-carbaldehyd in abs. Ethanol.

$C_{30}H_{33}NO_2$ (339.59), F = 246-248 °C (aus EtOH/THF = 1:1), $R_{f.}$ = 0.82.

17β-[(E)-Salicylidenimino)-3-methoxy-estra-1,3,5(10)-trien:

erhalten durch Umsetzung von 17β-Amino-3-methoxy-estra-1,3,5(10)-trien) und Salicylaldehyd in abs. Ethanol.

$C_{26}H_{31}NO_2$ (389.54), F = 85-88 °C (aus MeOH), $R_f$ = 0.56, $[\alpha]_D$ +105.8 ° (c = 9.837). $^1$H-NMR: 0.864 (s, 3H, 18-$H_3$), 2.876 (s, breit, 2H, 6-$H_2$), 3.222 (t, $^3J$ = 7.5 Hz, 1H, 17α-H), 3.764 (s, 3H, 3-MeO), 6.672 (m, 2H, 2- and 4-H), 6.910 (m, 2H, 3- and 5-Phenyl-H), 7.256 (m, 3H, 1-H und 4-H und 6-Phenyl-H), 8.273 (s, 1H, N=CH), 13.866 (s, 1H, 2-Phenyl-OH).

$^{13}$C-NMR: (incl. DEPT 135): 13.0 (18-$CH_3$), 24.3 (15-C), 26.1 ((11-C), 27.7 (7-C), 29.8 (6-C), 29.9 (12-C), 36.9 (16-C), 55.2 ($CH_3O$), 79.6 (17-C), 111.4 (2-C), 113.9 (4-C), 126.3 (1-C), 132.5 (10-C), 137.9 (5-C), 157.4 (3-C), 163.1 (CH=N); Phenyl-C: 117.0, 118.3, 118.9, 131.0, 132.0, 161.5.

IR: 1630 (s, C=N Valenz); 1610 (m), 1579 (w), und 1497 (s, arom. C=C).

17α-[(E)-Salicylidenimino)-3-methoxy-estra-1,3,5(10)-trien:

erhalten durch Umsetzung von 17α-Amino-3-methoxy-estra-1,3,5(10)-trien) und Salicylaldehyd in abs. Ethanol.

$C_{26}H_{31}NO_2$ (389.54), F = 161-162.5 °C (aus MeOH), $[\alpha]_D$ -116.3 ° (c = 8.432).

$^1$H-NMR: 0.864 (s, 3H, 18-$H_3$), 2.855 (m, breit, 2H, 6-$H_2$), 3.357 (d, J = 6.6 Hz, 1H, 17ß-H), 3.760 (s, 3H, 3-MeO), 6.662 (m, 2H, 2- und 4-H), 6.889 (m, 2H, 3- und 5-Phenyl-H), 7.254 (m, 3H, 1-H und 4-H und 6-Phenyl-H), 8.246 (s, 1H, N=CH), 13.754 (s, 1H, 2-Phenyl-OH).

$^{13}$C-NMR: (incl. DEPT 135): 18.5 (18-$CH_3$), 25.1 (15-C), 26.4 ((11-C), 28.1 (7-C), 30.0 (6-C), 31.7 (12-C), 33.4 (16-C), 55.2 ($CH_3O$), 78.7 (17-C), 111.5 (2-C), 113.8 (4-C), 126.4 (1-C), 132.0 (10-C), 138.0 (5-C), 157.4 (3-C), 162.0 (CH=N); Phenyl-C: 116.9, 118.5, 118.8, 131.1, 132.8, 161.0.

16β-[(E)-Salicylidenimino]-3-methoxy-estra-1,3,5(10)-trien:

erhalten durch Umsetzung von 16β-Amino-3-methoxy-estra-1,3,5(10)-trien) und Salicylaldehyd in abs. Methanol.

$C_{26}H_{31}NO_2$ (389.54), Ausbeute 90 %, F = 181-183 (aus Methanol), $[\alpha]_D$ + 24.6 ° (c = 7.315), $R_f$ = 0.74.

$^1$H-NMR: 1.02 (s, 3H, 18-$H_3$), 3.76 (s, 3H, $OCH_3$), 3.88 (m, 1H, 16α-H), 6.61-7.30 (7H, Ar-H), 8.25 (s, 1H, CH=N), 13.70 (s, 1H, -XH).

$^{13}$C-NMR: 19.1 (C-18), 26.5 (C-11), 28.1 (C-7), 29.8 (C-6), 35.9 (C-15), 38.8 (C-12), 38.6 (C-8), 41.4 (C-13), 43.9 (C-9), 50.0 (C-17), 53.6 (C-14), 55.2 ($OCH_3$), 67.1 (C-16), 111.5 (C-2), 113.8 (C-4), 126.2 (C-1), 132.7 (C-10), 137.8 (C-5), 157.4 (C-3), 162.1 (CH=N); Ar-C: 116.9, 118.4, 118.8 (q), 131.0, 131.9, 161.1 (q).

Ber: C 80.16, H 8.02, N 3.67; Gef: 80.39, 8.39, 3.67.

IR: 1630 (CH=N), 1582, 1503.

16α-[(E)-Salicylidenimino]-3-methoxy-estra-1,3,5(10)-trien:

erhalten durch Umsetzung von 16a-Amino-3-methoxy-estra-1,3,5(10)-trien) und Salicylaldehyd in abs. Methanol.

$C_{26}H_{31}NO_2$ (389.54), Ausbeute 88 %, F = 118-120 (aus Methanol), $[\alpha]_D$ + 126.4 ° (c = 8.027), $R_f$ = 0.77.

$^1$H-NMR: 0.84 (s, 3H, 18-$H_3$), 3.76 (s, 3H, $OCH_3$), 3.97 (m, 1H, 16β-H), 6.61-7.30 (7H, Ar-H), 8.27 (s, 1H, CH=N), 13.68 (s, 1H, -XH).

$^{13}$C-NMR: 18.7 (C-18), 26.4 (C-11), 28.0 (C-7), 29.6 (C-6), 35.5 (C-15), 38.4 (C-12), 38.9 (C-8), 42.3 (C-13), 43.8 (C-9), 50.5 (C-17), 52.1 (C-14), 55.2 ($OCH_3$), 66.9 (C-16), 111.4 (C-2), 113.8 (C-4), 126.2 (C-1), 132.7 (C-10), 137.9 (C-5), 157.4 (C-3), 162.0 (CH=N); Ar-C: 116.9, 118.4, 118.8 (q), 131.0, 131.8, 161.1 (q).

Ber: C 80.16, H 8.02, N 3.67; Gef: 80.01, 8.09, 3.77.

17β-Hydroxy-16α-(E)-salicylidenimino-3-methoxy-estra-1,3,5(10)-trien:

erhalten durch Umsetzung von 16α-Amino-17β-hydroxy-3-methoxy-estra-1,3,5(10)-trien) und Salicylaldehyd in abs. Methanol.

$C_{26}H_{31}NO_3$ (405,56). Ber: C 76.99, H 7.71, N 3.47, Gef: C 76.82, H 7.87, N 3.54. Umkristallisiert wird aus Methanol, Ausbeute: 89 %, F = 169-172 °C, $[\alpha]_D$ + 81,7° (c = 0.969).

$^1$H-NMR: 0.89 (s, 3H, 18-$H_3$); 3.63 (m, 1H, 16β-H); 3.70 (d, $^3J$ = 6.5 Hz, 1H,
17a-H); 3.77 (s, 3H, $OCH_3$); 6.62 (d, $^4J$ = 2.8 Hz, 1H, 4-H); 6.70 (dd, $^3J$ = 8.5 Hz, $^4J$ = 2.8 Hz, 1H, 2-H); 6.86 (m, 1H, $C_6H_4$); 6.94 (m, 1H, Ar-H); 7.17-7.32 (m, 3H, 1-H und Ar-H); 8.31 (s, 1H, N=CH).

$^{13}$C-NMR: 12.23 (s, C-18), 25.97 (s, C-11), 27.11 (s, C-7), 29.68 (s, C-6), 32.78 (s, C-15), 36.49 (s, C-12), 38.42 (s, C-8), 43.87 (s, C-9), 43.99 (s, C-13), 48.36 (s, C-14), 55.15 (s, $OCH_3$), 74.64 (s, C-16), 87.91 (s, C-17), 111.47 (s, C-2), 113.75 (s, C-4), 116.91, 118.59 (23 s, $C_6H_4$), 118.66 (s, C-$C_6H_4$), 126.23 (s, C-1), 131.24, 132.12 (2x s, Ar-C), 132.29 (s, C-10), 137.83 (s, C-5), 157.42 (s, C-3), 161.04 (s, HO-Ar), 163.87 (CH=N).

16α-[(E)-3-Hydroxy-salicylidenimino]-17β-hydroxy-3-methoxy-estra-1,3,5(10)-trien erhalten durch Umsetzung von 16α-Amino-17β-hydroxy-3-methoxy-estra-1,3,5(10)-trien) und 2,3-Dihydroxybenzaldehyd in abs. Methanol.
$C_{26}H_{31}NO_4$ (421,54). Ber: C 74.79, H 7.41, N 3.32, Gef: C 73.36, H 7.45, N 3.28.
Umkristallisiert wird aus Essigester unter langsamer Zugabe von Ether, dann Petrolether, Ausbeute: 82 %, F = 109-114 °C, $[\alpha]_D$ + 80,0 ° (c = 10.037).
MS (FAB): 421.5 ($M^+$), 138.1 (100 %).
$^1$H-NMR: 0.863 (s, 3H, 18-$H_3$), 2.830 (m, 2H, 6-$H_2$), 3.678 - 3.738 (m, überl., 2H, 16β-H und 16α-H), 3.767 (s, 3H, $OCH_3$), 3.88 (m, 1H, 6.530 - 6.726 (m, überl., 4H, 4x Ar-H), 6.910 (d, $^3J$ = 8.9 Hz, 1H, 2-H), 7.171 (d, $^3J$ = 8.9 Hz, 1H, 1-H), 8.120 (s, 1H, CH=N).
$^{13}$C-NMR: 12.04, 25.92, 27.10, 29.65, 32.28, 36.33, 38.37, 43.80, 43.92, 48.29, 55.21, 71.36, 87.75, 111.57, 113.84, 115.90, 116.36, 116.70, 122.31, 126.23, 132.20, 137.78, 146.52, 156.29, 157.54, 163.99.

3,17β-Dihydroxy-16α-(E)-salicylidenimino-estra-1,3,5(10)-trien:
erhalten durch Umsetzung von 16α-Amino-3,17β-dihydroxy-estra-1,3,5(10)-trien) und Salicylaldehyd in abs. Methanol.
$C_{25}H_{29}NO_3$ (391.53), Ber: C 76.69, H 7.47, N 3.58; Gef: C 76.36, H 7.18, N 3.56;
F = 235 - 238 °C, $[\alpha]_D$ - 2,8 ° (c = 9.432, Pyridin).
$^1$H-NMR: 0.89 (s, 3H, 18-$H_3$), 3.62-3.66 (1H, 16β-H), 3.71 (d, 1H, 17a-H), 6.55-7.32 (Ar-H), 8.33 (s, 1H, CH=N), 13.65 (s, 1H, 3-OH).
$^{13}$C-NMR: 12.2 (C-18), 32.8 (C-15), 36.5 (C-12), 44.0 (C-13), 48.4 (C-14), 74.6 (C-16), 163.9 (CH=N).

17α-Hydroxy-16β-[(E)-salicylidenimino)-3-methoxy-estra-1,3,5(10)-trien:
erhalten durch Umsetzung von 16β-Amino-17α-hydroxy-3-methoxy-estra-1,3,5(10)-trien) und Salicylaldehyd in abs. Methanol.
$C_{26}H_{31}NO_3$ (405,56), Ausbeute: 90.5 %, F = 198-203 °C (aus MeOH).
Ber.:C 76.99, H 7.71, N 3.47, Gef.: C 77.07, H 7.89, N 3.39; $[\alpha]_D$ + 62,7 °.
$^1$H-NMR: 0.96 (s, 3H, 18-$H_3$); 3.66 (t, $^3J$ = 7.5 Hz, 1H, 16ß-H); 3.76 (s, 3H, $OCH_3$); 3.78 (s, 1H, 17β-H); 6,62-7.32 (m, 7H, aromat. H); 8.33 (s, 1H, N=CH);
$^{13}$C-NMR ($CDCl_3$); 17.31 (C-18), 77.71 (C-16), 77.71 (16-C); 86.80 (17-C); 116.88, 118.65, 131.22, 132.19 ($C_6H_4$); 118.72 (C-$\underline{C}_6H_4$); 160.92 (HO-$\underline{C}_6H_4$); 163.55 (N=CH). IR (KBr): 1628 (s, CH=N).

17α-Hydroxy-16α-[(E)-salicylidenimino)-3-methoxy-estra-1,3,5(10)-trien:
erhalten als Tautomeren-Gemisch mit dem entsprechenden 1,3-Oxazolidin-C2-Epimeren durch Umsetzung von 16α-Amino-17α-hydroxy-3-methoxy-estra-1,3,5(10)-trien) und Salicylaldehyd in abs. Methanol.
$C_{26}H_{31}NO_3$ (405,56), Ausbeute: 72, %, F = 128-132 °C. Umkristallisation aus Ethanol ergibt unverändert dasselbe Isomerengemisch:
$[\alpha]_D$ 103.7 ° (c = 0.875). IR (ATR):1637 (s, CH=N), 3104 (br, NH).
Ber: C 76.99, H 7.71, N 3.47; Gef: C 76.57, H 7.87, N 3.53.
$^1$H-NMR: 0.72, 078 und 0.84 (3 x s, 3 x 3H, 3 x 18-$H_3$); 5.38 und 5.98 (2 x s, 2 x 1 H, 2 x CH-N); 8.43 (s, 1H, CH=N).
(E)-17β-Hydroxy-16β-(2-hydroxy-naphth-1-carbaldimino)-3-methoxy-estra-1,3,5(10)-trien:
erhalten durch Umsetzung von 16β-Amino-17β-hydroxy-3-methoxy-estra-1,3,5(10)-trien) und 2-Hydrox-naphth-1-carbaldehyd in abs. Ethanol.
$C_{30}H_{33}NO_3$ (455,6): Ber: C 79.10, H 7.30, N 3.07; Gef: C 78.98, H 7.37, N 3.13. 63 %, F = 260-262 °C (aus THF/Acetonitril), $[\alpha]_D$ + 59 ° (c = 0.974, Pyridin).
IR (KBr): 1628 $cm^{-1}$ (vs, CH=N), 3183 (m, br., OH ass.).
$^1$H-NMR: 0.97 (s, 3H, 18-$H_3$), 2.85 ($m_c$, 2H, 6-$H_2$), 3.76 (s, 3H, $OCH_3$), 3.71 (d, $^3J$ = 8.5 Hz, 1H, 17α-H), 4.04 ($m_c$, 1H, 16α-H), 6.62 (d, $^4J$ = 2.7 Hz, 1H, 4-H), 6.71 (dd, $^3J$ = 8.5 Hz, $^4J$ = 2.8 Hz, 1H, 2-H), 6.90 (d, $^4J$ = 2.7 Hz, 1H, Ar-H), 7.16 - 7.24 (m, Überlagerung in $CDCl_3$, ca. 2H, 1-H und Ar-H), 7.40 (t, $^3J$ = 7.1 Hz, 1H, Ar-H), 7.53 (d, $^3J$ = 7.9 Hz, 1H, Ar-H), 7.62 (d, $^3J$ = 8.4 Hz, 1H, Ar-H), 7.83 (d, $^3J$ = 8.4 Hz, 1H, Ar-H), 8.72 (s, 1H, CH=N), 14.46 (s, br., 1H, OH).

(E)-17α-Hydroxy-16α-(2-hydroxy-naphth-1-carbaldimino)-3-methoxy-estra-1,3,5(10)-trien:
erhalten durch Umsetzung von 16α-Amino-17α-hydroxy-3-methoxy-estra-1,3,5(10)-trien) und 2-Hydrox-1-naphthcarbaldehyd in abs. Ethanol, Ausbeute 63 %. $C_{30}H_{33}NO_3$ (455,6): Ber: C 79.10, H 7.30, N 3.07; Gef: C 77.27, H 7.43, N 3.06. - MS (70 eV); m/z (%): 455 (100) [$M^+$]. - HR-MS $C_{30}H_{33}NO_3$: Ber: 455.2450, Gef: 455.2461.
63 %, F = 235.5-237 °C (aus Methanol oder Toluen/Heptan), $[\alpha]_D$ + 286 ° (c = 0.981).
$^1$H-NMR (400 MHz, DMSO-$D_6$; HMQC, HMBC, TOCSY, NOE, NOESY): 0.78 (s, 3H, 18-$H_3$), 2.76 ($m_c$, 2H, 6-$H_2$), 3.68 (s, 3H, $OCH_3$), 3.71 (t, $^3J$ = 5.2 Hz, 1H, 17β-H), 4.24 ($m_c$, 1H, 16ß-H), 5.48 (d, $^3J$ = 5.2 Hz, 1H, 17-OH), 6.60 und 6.61 (2x d, überl., $^3J$ = 9.5 Hz, $^4J$ = 3.5 Hz, 2H, Ar-H und 4-H), 6.67 (dd, $^3J$ = 8.5 Hz, $^4J$ = 2.7 Hz, 1H, 2-H), 7.12 (t, J = 7.1 Hz, 1H, Ar-H), 7.18 (d, $^3J$ = 8.7 Hz, 1H, 1-H), 7.38 (t, $^3J$ = 8.3 Hz, 1H, Ar-H), 7.56 (d, $^3J$ = 7.8 Hz, 1H, Ar-H), 7.64 (d, $^3J$ = 9.4 Hz, 1H, Ar-H), 7.98 (d, $^3J$ = 8.4 Hz, 1H, Ar-H), 9.02 (d, $^3J$ = 12.3 Hz, 1H, CH=N), 13.60 (dd, $^3J$ = 12.2 Hz, $^4J'$ = 9.0

Hz, 1H, Ar-OH).
IR (KBr): 1629 $cm^{-1}$ (vs, CH=N), 3230 (w-m, br., OH ass.).

3,17β-Diydroxy-16α-salicylidenimino-estra-1,3,5(10)-trien :
erhalten durch Umsetzung von 16α-Amino-3,17β-dihydroxy-estra-1,3,5(10)-trien) und Salicylaldehyd in abs. Methanol. $C_{25}H_{29}NO_3$ (391.51), F = 235-238 °C (aus Methanol), $[\alpha]_D$ - 2.80 (c = 9.432, Pyridin), $R_f$ = 0.34.
$^1$H-NMR: 0.89 (s, 3H, 18-$H_3$), 3.62-3.66 (1H, 16β-H), 3.71 (d, 1H, 17α-H), 6.55-7.32 (Ar-H), 8.33 (s, 1H, CH=N), 13.65 (s, 1H, 3-OH).
$^{13}$C-NMR: 12.2 (C-18), 32.8 (C-15), 36.5 (C-12), 44.0 (C-13), 48.4 (C-14), 74.6 (C-16), 163.9 (CH=N).
Ber: C 76.69, H 7.47, N 3.58; Gef: C 76.36, H 7.18, N 3.56.

16α-[(E)-3-Hydroxy-5-hydroxymethyl-pyrid-4-carbaldimino]-17β-hydroxy-3-methoxy-estra-1,3,5(10)-trien:
erhalten durch Umsetzung von 16α-Amino-17β-hydroxy-3-methoxy-estra-1,3,5(10)-trien) und Pyridoxaldehyd-hydrochlorid in abs. Methanol. Wässrige Aufarbeitung unter Zusatz von wenig Kaliumcarbonat.
$C_{27}H_{34}N_2O_4$ (450.59), Ber: C 71.98, H 7.61, N 6.22, Gef: C 71.34, H 7.82, N 6.17. MS (FAB): M+HI$^+$ = 451.2 (100 %), F = 244-246 °C (Zers., aus 70 %-igem Methanol), Ausbeute 82 %, $[\alpha]_D$ + 14 ° (c = 10.261).
$^1$H-NMR (DMSO-$D_6$): 0.799 (s, 3H, 18-$H_3$), 1.34 - 2.30 (m, überl., 14H, steroid-C-H), 2.367 (s, 3H, Pyridin-$CH_3$), 2.768 (s, breit, 2H, 6-$H_2$), 3.680 (s, 3H, 3-MeO), 4.625 (s, 2H, C$\underline{H}_2$-OH) 5.130 (s, 1H,17α-H), 5.350 (s, 1H, 16β-H), 6.602 (s, 1H, 4-H), 6.660 (d, $^3$J = 8.6 Hz, 1H, 2-H), 7.160 (d, $^3$J = 8.6 Hz, 1H, 1-H), 7.893 (s, 1H, Pyridin-H), 8.797 (s, 1H, N=CH).
$^{13}$C-NMR (DMSO-$D_6$): 12.22, 18.60, 25.73, 26.68, 29.19, 32.34, 36.31, 43.42, 43.67, 47.87, 54.83, 47.87, 54.83, 58.33, 73.78, 86.68, 111.46, 113.43, 119.16, 126.08, 131.99, 132.62, 137.35, 148.04, 154.02, 157.03, 161.54.
IR (KBr): 1626 (CH=N), 1500, 1401.

16β-(2-Hydroxy-3,5-di-tert-butylphenyl-carbaldimino)-17α-hydroxy-estra-1,3,5(10)-trien
erhalten durch Umsetzung von 16β-Amino-17α-hydroxy-3-methoxy-estra-1,3,5(10)-trien) und 3,5-Di-t-Butylsalicylaldehyd in abs. Methanol.
$C_{34}H_{47}NO_3$ (517,54), Ausbeute: 90 %, F = 185-188 °C (aus MeOH oder Heptan). Ber.: C 78.91, H 9.15, N 2.71, Gef.: C 78.62, H 9.16, N 2.74; $[\alpha]_D$ + 45,5 °.
$^1$H-NMR: 0.983 (s, 3H, 18-$H_3$); 1.284 (s, 9H, *tert*-Bu);1.413 (s, 9H, tert-Bu); 2.836 ($m_c$, 2H, 6-$H_2$); 3.620 (t, $^3$J = 8 Hz, 1H, 17β-H); 3.762 (s, 3H, $OCH_3$); 3.807(s, 1H, 16α-H); 6.618 (s, 1H, 4-H), 6.695 (d, $^3$J = 9.4 Hz, 1H, 2-H), 7.066 (s, 1H, Ar-H), 7.222 (d, $^3$J = 9.4 Hz, 1H, 1-H), 7.346 (s, 1H, Ar-H), 8.360 (s, 1H, CH=N).

16α-{[2-Hydroxy-4-(4'-phenylcarbonyloxy)phenyl]-carbaldimino}-17β-hydroxy-3-methoxy-estra-1,3,5(10)-trien
erhalten durch Umsetzung von 16α-Amino-17β-hydroxy-3-methoxy-estra-1,3,5(10)-trien) und 2-Hydroxy-4-(4'-phenylcarbonyloxy)benzaldehyd in abs. Ethanol. $C_{33}H_{35}NO_5$ (525,64), Ausbeute: 37 %, F = 219-223 °C (langsame Zers., aus MeOH/Essigester = 3:1), $[\alpha]_D$ + 84 ° (c = 7.675). MS (FAB): M$^+$ = 526.8.
$^1$H-NMR: 0.895 (s, 3H, 18-$H_3$), 1.24 - 2.35 (m, überl., aliph H), 2.845 ($m_c$, 2H, 6-$H_2$), 3.470 (s, 3H, $OCH_3$), 3.65 - 3.34 (m, überl., 2H, 16β-H und 17α-H), 6.628 ($m_c$, 1H, 4-H), 6.690 (d, überl., $^3$J = 9 Hz, 2H, 2-H und Ar-H), 6.800 (s, 1H, Ar-H), 7.176 - 7.263 (m, über., 2H, 2x Ar-H), 7.530 (t, $^3$J = 8 Hz, 2H, Ar-H), 7.632 (t, $^3$J = 8 Hz, 1H, Ar-H), 8.718 (d, $^3$J = 8 Hz, 2H, 2x Ar-H), 8.313 (s, 1H, CH=N).
IR: 3536 (m, OH), 1732 (s, CH=N), 1631 (s), 1611 (s), 1500 (s), 1451 (m), 1257 (vs).

16α-{1-[2-Hydroxy-(4-phenylcarbonyloxy)-phenyl]-eth-1-ylenimino}-17β-hydroxy-3-methoxy-estra-1,3,5(10)-trien
erhalten durch Umsetzung von 16α-Amino-17β-hydroxy-3-methoxy-estra-1,3,5(10)-trien) und 2-Hydroxy-4-(4'-phenylcarbonyloxy)acetophenon in abs. Ethanol, 8 Stunden Reaktionsdauer bei Raumtemperatur.
$C_{34}H_{37}NO_5$ (539,67), Ausbeute: 50 %; F = 157-161 °C, Differentialthermoanalyse: F = 173 °C, aus Essigester/Heptan umkristallisiert; $[\alpha]_D$ + 132 ° (c = 9.017). MS (FAB): M$^+$ = 539.3.
$^1$H-NMR (400 MHz): 0.934 (s, 3H, 18-$H_3$), 1.24 - 2.08 (m, überl., 11H, aliph H), 2.287 (s m, überl., 5H, $CH_3$-C=N und 2x aliph H), 2.840 ($m_c$, 2H, 6-$H_2$), 3.765 (s, 3H, $OCH_3$), 3.857 (d, $^3$J = 7 Hz, 1H, 17α-H), 4.190 (d, $^3$J = 7 Hz, 1H, 16β-H), 6.099 (s, sher breit, 1H, OH), 6.383 (dd, $^3$J = 9 Hz, $^4$J = 2 Hz, 2H, 2x Ar-H), 6.646 (s, 2H, 2x Ar-H), 7.089 (d, $^3$J = 9 Hz, 1H, Ar-H), 7.275 (t, $^3$J = 9 Hz, 1H, Ar-H), 7.502 (t, $^3$J = 7 Hz, 2H, 2x Ar-H), 7.630 (t, $^3$J = 7 Hz, 1H, Ar-H), 8.159 (d, $^3$J = 7 Hz, 1H, Ar-H).
$^{13}$C-NMR (400 MHz): 12.10, 14.12, 13.85, 22.69, 26.14, 27.20, 29.80, 31.88, 32.98, 36.92, 38.43, 44.06, 44.10, 48.64, 55.231, 62.48, 88.40, 109.14, 111.59, 113.55, 113.737, 114.712, 126.528, 128.61, 129.41, 130.17, 130.66, 132.08, 133.68, 137.72, 155.97, 157.48, 164.81, 172.39, 172.97.

16α-{[2-Hydroxy-(4-(4'-n-butoxy-phenyl)carbonyloxy)-phenyl]-carbaldimino}-17β-hydroxy-3-methoxy-estra-1,3,5(10)-trien
erhalten durch Umsetzung von 16α-Amino-17β-hydroxy-3-methoxy-estra-1,3,5(10)-trien) und 2-Hydroxy-4-[4'-(4"-n-butoxyphenyl)carbonyloxy]benzaldehyd in abs. Methanol.
$C_{37}H_{43}NO_6$ (597,75), Ausbeute: 75 %, nach Umkristallisation aus Ethanol/Essigester: 55 %, F = 195 - 197 °C, $[\alpha]_D$ + 132 ° (c = 9.017).

$^1$H-NMR (400 MHz, Tocsy, Cosydqf, HMQC, HMBC, Dept90): 0.891 (s, 3H, 18-$H_3$), 0.979 (t, $^3$J = 7.2 Hz, 3H, $C\underline{H}_3$-$CH_2$-), 1.38-1.52 (m, überl., 7H, aliph H), 1.75-1.85 (m, überl., 3H, aliph H), 1.94-2.01 (m, überl., 2H, aliph. H), 2.298 ($m_c$, 2H, aliph. H), 2.844 ($m_c$, 2H, 6-$H_2$), 3.626 ($m_c$, 1H, 16β-H), 3.709 (d, $^3$J = 6.5 Hz, 1H, 17α-H), 3.763 (s, 3H, $OCH_3$), 4.034 (t, $^3$J = 6.5 Hz, 2H, -$CH_2$-O), 6.618 ($m_c$, 2H, 4-H und Ar-H), 6.700 (dd, $^3$J = 8.3 Hz, $^4$J = 2.9 Hz, 1H, 2-H), 6.742 (d, $^4$J = 2.8 Hz, 1H, Ar-H), 6.955 (d, $^3$J = 7.6 Hz, 2H, 2x Ar-H), 7.183 (dd, $^3$J = 8.3 Hz, $^4$J = 2.9 Hz, 1H, 1-H), 7.239 (s, 1H, Ar-H), 8.111 (d, $^3$J = 7.6 Hz, 2H, 2x Ar-H),
8.257 (s, 1H, CH=N).
$^{13}$C-NMR (400 MHz): 12.23, 13.81, 19.17, 26.02, 27.15, 29.72, 31.10, 32.81, 36.62, 38.43, 43.94, 44.00, 48.42, 55.19, 67.99, 73.74, 87.90, 98.71, 110.87, 111.52, 112.04, 113.78, 114.29, 116.17, 121.26, 126.28, 132.31, 132.72, 137.86, 154.62, 157.46, 163.38, 163.61, 164.57.
IR: 3435 (vs, breit), 1729 (m-s), 1628 (s), 1605 (s), 1501 (m-s).

16β-{[2-Hydroxy-(4-(4'-n-butoxy-phenyl)carbonyloxy)-phenyl]-carbaldimino}-17α-hydroxy-3-methoxy-estra-1,3,5(10)-trien
erhalten durch Umsetzung von 16β-Amino-17α-hydroxy-3-methoxy-estra-1,3,5(10)-trien) und 2-Hydroxy-4-[4'-(4"-n-butoxyphenyl)carbonyloxy]benzaldehyd in abs. Ethanol.
$C_{37}H_{43}NO_6$ (597,75), Ausbeute nach Umkristallisation aus Ethanol/Essigester: 67 %, F 154-157 °C, $[\alpha]_D$ + 31 ° (c = 10.010); nochmals aus Essigester/Heptan: F = 165-168 °C, $[\alpha]_D$ + 30 ° (c = 5.006).
EA, Ber: C 74.35, H 7.25, N 2.34, Gef: C 74.60, H 7.19, N 2.27.
$^1$H-NMR: 0.958 (s, 3H, 18-$H_3$), 0.979 (t, $^3$J = 7.2 Hz, 3H, $C\underline{H}_3$-$CH_2$-), 1.427-1.924 (m, überl., 12H, aliph H), 2.346 ($m_c$, 3H, aliph. H), 2.854 ($m_c$, 2H, 6-$H_2$), 3.666 (t, $^3$J = 7.4 Hz, 1H, 16α-H), 3.763 (s, overl., 4H, 17β und $OCH_3$), 4.034 (t, $^3$J = 6.5 Hz, 2H, -$CH_2$-O), 6.618 ($m_c$, 1H, 4-H), 6.686 - 6.779 (m, overl., 3H, 3x Ar-H), 6.948 (d, $^3$J = 8.8 Hz, 2H, 2x Ar-H), 7.149 - 7.265 (m, overl, 2H, 2x Ar-H), 8.113 (d, $^3$J = 8.8 Hz, 2H, 2x Ar-H), 8.315 (s, 1H, CH=N), 13.766 (s, 1H, OH).
$^{13}$C-NMR (DEPT 135, DEPT 90): 13.79, 17.36, 19.18, 25.92, 28.02, 29.80, 31.12, 31.86, 34.93, 38.68, 43.42, 45.37, 48.54, 55.21, 68.2, 86.86, 110.46, 111.56, 112.55, 113.81, 114.32, 116.55, 121.28, 126.30, 132.12, 132.34, 132.38, 137.88, 154.23, 157.52, 162.76, 162.95, 163.66, 164.47.
IR: 3440 (vs, breit), 1731 (m), 1628 (s), 1606 (s), 1510 (m), 1501 (m).

16β-{[2-Hydroxy-(4-(4'-n-butoxy-phenyl)carbonyloxy)-phenyl]-carbaldimino}-17β-hydroxy-3-methoxy-estra-1,3,5(10)-trien
erhalten durch Umsetzung von 16β-Amino-17β-hydroxy-3-methoxy-estra-1,3,5(10)-trien) und 2-Hydroxy-4-[4'-(4"-n-butoxyphenyl)carbonyloxy]benzaldehyd in abs. Ethanol.
$C_{37}H_{43}NO_6$ (597,75), Ausbeute nach Umkristallisation aus THF/Isopropanol/Essigester (1:2:2): 95 %, F 229-232 °C, $[\alpha]_D$ + 18 ° (c = 8.376); nochmals 2x aus Methanol/THF: F = 230-233.5 °C. MS (FAB): $M^+$ = 597.5.
$^1$H-NMR: 0.949 (s, 3H, 18-$H_3$), 0.989 (t, $^3$J = 7 Hz, 3H, $C\underline{H}_3$-$CH_2$-), 1.24-1.57 (m, überl., 8H, aliph H), 1.76-1.85 (m, überl., 3H, aliph H), 2.027 ($m_c$, 1H, aliph. H), 2.362 ($m_c$, überl., 3H, aliph. H), 2.298 ($m_c$, 3H, aliph. H), 2.847 ($m_c$, 2H, 6-$H_2$), 3.727 und 3.899 (jeweils: $m_c$, 1H, 16-α und 17α-H), 3.763 (s, 3H, $OCH_3$), 4.032 ($m_c$, 2H, -$CH_2$-O), 6.62 - 6.78 (m, 4H, 4-H und 3x Ar-H), 6.941 (d, $^3$J = 9 Hz, 2H, 2x Ar-H), 6.213 (d, $^3$J = 9 Hz, 1H, Ar-H), 7.283 (d, $^3$J = 9 Hz, 1H, Ar-H), 8.109 (d, $^3$J = 9 Hz, 2H, 2x Ar-H), 8.300 (s, 1H, CH=N), 13.045 (s, 1H, OH).
$^{13}$C-NMR (DEPT 135, DEPT 90): 12.10, 13.79, 19.17, 26.15, 27.34, 29.72, 31.11, 34.48, 37.16, 38.34, 43.47, 44.14, 47.92, 55.20, 67.99, 68.02, 68.75, 82.88, 110.51, 111.57, 112.78, 113.84, 114.33, 116.46, 121.25, 126.33, 132.34, 132.53, 137.78, 154.51, 157.54, 162.72, 163.67, 164.34, 165.62.
IR: 3445 (vs, breit), 1728 (m-s), 1628 (s), 1605 (s), 1501 (m-s).

**Allgemeine Arbeitsvorschrift für die Reduktion Schiffscher Basen zu den Arylmethylaminen mit $NaBH_4$ gemäß Schema 1** 1 mmol entsprechend vorangehenden Beispielen hergestellte Steroid-Schiff-Base wird in 10 ml abs. Methanol unter Argon bei Raumtemperatur mit 2 Äquivalenten $NaBH_4$ versetzt. Nach 5 - 10 min entsteht zumeist eine klare farblose Lösung. Es wird je nach Reaktivität 20 bis 100 min bei Raumtemperatur gerührt und anschließend $H_2O$ und wenige Tropfen Essigsäure zugegeben. Der entstandene Niederschlag wird abgesaugt, mehrmals mit $H_2O$ gewaschen und aus Methanol oder Ethanol, ggfs. unter Zusatz von wenig Wasser, umkristallisiert. Schwerer lösliche Steroid-Amine werden vorteilhafterweise unter Zugabe von THF umkristallisiert.

Auf diese Weise erhält man beispielsweise:
17β-(2-Hydroxyphenyl-methylamino)-3-methoxy-estra-1,3,5(10)-trien (**1**):
erhalten durch Umsetzung von 17β-[(E)-Salicylidenimino]-3-methoxy-estra-1,3,5(10)-trien und $NaBH_4$.
$C_{26}H_{33}NO_2$ (391.55), F = 117.5-119.5 °C (aus Methylenchlorid/Hexan), $R_f$ = 0.29. $^1$H-NMR: 0.789 (s, 3H, 18-$H_3$), 1.170-2.332 (m, überll, ca. 13H, 13x aliph. H), 2.720 (t, $^3$J = 8.4 Hz, 1H, 17α-H), 2.847 (s, breit, 2H, 6-$H_2$), 3.780 (s, 3H, 3-MeO), 3.959 (d, $^2$J = 15.1 Hz, 1H, Benzyl-H), 4.106 (d, $^2$J = 15.1 Hz, 1H, Benzyl-H), 6.627 (m, 1H, 4-H), 6.694-6.861 (m, überl. 3H, 1-H und 2x Ar-H), 7.001 (d, $^3$J = 7.5 Hz, 1H, Ar-H), 7.184 (m, 2H, 2x Ar-H).
$^{13}$C-NMR: 11.86, 23.46, 26.29, 27.38, 28.84, 29.76, 37.56, 38.72, 42.92, 43.96, 51.68, 52.02, 55.19, 67.78, 111.46, 113.82, 116.43, 118.90, 122.94, 126.28, 128.10, 128.68, 132.53, 137.89, 157.47, 158.47.

17α-(2-Hydroxyphenyl-methylamino)-3-methoxy-estra-1,3,5(10)-trien (2):

erhalten durch Umsetzung von 17α-[(E)-Salicylidenimino]-3-methoxy-estra-1,3,5(10)-trien und $NaBH_4$.
$C_{26}H_{33}NO_2$ (391.55), F = 145.5-147.5 °C (aus Methanol), $R_f$ = 0.71, $[\alpha]_D$ +7.1 ° (c = 10.508).
$^1$H-NMR (300 MHz): 0.914 (s, 3H, 18-$H_3$), 1.458-2.364 (m, überl.,13H, aliph. H), 2.788 (d, $^3$J = 6.6 Hz, 1H, 17β-H), 2.868 (m, breit, 2H, 6-$H_2$), 3.789 (s, 3H, 3-MeO), 3.826 (d, $^2$J = 13.8 Hz, 1H, NH-C<u>H</u>$_2$-Phenyl), 4.062 (d, $^2$J = 13.8 Hz, 1H, NH-C<u>H</u>$_2$-Phenyl), 6.645 (s, 1H, 4-H), 6.726 (d, $^3$J = 8.4 Hz, 1H, 2-H), 6.760-6.866 (m, überl., 2H, Ar-H), 7.014 (d, $^3$J = 8.3 Hz, 1H, 1-H), 7.158-7.205 (m, überl. 2H, Ar-H).
$^{13}$C-NMR (75 MHz): 19.08, 24.82, 26.31, 28.08, 29.05, 29.89, 32.83, 39.24, 43.56, 44.95, 49.59, 51.06, 55.20, 66.70, 111.49, 113.80, 116.42, 118.94, 122.96, 126.36, 128.13, 128.70, 132.51, 137.92, 157.47, 158.34.
IR: 1613 (m), 1586 (m) und 1495 (m; arom. C=C), kein Signal bei 1630 (für CH=N).

16β-(2-Hydroxyphenyl-methylamino)-3-methoxy-estra-1,3,5(10)-trien (3):
erhalten durch Umsetzung von 16β-[(E)-Salicylidenimino]-3-methoxy-estra-1,3,5(10)-trien und $NaBH_4$.
$C_{26}H_{33}NO_2$ (391.55), F = 149-151.5 °C (aus Methanol), Ausbeute 88 %, $R_f$ = 0.43, $[\alpha]_D$ + 61.7 ° (c = 7.315).
$^1$H-NMR: 0.92 (s, 3H, 18-$H_3$), 3.29 (m, 1H, 16α-H), 3.93 (q, 2H, N-$CH_2$), 3.76 (s, 3H, $OCH_3$), 6.61-7.19 (7H, Ar-H).
$^{13}$C-NMR: 19.9 (C-18), 26.4 (C-11), 28.0 (C-7), 29.8 (C-6), 34.2 (C-15), 38.9 (C-12), 38.4 (C-8), 40.5 (C-13), 43.8 (C-9), 47.6 (C-17), 52.7 (C-14), 55.2 ($OCH_3$), 56.4 (C-16), 111.5 (C-2), 113.7 (C-4), 126.2 (C-1), 132.6 (C-10), 137.8 (C-5), 157.4 (C-3); 51.6 ($CH_2$-N); Ar-C: 116.4, 119.0, 122.7 (q), 128.2, 128.7, 158.2 (q).
Ber: C 79.75, H 8.49, N 3.58; Gef: 78.70, 8.37, 3.95.
HRMS: Ber: 391.25113, Gef: 391.25171.
IR: 1472, 1499, 1608, 3687.

16α-(2-Hydroxyphenyl-methylamino)-3-methoxy-estra-1,3,5(10)-trien (4):
erhalten durch Umsetzung von 16α-[(E)-Salicylidenimino]-3-methoxy-estra-1,3,5(10)-trien und $NaBH_4$.
$C_{26}H_{33}NO_2$ (391.55), 148-149.5 °C (aus Methanol), Aussbeute 87 %, $R_f$ = 0.30, $[\alpha]_D$ +54.8 ° (c = 6.780).
$^1$H-NMR: 0.75 (s, 3H, 18-$H_3$), 3.38 (m, 1H, 16β-H), 3.39 (q, 2H, N-$CH_2$), 3.76 (s, 3H, $OCH_3$), 6.61-7.20 (7H, Ar-H).
$^{13}$C-NMR: 18.3 (C-18), 26.3 (C-11), 28.0 (C-7), 29.6 (C-6), 33.0 (C-15), 38.6 (C-12), 38.7 (C-8), 41.6 (C-13), 43.9 (C-9), 49.5 (C-17), 51.7 (C-14), 55.2 ($OCH_3$), 55.8 (C-16), 111.4 (C-2), 113.8 (C-4), 126.2 (C-1), 132.7 (C-10), 137.9 (C-5), 157.4 (C-3); 51.7 ($CH_2$-N); Ar-C: 116.4, 118.9, 122.8 (q), 128.1, 128.6, 154.4 (q).
Ber: C 79.75, H 8.49, N 3.58; Gef: 79.59, 8.44, 3.59.
HRMS: Ber: 391.25113, Gef: 391.25390.

17a-(2-Methoxyphenyl-methylamino)-3-methoxy-estra-1,3,5(10)-trien (5):
erhalten durch Umsetzung von 17α-[(E)-2-Methoxyphenyl-carbaldimino]-3-methoxy-estra-1,3,5(10)-trien und $NaBH_4$.
$C_{27}H_{35}NO_2$ (405.58), F = 71-72 °C (aus Hexan oder MeOH), $R_f$ = 0.42.
$^1$H-NMR (400 MHz): 0.747 (s, 3H, 18-$H_3$), 1.270-2.348 (m, overl, ca. 13H, aliphatic H), 2.702 (d, überl., $^3$J = 6.7 Hz, 1H, 17β-H), 2.847 (s, breit, 2H, 6-$H_2$), 3.705 (d, $^2$J = 13.6 Hz, 1H, N-Benzyl-H), 3.789 (s, 3H, 3-MeO), ca. 3.800 (d, überl., $^2$J = 13.6 Hz, 1H, N-Benzyl-H), 3.870 (s, 3H, 2-MeO-Benzyl), 6.638 (s, 1H, 4-H), 6.712 (d, $^3$J = 8.6 Hz, 1H, 2-H), 6.876-6.934 (m, überl., 1-H und Phenyl-H), 7.219-7276 (m, überl. 3H, 3x Phenyl-H).
$^{13}$C-NMR (100 MHz): 19.02, 25.04, 26.48, 28.06, 29.97, 30.10, 32.69, 39.34, 43.46, 43.68, 45.05, 48.23, 48.79, 55.20, 55.25, 66.37, 110.31, 111.43, 113.80, 120.45, 126.27, 128.01, 129.82, 133.07, 138.14, 157.42, 157.77.

17a-(3-Hydroxyphenylmethylamino)-3-methoxy-estra-1,3,5(10)-trien (6):
erhalten durch Umsetzung von 17α-[(E)-3-Hydroxyphenyl-carbaldimino]-3-methoxy-estra-1,3,5(10)-trien und $NaBH_4$.
$C_{26}H_{33}NO_2$ (391.55), F = 128-129 °C (Rohprodukt), $R_f$ = 0.19 (einheitlich).
$^1$H-NMR (in DMSO-$D_6$): 0.668 (s, 3H, 18-$H_3$), 1.000-2.400 (m, overl, ca. 13H, aliph. H), 2.587 (d, $^3$J = 6.7 Hz, 1H, 17β-H), 2.771 (s, breit, 2H, 6-$H_2$), 3.513 (d, $^2$J = 13.8 Hz, 1H, N-Benzyl-H), 3.634 (d, überl., $^2$J = 13.8 Hz, 1H, N-Benzyl-H), 3.673 (s, überl., 3H, 3-MeO), 6.590 (s, 1H, 4-H), 6.630-6.748 (m, überl., 3H, 2-H and 2x Ar-H), 7.059 (t, $^3$J = 7.8 Hz, 1H, Ar-H), 7.164 (d, $^3$J = 8.5 Hz, 1H, 1-H), 9.214 (s, 1H, X-H).
$^{13}$C-NMR(in DMSO-$D_6$): 19.93, 24.87, 26.61, 28.24, 29.85, 30.07, 32.92, 43.69, 45.32, 48.61, 52.57, 55.31, 66.59, 111.91, 113.76, 113.86, 115.15, 118.88, 126.64, 129.38, 132.73, 137.89, 143.37, 157.46, 157.66.

17a-(4-Hydroxyphenyl-methylamino)-3-methoxy-estra-1,3,5(10)-trien (7):
erhalten durch Umsetzung von 17α-[(E)-4-Hydroxyphenyl-carbaldimino]-3-methoxy-estra-1,3,5(10)-trien und $NaBH_4$.
$C_{26}H_{33}NO_2$ (391.55), F = 143-145 °C (aus EtOH, 90 %), $R_f$ = 0.19.
$^1$H-NMR: 0.759 (s, 3H, 18-$H_3$), 1.284-2.000 (m, overl, ca. 13H, aliph. H), 2.772 (d, überl., $^3$J = 6.8 Hz, 1H, 17β-H), 2.843 (s, breit, 2H, 6-$H_2$), 3.593 (d, $^2$J = 12.8 Hz, 1H, N-Benzyl-H), ca. 3.740 (d, überl., $^2$J = 12.8 Hz, 1H, N-Benzyl-H), 3.778 (s, 3H, 3-MeO), 6.634-6.722 (s and d, überl., $^3$J = 8.4 Hz, 4H, 4-H, 2-H and 2x Phenyl-H), 7.128-7.263 (d and m, überl., $^3$J = 8.4 Hz, 1-H and 2x Phenyl-H).
$^{13}$C-NMR: 19.12, 25.04, 26.43, 28.03, 29.93, 30.08, 32.86, 39.24, 43.42, 45.06, 48.92, 52.30, 55.20, 67.03, 111.42, 113.76, 115.11, 115.53, 126.30, 129.49, 132.16, 132.92, 138.09, 155.01, 157.37.

17a-[(2-Hydroxynaphth-1-yl)-methylamino]-3-methoxy-estra-1,3,5(10)-trien (17): erhalten durch Umsetzung von 17α-[(E)-2-Hydroxynaphth-1-carbaldimino]-3-methoxy-estra-1,3,5(10)-trien und $NaBH_4$.
$C_{30}H_{35}NO_2$ (441.61), MS: M+HI$^+$ = 442.4 (100 %), F = 105-106 °C (aus EtOH, Zusatz von 10 % $H_2O$), $R_f$ = 0.58.

$^1H$-NMR (ausgewählte Daten): 0.858 (s, 3H, 18-$H_3$), 2.875 (m, breit, 2H, 6-$H_2$), 3.796 (s, 3H, 3-MeO), 4.373 (d, $^2J$ = 9.3 Hz, 2H, Benzyl-H), 4.483 (d, $^2J$ = 9.3 Hz, 2H, Benzyl-H), 6.654 (s, 1H, 4-H), 6.736 (d, $^3J$ = 4.7 Hz, 1H, 2-H), 7.172 (d, $^3J$ = 4.7 Hz, 1H, 1-H), 7.200-7.480 (m, überl., ca. 6H, Naphthyl-H).

17β-[(3-Hydroxynaphth-2-yl)-methylamino]-3-methoxy-estra-1,3,5(10)-trien (18): erhalten durch Umsetzung von 17β-[(E)-3-Hydroxynaphth-2-carbaldimino]-3-methoxy-estra-1,3,5(10)-trien (25) und $NaBH_4$.
$C_{30}H_{35}NO_2$ (441.61), MS: M+HI$^+$ = 442.4 (100 %), F = 192-194 °C (aus EtOH/THF, 1:1, Zusatz von 10 % $H_2O$). $R_f$ = 0.40.
$^1H$-NMR: 0.814 (s, 3H, 18-$H_3$), 1.293-2.291 (m, überl, ca. 13H, aliph. H), 2.706 (t, $^3J$ = 8.6 Hz, 1H, 17α-H), 2.850 (s, breit, 2H, 6-$H_2$), 3.784 (s, 3H, 3-MeO), 4.130 (d, $^2J$ = 13.7 Hz, 1H, Benzyl-H), 4.273 (d, $^2J$ = 13.7 Hz, 1H, Benzyl-H), 6.639 (s, breit, 1H, 4-H), 6.717 (d, $^3J$ = 8.2 Hz, 1H, 2-H), 7.204-7.308 (m, überl. 3H, 1-H and 2x Naphthyl-H), 7.390 (t, $^3J$ = 7.8 Hz, 1H, Naphthyl-H), 7.508 (s, 1H, Naphthyl-H), 7.692 (d, $^3J$ = 8.1 Hz, 2H, 2x Naphthyl-H).
$^{13}C$-NMR: 11.88, 23.46, 26.29, 27.37, 28.82, 29.76, 37.50, 38.71, 42.92, 43.94, 51.96, 51.99, 55.20, 67.69, 110.61, 111.46, 113.82, 123.03, 125.76, 125.93, 126.19, 126.29, 127.16, 127.26, 127.98, 132.51, 134.52, 137.89, 156.50, 157.47.

17β-[(2-Hydroxynaphth-1-yl)-methylamino]-3-methoxy-estra-1,3,5(10)-trien Hydroperchlorat (**19**):
erhalten durch Umsetzung von 17β-[(E)-2-Hydroxynaphth-1-carbaldimino]-3-methoxy-estra-1,3,5(10)-trien und $NaBH_4$. Das Rohprodukt wird an Kieselgel chromatographiert (Elutionsmittel Essigester/Hexan, Gradient 1:7 bis 1:4). Das erhaltene Öl wird in THF gelöst, mit 2 Äquivalenten Perchlorsäure versetzt und das Produkt bei 4 °C Wasser gefällt.
$C_{30}H_{36}NO_6Cl$ (542.07), MS: 442.4 (M$^+$ - $ClO_4$), F = 138-143 °C (glasartig, Zers.) $R_f$ = 0.50.
$^1H$-NMR (breite Signale, ausgewählte Daten): 0.896 (s, 3H, 18-$H_3$), 2.770 (s, breit, 2H, 6-$H_2$), 3.752 (s, 3H, 3-MeO), 4.716 (m, 2H, $CH_2$-N), 8.455 (s, breit, 1H, X-H). $^{13}C$-NMR: 11.51, 23.21, 25.15, 25.76, 27.08, 29.45, 35.97, 38.16, 38.45, 42.73, 43.29, 43.74, 51.25, 55.19, 66.89, 107.72, 111.54, 113.82, 117.64, 121.26, 123.83, 126.24, 127.98, 129.07, 131.64, 132.03, 132.44, 137.52, 153.71, 157.54.

17β-(3-Hydroxyphenyl-methylamino)-3-methoxy-estra-1,3,5(10)-trien (20):
erhalten durch Umsetzung von 17β-[(E)-3-Hydroxyphenyl-carbaldimino]-3-methoxy-estra-1,3,5(10)-trien und $NaBH_4$.
$C_{26}H_{33}NO_2$ (391.55), MS: M+HI$^+$ = 392.3 (100 %), 184-187 (aus EtOH/THF, 4:1), $R_f \approx 0$.
$^1H$-NMR (breite Signale): 0.795 (s, 3H, 18-$H_3$), 1.100-2.350 (m, überl., ca. 13H, aliph. H), 2.704 (t, $^3J$ ca. 7 Hz, 1H, 17α-H), 2.482 (s, sehr breit, ca. 4H, 6-$H_2$ und 2x N-Benzyl-H), 3.779 (s, 3H, 3-MeO), 6.663 (s, 1H, 4-H), 6.700 (s, breit, überl., 2H, 2-H und Phenyl-H), 6.799-6.869 (m, überl., 2H, 2x Ar-H), 7.181 (m, überl., 2x Ar-H).
$^{13}C$-NMR: 11.92, 23.50, 26.50, 27.42, 29.54, 29.82, 38.12, 38.79, 43.17, 43.99, 52.28, 52.44, 55.19, 68.30, 111.43, 113.79, 114.05, 115.17, 120.17, 126.27, 129.50, 132.76, 137.99, 142.43, 155.96, 157.40.

3β-Hydroxy-2α-(2-hydroxyphenyl-methylamino)-cholan (**21**):
erhalten durch Umsetzung von 3β-Hydroxy-2α-[(E)-Salicylidenimino]-cholan und $NaBH_4$.
$C_{34}H_{55}NO_2$ (509.81), Klärpunkt 167-168 °C (aus Methanol), $R_f$ = 0.60.
$^1H$-NMR (ausgewählte Daten): 0.665 (s, 3H, 18-$H_3$), 2.633 (m, 1H), 3.432 (m, 1H), 3.958 (d, $^2J$ = 13.0 Hz, 1H, Benzyl-H), 4.100 (d, $^2J$ = 13.0 Hz, 1H, Benzyl-H), 6.780-7.165 (m, 4H, Phenyl-H).
$^{13}C$-NMR: 11.50, 13.20, 18.68, 21.32, 22.53, 22.77, 23.83, 24.19, 27.99, 28.10, 28.20, 31.89, 35.02, 35.77, 36.18, 36.73, 37.31, 39.53, 39.89, 42.59, 44.72, 50.30, 54.26, 56.27, 56.37, 59.64, 116.48, 119.03, 123.55, 127.94, 128.61, 158.10.

2β,3β-Dihydroxy-1α-(2-hydroxypheny)-methylamino)-cholan (**22**):
erhalten durch Umsetzung von 2β,3β-Dihydroxy-1a[(E)-Salicylidenimino]-cholan und $NaBH_4$.
$C_{34}H_{55}NO_3$ (525.81), M+HI$^+$ = 526.4 (100%), Klärpunkt 162-164 °C, $R_f$ = 0.16. $^1H$-NMR: 0.670 (s, 3H, 18-$H_3$), 2.861 (s, breit, 1H), 3.863 (d, $^2J$ = 13.4 Hz, 1H, Benzyl-H), 3.900 (m, überl., 1H), 4.086 (d, $^2J$ = 13.4 Hz, 1H, Benzyl-H), 4.221 (s, breit, 1H), 6.774-7.194 (m, 4H, Phenyl-H).
$^{13}C$-NMR: 12.11, 14.40, 18.73, 21.10, 22.54, 22.79, 23.79, 24.20, 27.99, 28.15, 28.43, 31.41, 32.55, 34.86, 35.77, 36.12, 39.10, 39.24, 39.46, 39.49, 42.58, 48.81, 52.68, 56.05, 56.23, 64.48, 68.81, 70.77, 116.41, 119.34, 122.95, 128.33, 129.10, 157.67.

17β-[4-(N,N-Diethylamino)-2-hydroxyphenyl-methylamino]-3-methoxy-estra-1,3,5(10)-trien (**23**):
erhalten durch Umsetzung von 17β-[(E)-[4-(N,N-Diethylamino)-2-hydroxyphenyl-carbaldimino]-3-methoxy-estra-1,3,5(10)-trien (24) und $NaBH_4$ in EtOH/THF (2:1). $C_{30}H_{42}N_2O_2$ (462.67); MS: M+HI$^+$ = 463.3 (11%), 461.3 (59%), 286.2 (44%), 178.1 (100%); F = 137-142 °C (Rohprodukt), $R_f$ = 0.30.
$^1H$-NMR (sehr breite Signale): 0.773 (s, 3H, 18-$H_3$), 1.157 (s, überl., 6H, 2x$CH_3$), 2.851 (s, breit, 2H, 6-$H_2$), 3.310 (s, 4H, 2x$CH_2$N), 3.783 (s, 3H, 3-MeO).
$^{13}C$-NMR: 11.20, 12.10, 23.49, 28.86, 29.78, 30.31, 31.17, 37.57, 38.73, 42.89, 44.37, 44.47, 51.05, 52.21, 55.19, 67.54, 99.37, 102.73, 110.18, 111.44, 113.80, 126.30, 128.69, 132.74, 138.02, 148.78, 157.44, 159.37.

17α-(5-Chlor-2-hydroxyphenyl-methylamino)-3-methoxy-estra-1,3,5(10)-trien (**26**): erhalten durch Umsetzung von 17α-[(E)-5-Chlorsalicylidenimino]-3-methoxy-estra-1,3,5(10)-trien und $NaBH_4$. Reinigung des Rohproduktes durch Säulenchromatographie (Silicagel, Essigester/Hexan, Gradient 1:8 bis 1:2). $C_{26}H_{32}NO_2Cl$ (425.99), MS: M+HI$^+$ = 426.2 (100 %), Öl, $R_f$ = 0.61.

17β-(5-Chlor-2-hydroxyphenyl-methylamino)-3-methoxy-estra-1,3,5(10)-trien (**27**): erhalten durch Umsetzung von 17β-[(E)-5-Chlorsalicylidenimino]-3-methoxy-estra-1,3,5(10)-trien und $NaBH_4$.

$C_{26}H_{32}NO_2Cl$ (425.99), MS: M+HI+ = 426.2 (100 %), F = 161-163 (aus EtOH), $R_f$ = 0.69.

17β-(2-Hydroxy-5-nitrophenyl-methylamino)-3-methoxy-estra-1,3,5(10)-trien **(28)**: erhalten durch Umsetzung von 17β-[(E)-5-Nitrosalicylidenimino]-3-methoxy-estra-1,3,5(10)-trien und $NaBH_4$.

$C_{26}H_{32}N_2O_4$ (436.55), F = 212-215 °C (aus EtOH/THF 1:1), $R_f$ = 0.14.

17β-(5-Brom-2-hydroxyphenyl-methylamino)-3-methoxy-estra-1,3,5(10)-trien (**29**): erhalten durch Umsetzung von 17β-[(E)-5-Bromsalicylidenimino]-3-methoxy-estra-1,3,5(10)-trien und $NaBH_4$.

$C_{26}H_{32}NO_2Br$ (470.44), F = 164-166 °C (aus EtOH, Zusatz von 20 % Wasser), $R_f$ = 0.62.

$^1$H-NMR: 0.798 (s, 3H, 18-$H_3$), 1.115-2.25 (m, überl., Steroid C-H), 2.733 (t, $^3J$ = 8.5 Hz, 1H, 17α-H), 2.862 (s, 2H, 6-$H_2$), 3.775 (s, 3H, $OCH_3$), 3.941 (d, $^2J$ = 14.0 Hz, 1H, Benzyl-H), 4.112 (d, $^2J$ = 14 Hz, 1H, Benzyl-H), 6.631 (s, 1H, 4-H), 6.715 (d, $^3J$ = 8.7 Hz, 1H, 2-H), 6.828 -7.287 (m, ca. 4H, Ar-H).

17β-(2,5-Dihydroxyphenyl-methylamino)-3-methoxy-estra-1,3,5(10)-trien (**30**): erhalten durch Umsetzung von 17β-[(E)-5-Hydroxysalicylidenimino]-3-methoxy-estra-1,3,5(10)-trien und $NaBH_4$.

$C_{26}H_{33}NO_3$ (407.55), F = 220-226 °C (ab 213 °C Zers., aus EtOH, anschl. Zusatz von Wasser), $R_f$ = 0.14.

$^1$H-NMR (ausgewählte Daten): 0.792 (s, 18-$H_3$), 1.10 - 2.40 (m, überl., Steroid C-H), 2.857 (s, 2H, 6-$H_2$), 3.773 (s, 3H, $OCH_3$), 3.920 (d, $^2J$ = 14.0 Hz, 1H, Benzyl-H), 4.110 (d, $^2J$ = 14 Hz, 1H, Benzyl-H), 6.586-7.201 (m, ca. 6H, Ar-H).

17β-(2,3,4-Trihydroxyphenyl-methylamino)-3-methoxy-estra-1,3,5(10)-trien (**31**): erhalten durch Umsetzung von 17β-[(E)-2,3,4-Trihydroxyphenyl-carbaldimino]-3-methoxy-estra-1,3,5(10)-trien und $NaBH_4$.

$C_{26}H_{33}NO_4$ (423.55), MS: 424.3 (56%), 285 (100%), F > 330 °C (ab ca. 200 °C Zers.; Rohprodukt), $R_f$ = 0.

$^1$H-NMR (DMSO): 0.750 (s, 3H, 18-$H_3$), 1.0 - 2.2 (Steroid-C-H), 2.500 (s, 1H, 17α-H), 2.756 (s, breit, 2H, 6-$H_2$), ca. 3.54 (sehr breit, X-H), 3.673 (s, 3H, 3-MeO), 6.043 - 7.165 (m, 5H, Ar-H), 8.474 (s, sehr breit, X-H).

16α-[(3-Hydroxy-naphth-2-yl)-methylamino]-17β-hydroxy-3-methoxy-estra-1,3,5(10)-trien (**32**): erhalten durch Umsetzung von 16α-[(E)-3-Hydroxy-naphth-2-carbaldimino]-3-methoxy-estra-1,3,5(10)-trien und $NaBH_4$.

$C_{30}H_{35}NO_3$ (457.61), F = 218-222 °C (Zers., aus Ethanol), $R_f$ = 0.09.

16α-(2,3-Dihydroxyphenyl-methy)amino)-17β-hydroxy-3-methoxy-estra-1,3,5(10)-trien (**33**): erhalten durch Umsetzung von 16α-[(E)-3-Hydroxy-salicylidenimino]-17β-hydroxy-3-methoxy-estra-1,3,5(10)-trien und $NaBH_4$.

$C_{26}H_{33}NO_4$ (423.55), F > 250 °C, Zers. (aus Ethanol/Wasser, 4:1), $R_f$ = 0.

$^1$H-NMR: 0.821 (s, 3H, 18-$H_3$), 1.26-2.00 (Steroid-C-H), 2.879 (s, breit, 2H, 6-$H_2$), 3.792 (s, 3H, 3-MeO), 6.690 (m, 2H, 2 x Ar-H), 7.221-7.801 (m, ca. 7H, 7 x Ar-H).

16a-(5-Chlor-2-hydroxyphenyl-methylamino)- 17β-hydroxy-3-methoxy-estra-1,3,5(10)-trien (**34**): erhalten durch Umsetzung von 16α-[(E)-5-Chlor-salicylidenimino]-17β-hydroxy-3-methoxy-estra-1,3,5(10)-trien und $NaBH_4$.

$C_{26}H_{32}NO_3Cl$ (441.99), F =153.5-154 °C (aus Ethanol/Wasser), $R_f$ = 0.14.

16a-[(3-Hydroxy-naphth-2-yl)-methylamino]-17α-hydroxy-3-methoxy-estra-1,3,5(10)-trien (**36**): erhalten durch Umsetzung von 16α-[(E)-3-Hydroxy-naphth-2-cabaldimin]-17α-hydroxy-3-methoxy-estra-1,3,5(10)-trien und $NaBH_4$.

$C_{30}H_{35}NO_3$ (457.61), F > 200 °C (Zers., aus Ethanol/THF), $R_f$ = 0.

$^1$H-NMR: 0.477 (s, 3H, 18-$H_3$), 1.2 - 2.5 (m, überl., Steroid C-H), 2.718 (m, 2H, 6-$H_2$), 3.732 (s, 3H, $OCH_3$), 6.531-7.766 (Ar-H).

16α-(5-Chlor-2-hydroxyphenyl-methylamino)-17α-hydroxy-3-methoxy-estra-1 ,3,5(1 0)-trien (**37**): erhalten durch Umsetzung von 16α-[(E)-5-Chlor-salicylidenimino]-17α-hydroxy-3-methoxy-estra-1,3,5(10)-trien und $NaBH_4$.

$C_{26}H_{32}NO_3Cl$ (441.99), MS: M+HI+ = 442.2; F = 250-255 (Zers., aus Ethanol/Wasser), $R_f$ = 0.

2a-(2-Hydroxyphenyl-methylamino)-3a-hydroxycholan **(38):** erhalten durch Umsetzung von 2α-[(E)-Salicylidenimino]-3α-hydroxy-cholan und $NaBH_4$.

$C_{34}H_{55}NO_2$ (509.81), Klärpunkt: 285-290 °C (aus Ethanol/Wasser; dann AcOEt/Hexan), $R_f$ = 0.22.

$^1$H-NMR: 0.537 (s, 3H, 18-$H_3$), 0.620 (s, 3H, 19-$H_3$); 0.742, 0.7967 und 0.803: überl., 21-, 26- und 27-$H_3$), 0.7 - 2.0 (m, überl., aliph. H), 2.478 (s, 1H, 2β-H), 3.010 (s, 1H, 3β-H), 4.057 and 4.138 (jeweils: s, breit, 1H; $CH_2$-N), 6.737 (t, $^3J$ = 8 Hz, 1H, Ar-H), 6.924 (d, $^3J$ = 8 Hz, 1 H, Ar-H), 7.108 (t, $^3J$ = 8 Hz, 1H, Ar-H), 7.256 (d, $^3J$ = 8 Hz, 1H, Ar-H), 8.366 (s, broad, 1H, OH), 8.495 (s, broad, 1H, OH).

$^{13}$C-NMR: 17.01, 17.08, 20.44, 27.08, 28.40, 28.82, 32.33, 32.61, 32.86, 36.41, 39.63, 39.70, 39.95, 40.37, 40.78, 41.31, 42.86, 44.84, 47.21, 48.99, 58.60, 60.80, 60.97, 61.49, 62.55, 68.21, 120.76, 122.59, 124.40, 135.44, 136.30, 160.95.

2α-[(3-Hydroxy-naphth-2-yl)-methylamino]-3β-hydroxy-cholan (**39**): erhalten durch Umsetzung von 2α-[(E)-3-Hydroxy-naphth-2-carbaldimino]-3β-hydroxy-cholan und $NaBH_4$.

$C_{38}H_{57}NO_2$ (559.87), Klärpunkt: 189-191 °C (aus Ethanol/THF = 4:1), $R_f$ = 0.07. $^1$H-NMR (sehr breite Signale): 0.5-2.3 (m, überl., aliph. H), 2.86 (s, breit, 1H, 2β-H), 3.60 (s, breit, 1H, 3α-H), 4.10 und 4.42 (jeweils: s, breit, 1H; $CH_2$-N), 7.29 - 7.80 (m, überl., 6H, Ar-H), 9.54 (s, breit, OH).

3a-[(3-Hydroxy-naphth-2-yl)-methylamino]-cholan (**40**):

erhalten durch Umsetzung von 3α-[(E)-3-Hydroxy-naphth-2-carbaldimino]-cholan und $NaBH_4$.
$C_{38}H_{57}NO$ (543.87), Klärpunkt: 186.5-189 °C (aus Ethanol/THF = 6:1), $R_f$ = 0.74. $^1$H-NMR: 0.549 (s, 3H, 18-$H_3$), 0.691 (s, 3H, 19-$H_3$); 0.857, 0.879 and 0.900: überl., 21-, 26- and 27-$H_3$), 0.9-2.0 (m, überl., aliph. H), 3.172 (s, 1H, 3β-H), 4.337 (m, 2H, $CH_2$-H), 7.300 - 7.713 (m, überl., 6H, Ar-H).

17α-[(3-Hydroxy-naphth-2-yl)-methylamino]-3-methoxy-estra-1,3,5(10)-trien (**41**): erhalten durch Umsetzung von 17α-[(E)-3-Hydroxy-naphth-2-carbaldimino]-3-methoxy-estra-1,3,5(10)-trien und $NaBH_4$.
$C_{30}H_{35}NO_2$ (441.61), Ausbeute: 90 %. F = 196-198 °C (aus EtOH/THF = 1:1, dann Zusatz von 10 % Wasser), $R_f$ = 0.60. $^1$H-NMR: 0.814 (s, 3H, 18-$H_3$), 1.293-2.291 (m, überl, ca. 13H, aliph. H), 2.706 (t, $^3$J = 8.6 Hz, 1H, 17α-H), 2.850 (s, breit, 2H, 6-$H_2$), 3.784 (s, 3H, 3-MeO), 4.130 (d, $^2$J = 13.7 Hz, 1H, Benzyl-H), 4.273 (d, $^2$J = 13.7 Hz, 1H, Benzyl-H), 6.639 (s, breit, 1H, 4-H), 6.717 (d, $^3$J = 8.2 Hz, 1H, 2-H), 7.204-7.308 (m, überl. 3H, 1-H und 2x Naphthyl-H), 7.390 (t, $^3$J = 7.8 Hz, 1H, Naphthyl-H), 7.508 (s, 1H, Naphthyl-H), 7.692 (d, $^3$J = 8.1 Hz, 2H, 2x Naphthyl-H).
$^{13}$C-NMR: 11.88, 23.46, 26.29, 27.37, 28.82, 29.76, 37.50, 38.71, 42.92, 43.94, 51.96, 51.99, 55.20, 67.69, 110.61, 111.46, 113.82, 123.03, 125.76, 125.93, 126.19, 126.29, 127.16, 127.26, 127.98, 132.51, 134.52, 137.89, 156.50, 157.47.

16β-(2-Hydroxyphenyl-methylamino)-17β-hydroxy-3-methoxy-estra-1,3,5(10)-trien (**42**):
erhalten durch Umsetzung von 16β-[(E)-Salicylidenimino]-17β-hydroxy-3-methoxy-estra-1,3,5(10)-trien und $NaBH_4$.
$C_{26}H_{33}NO_3$ (407.6), Ausbeute: 88 %. F = 163-167 °C. $[\alpha]_D$ + 97.7 ° (c = 0.909). Ber: C 76.62, H 8.16, N 3.44; Gef.: C 75.91, H 8.32, N 3.50. MS (Cl): 408 [$M^+$].
$^1$H-NMR ($CDCl_3$): 0.85 (s, 3H, 18-$H_3$); 3.20 (m, 1H, 16-H); 3.71 (d, 1H, 17-H); 3.76 (s, 3H, $OCH_3$); 3.93 (q, 2H, -$CH_2$); 6.61-7.20 (m, 7H, aromat. H).
$^{13}$C-NMR ($CDCl_3$): 12.39 (C-18), 52.04 (N-$CH_2$), 57.62 (C-16), 80.92 (C-17), 116.30, 119.10, 128.26, 128.74 ($C_6H_4$ ); 123.26 ($H_2C$-$\underline{C}_6H_4$); 157.94 (HO-$\underline{C}_6H_4$).

16α-(2-Hydroxyphenyl-methylamino)-17β-hydroxy-3-methoxy-estra-1,3,5(10)-trien **(43):**
erhalten durch Umsetzung von 16α-[(E)-Salicylidenimino]-17β-hydroxy-3-methoxy-estra-1,3,5(10)-trien und $NaBH_4$.
$C_{26}H_{33}NO_3$ (407.6), Ausbeute: 90,5 %. F = 167-169°C. $[\alpha]_D$ + 26.2 ° (c = 0.979). Ber.: C 76.62, H 8.16, N 3.44, Gef.: C 76.65, H 8.38, N 3.51. MS (Cl): 408 [$M^+$].
$^1$H-NMR ($CDCl_3$): 0.79 (s, 3H, 18-$H_3$); 3.07 (m, 1H, 16-H); 3.50 (d, $^3$J = 6.6 Hz, 1H, 17-H); 3.75 (s, 3H, $OCH_3$); 3.97 (d, $^3$J = 13.9 Hz, 1H, -$CH_2$); 4,09 (d, $^3$J = 13.9 Hz, 1H, -$CH_2$); 6.61-7.18 (m, 7H, aromat. H).
$^{13}$C-NMR ($CDCl_3$): 12.11 (C-18), 51.51 (N-$CH_2$), 63.55 (C-16), 88.17 (C-17), 116.52, 119.17, 128.18, 128.76 ($C_6H_4$); 122.77 ($H_2C$-$\underline{C}_6H_4$); 158.12 (HO-$\underline{C}_6H_4$).

16β-(2-Hydroxyphenyl-methylamino)-17α-hydroxy-3-methoxy-estra-1,3,5(10)-trien **(44):**
erhalten durch Umsetzung von 16β-[(E)-Salicylidenimino]-17α-hydroxy-3-methoxy-estra-1,3,5(10)-trien und $NaBH_4$.
$C_{26}H_{33}NO_3$ (407.6), Ausbeute: 93,5%. F = 158-161 °C. $[\alpha]_D$ + 62.0 ° (c = 0.981). Ber: C 76.62, H 8.16, N 3.44, Gef: C 76.46, H 8.37, N 3.51. MS (Cl): 408 [$M^+$].
$^1$H-NMR ($CDCl_3$): 0.88 (s, 3H, 18-$H_3$); 3.03 (t, 1H, 16-H); 3.70 (s, 1H, 17-H); 3.76 (s, 3H, $OCH_3$); 4.02 (s, 2H, -$CH_2$); 6.62-7.21 (m, 7H, aromat. H).
$^{13}$C-NMR ($CDCl_3$): 17.87 (C-18), 51.99 (N-$CH_2$), 67.88 (C-16), 84.88 (C-17), 116.42, 119.24, 128.23, 128.86 ($C_6H_4$); 122.77 ($H_2C$-$\underline{C}_6H_4$); 157.96 (HO-$\underline{C}_6H_4$).

16α-(2-Hydroxyphenyl-methylamino)-17α-hydroxy-3-methoxy-estra-1,3,5(10)-trien **(45):**
erhalten durch Umsetzung von 16α-[(E)-Salicylidenimino]-17α-hydroxy-3-methoxy-estra-1,3,5(10)-trien und $NaBH_4$.
$C_{26}H_{33}NO_3$ (407.6), Ausbeute: 87.6 %. F = 176-182 °C. $[\alpha]_D$ + 31.4 ° (c = 0.921). Ber: C 76.62, H 8.16, N 3.44, Gef: C 76.46, H 8.37, N 3.51. MS (Cl): 408 [$M^+$].
$^1$H-NMR ($CDCl_3$): 0.71 (s, 3H, 18-$H_3$); 3.42 (m, 1H, 16-H); 3.76 (s, 3H, $OCH_3$); 3.81 (d, 1H, 17-H); 3.98 (q, 2H, -$CH_2$); 6.60-7.20 (m, 7H, aromat. H).
$^{13}$C-NMR ($CDCl_3$): 17.15 (C-18), 51.23 (N-$CH_2$), 58.01 (C-16), 78.14 (C-17), 116.37, 118.98, 128.42, 128.65 ($C_6H_4$); 122.72 ($H_2C$-$\underline{C}_6H_4$ ); 158.33 (HO-$\underline{C}_6H_4$).

3,17β-Diydroxy-16α-(2-hydroxyphenyl-methylamino)-estra-1,3,5(10)-trien (**46**): erhalten durch Umsetzung von 3,17β-Diydroxy-16α-salicylidenimino-estra-1,3,5(10)-trien und $NaBH_4$.
$C_{25}H_{31}NO_3$ (393.52), F = 144-145 °C (aus Methanol), $R_f$ = 0.

16β-(2-Hydroxyphenyl-methylamino)-17β-hydroxy-3-methoxy-13α-estra-1,3,5(10)-trien (**47**):
erhalten durch Umsetzung von 16β-Salicylidenimino-17β-hydroxy-3-methoxy-13α-estra-1,3,5(10)-trien) und $NaBH_4$.
$C_{26}H_{33}NO_3$ (407.55), F = 92-98 °C (aus Methanol), $R_f$ = 0.03.

16α-(2-Hydroxyphenyl-methylamino)-17β-hydroxy-3-methoxy-13α-estra-1,3,5(10)-trien (**48**):
erhalten durch Umsetzung von 16α-Salicylidenimino-17β-hydroxy-3-methoxy-13α-estra-1,3,5(10)-trien) und $NaBH_4$.
$C_{26}H_{33}NO_3$ (407.55), F = 131-136 °C (aus Methanol), $R_f$ = 0.09.

17a-(4-Methoxyphenyl-methylamino)-3-methoxy-estra-1,3,5(10)-trien (**49**):
erhalten durch Umsetzung von 17α-(4-Methoxyphenyl-methylenimino)-3-methoxy-estra-1,3,5(10)-trien) und $NaBH_4$.
$C_{27}H_{35}NO_2$ (405.58), farbloses Öl, $R_f$ = 0.51.
$^1$H-NMR: 0.746 (s, 3H, 18-$H_3$), 1.235-2.371 (m, überl., ca. 13H, aliph. H), 2.733 (d, überl., $^3$J = 5.6 Hz, 1H, 17β-H), 2.865

(s, breit, 2H, 6-$H_2$), 3.361 (d, $^2J$ = 13.0 Hz, 1H, N-Benzyl-H), ca. 3.740 (d, überl., $^2J$ = 13.0 Hz, 1H, N-Benzyl-H), 3.778 (s, 3H, 3-MeO), 3.805 (s, 3H, MeO-Phenyl), 6.637 (s, 1H, 4-H), 6.703 (d, $^3J$ = 8.4 Hz, 1H, 2-H), 6.868 (d, $^3J$ = 8.6 Hz, 2H, 2x Phenyl-H), 7.205-7.264 (2x d, überl., $^3J$ = 8.4 and 8.6 Hz, 2H, 1-H and 2x Phenyl-H).
$^{13}$C-NMR: 19.03, 24.97, 26.46, 28.08, 29.96, 30.30, 32.86, 39.28, 43.49, 45.13, 48.85, 52.27, 55.19, 55.26, 66.77, 111.41, 113.77, 126.31, 129.15, 132.98, 133.19, 138.10, 157.38, 158.50.

14α, 17β-Diydroxy-15β-(2-hydroxyphenyl-methylamino)-estra-1,3,5(10)-trien (**50**): erhalten durch Umsetzung von 14β,17β-Diydroxy-15α-salicylidenimino-estra-1,3,5(10)-trien und $NaBH_4$.
$C_{26}H_{33}NO_4$ (423.56), F = 178-181 °C (aus Ethanol), $R_f$ = 0.07.
14β,17β-Diydroxy-15α-(2-hydroxyphenyl-methylamino)-estra-1,3,5(10)-trien (**51**): erhalten durch Umsetzung von 14β,17β-Diydroxy-15α-salicylidenimino-estra-1,3,5(10)-trien und $NaBH_4$.
$C_{26}H_{33}NO_4$ (423.56), F = 163-164.5 °C (aus Ethanol), $R_f$ = 0.17.

16β-(2-Hydroxy-3,5-di-tert-butylphenyl-methylamino)-17α-hydroxy-3-methoxy-estra-1,3,5(10)-trien (**Ref 1**): erhalten durch Umsetzung von 16β-(2-Hydroxy-3,5-di-tert-butylphenyl-carbaldimino)-17α-hydroxy-estra-1,3,5(10)-trien und $NaBH_4$.
$C_{24}H_{49}NO_4$ (519.76), F = 184.5-186 °C (aus Ethanol/Wasser), $R_f$ = 0.71, Ausbeute 82%, $[\alpha]_D$ + 43 ° (*c* = 0.934).
$^1$H-NMR ((400 MHz, CDCl3, COSYDQF)):
d 5 0.88 (s, 3H, 18-$H_3$); 1.28 (s, 9H, *tert*-Bu),1.43 (s, 9H, *tert*-Bu), 2.85 ($m_c$, 2H, 6-H2), 3.09 (t, Linienformanalyse: 2x d mit 2x $^3J$ ca. 8 Hz, 1H, 16α-H), 3.04 (s, 1H, 17β-H), 3.70 (s, 3H, $OCH_3$), 4.00 ($m_c$, 2H, 2x Benzyl-H); 6.63-7.23 (m, 7H, Ar-H). $^{13}$C-NMR: 53.03 (s, N-$CH_2$), 68.29 (s, C-16), 84.77 (s, C-17).
IR (KBr): 3299 (m) and 3569 (s, NH, OH), keine CH=N-Bande.
MS (70 eV); *m/z* (%) 519 (71) [$M^+$], 214 (100).
HR-MS: Ber: 519.3710; Gef: 519.3712.
EA: Ber: C 78.60, H 9.51, N 2.70; Gef:: C 77.67, H 9.93, N 2.83.

**Synthesen der Aminosteroid-Vorstufen zu oben genannten Ausführungsbeispielen**

**16-Aminosteroide entsprechend Beispiel 1**

16α-Brom-3-methoxy-estra-1,3,5(10)-trien:
Zu einer gerührten Suspension von 2.67 g (15.0 mmol) *N*-Bromosuccinimid in 18 ml trockenem Dichlormethan wird bei 10 °C eine Lösung von 3.93 g (15.0 mmol) Triphenylphosphin in 18 ml trockenem Dichlormethan getropft. Nach 5 min werden 1.72 g (6.0 mmol) 16β-Hydroxy-3-methoxy-estra-1,3,5(10)-trien zugegeben und die Mischung 2h bei Raumtemp. gerührt. Nach Säulenchromatographie des Reaktionsproduktes an Silicagel (60 g) mittels 300 ml Dichlormethan wird die Zielverbindung in Form farbloser Kristalle erhalten.
$C_{19}H_{25}BrO$ (349.3); Ber: C 65.33, H 7.21, Br 22.88; Gef: C 65.40, H 7.41, Br 22.95. Ausbeute 1.86 g (89%), F= 108-111°C. $[\alpha]_D$ + 118° (c = 10.1).
$^1$H-NMR ($CDCl_3$): 0.718 (s, 3H, 18-$H_3$), 2.831 (m, 2 H, 6-$H_2$), 3.757 (s, 3 H, 3-MeO),
4.463 (m, 1 H, 16β-H), 6.607 (s, 1 H, 4-H), 6.694 (m, 1 H, 2-H), 7.183 (d, $^3J$ = 8.62 Hz, 1 H, 1-H).

16β-Azido-3-methoxy-estra-1,3,5(10)-trien:
Eine gerührte Lösung von 1.56 g (4.5 mmol) 16a-Brom-3-methoxy-estra-1,3,5(10)-trien und 0.67 g (13.7 mmol) Lithiumazid in 30 ml of DMSO wird 1 h auf 80 °C erhitzt. Nach dem Abkühlen wird tropfenweise Wasser zugegeben und der erhaltene Niederschlag abfiltriert, mit Wasser gewaschen und im Vakuum getrocknet: 1.33 g (96%). F = 80-85 °C, nach Umkristallisation aus Ether/Methanol: F = 84-87°C. $[\alpha]_D$ + 60° (*c* = 10.2).
$^1$H-NMR: 0.895 (s, 3 H, 18-$H_3$), 2.830 (s, 2 H, 6-$H_2$), 3.760 (s, 3 H, 3-MeO), 4.086 (m, 1 H, 16α-H), 6.613 (s, 1 H, 4-H), 6.690 (m, 1 H, 2-H), 7.181 (d, $^3J$ = 8.62 Hz, 1 H, 1-H). $C_{19}H_{25}N_3O$ (311.4), Ber: C 73.28, H 8.09, N 13.49; Gef: C 73.18, H 7.97, N 13.41.

16β-Amino-3-methoxy-estra-1,3,5(10)-trien:
Zu einer gerührten Lösung von 1.28 g (4.1 mmol) 16β-Azido-3-methoxy-estra-1,3,5(10)-trien in 30 ml abs. THF werden unter Kühlung 10 ml einer 1M Lithiumaluminiumhydrid in THF (Fluka) getropft. Man rührt noch 30 min bei RT, gibt vorsichtig Ether/Wasser bei 0 °C zu und versetzt mit einer Natriumtartrat-Lösung. Nach dreimaliger Extaktion der wässrigen Phase mit Ether werden die vereinigten organischen Phasen zweimal mit Wasser gewaschen, dann mit Natriumsulfat getrocknet und eingeengt. Ausbeute nach Umkristallisation aus Hexan: 1.05 g (89 %), weißer Feststoff mit F = 90-94°C.
$C_{19}H_{27}NO$ (285.4): Ber: C 79.95, H 9.53, N 4.90; Gef: C 79.23, H 9.44, N 4.74.
$[\alpha]_D$ (Pyridin) + 67 ° (c = 9.6).
$^1$H-NMR ($CDCl_3$): 0.923 (s, 3 H, 18-$H_3$), 2.850 (s, 2 H, 6-$H_2$), 3.755 (s, 3 H, 3-MeO), 3.481 (m, 1 H, 16α-H), 6.608 (s, 1 H, 4-H), 6.686 (m, 1 H, 2-H), 7.183 (d, $^3$= 5 8.64 Hz, 1 H, 1-H); nach Zugabe von Trichloracetylisocyanat: 0.918 (s, 1 H, 18-$H_3$), 4.320 (m, 1 H, 16α-H), 8.012 (d, $^3J$ = 7.00 Hz, 1 H, 16β-NH), 8.634 (s, 1H, NH-CO).

3-Methoxy-16β-tosyloxy-estra-1,3,5(10)-trien:
4.5 g (15.7 mmol) 16β-Hydroxy-estra-1,3,5(10)-trien, 9.0 g (47.2 mmol) p-Tosylchlorid und 45 ml abs. Pyridin werden 3 Tage bei 5 °C zur Reaktion gebracht. Nach Zugabe von Eiswasser wird abgesaugt, mit Wasser gewaschen und getrocknet Nach säulenchromatographischer Reinigung (75 g Silicagel, Benzen) erhält man 6.6 g (95 %) des Tosylats. F = 144-150 °C, nach Umkristallisation aus Dichlormethan/
Methanol: F = 151-155 °C.
$C_{26}H_{32}O_4S$ (440.6), Ber: C 70.88, H 7.32, S 7.28; Gef: C 71.05, H 7.46, S 7.31.
$[\alpha]_D$ + 55 ° (c = 10.3).
$^1$H-NMR ($CDCl_3$): 0.894 (s, 3 H, 18-$H_3$), 2.435 (s, 3 H, $CH_3$-phenyl), 3.745 (s, 3 H, 3-MeO), 4.973 (m, 1 H, 16α-H).

16α-Azido-3-methoxy-estra-1,3,5(10)-trien:
3.3 g (7.5 mmol) 3-Methoxy-16β-tosyloxy-estra-1,3,5(10)-trien und 0.99 g (15.7 mmol) Lithiumazid in 66 ml of DMSO werden wie oben beschrieben zur Reaktion gebracht. Ausbeute; 2.15 g (92 %), F = 80-89 °C, nach Umkristallisation aus Aceton: F = 89-92 °C.
$C_{19}H_{25}N_3O$ (311.4): Ber: C 73.28, H 8.09, N 13.49; Gef: C 73.32, H 8.30, N 13.53.
$[\alpha]_D$ + 90° (c = 10.1).
$^1$H-NMR ($CDCl_3$): 0.757 (s, 3 H, 18-$H_3$), 2.834 (s, 2 H, 6-$H_2$), 3.759 (s, 3 H, 3-MeO), 4.052 (m, 1 H, 16β-H), 6.614 (s, 1 H, 4-H), 6.695 (m, 1 H, 2-H), 7.181 (d, $^3$J = 8.52 Hz, 1 H, 1-H).

16α-Amino-3-methoxy-estra-1,3,5(10)-trien:
2.62 g (8.4 mmol) 16a-Azido-3-methoxy-estra-1,3,5(10)-trien in 80 ml abs. THF werden mit 20 ml 1M Lithiumaluminiumhydrid-Lösung wie oben beschrieben zur Reaktion gebracht. Nach Umkristallisation aus Heptan werden 2.05 g (85 %) eines weißen Feststoffes erhalten. F = 82-85 °C.
$C_{19}H_{27}NO$ (285.4), Ber: C 79.95, H 9.53, N 4.90; Gef: C 80.39, H 9.59, N 4.92.
$[\alpha]_D$ (Pyridin) + 81° (c = 9.5).
$^1$H-NMR ($CDCl_3$): 0.746 (s, 3 H, 18-$H_3$), 2.833 (m, 2 H, 6-$H_2$), 3.575 (m, 1 H, 16β-H), 3.754 (s, 3 H, 3-MeO); nach Zugabe von Trichloracetylisocyanat: 0.789 (s, 3 H, 18-$H_3$), 2.828 (m, 2 H, 6-$H_2$), 3.744 (s, 3 H, 3-MeO), 4.390 (m, 1 H, 16β-H), 7.912 (d, $^3$J = 7.23 Hz, 1 H, 16a-NH), 8.662 (s, 1 H, NH=CO).

**17-Aminosteroide entsprechend Beispiel 2**

17β-Amino-3-methoxy-estra-1,3,5(10)-trien:
Zu einer gut gerührten Lösung von 17-Hydroxyimino-3-methoxy-estra-1,3,5(10)-trien (2.42 g, 8.1 mmol) in 200 ml abs. Methanol und 40 ml abs. THF gibt man 2.19 g $MoO_3$ (15.2 mmol). Nach Kühlen auf 0 °C wird in kleinen Portionen bei einer Temperatur zwischen 0 und 10 °C $NaBH_4$ (14.8 g, 15.2 mmol) zugegeben. Man rührt noch 30 min bei RT und gibt dann eine Lösung von 5 g KOH in 20 ml Wasser zu. Man läßt 12 h bei 0 °C stehen und filtriert. Der Filterrückstand wird zweimal mit je 30 ml Methanol
gewaschen. Das Filtrat wird im Vakuum auf 100 ml eingeengt, in Eis/Wasser gegeben und mit $CHCl_3$ (2 x mit je 50 ml) extrahiert. Die vereinigten organischen Phasen werden dreimal mit je 70 ml Wasser gewaschen, über $Na_2SO_4$ getrocknet und eingeengt: 1.7 g (85.8 %) eines farblosen Feststoffes, F = 83-85 °C.
$^1$H-NMR ($CDCl_3$): 0.65 (s, 3H, 18-$H_3$), 2.73 (t, 1H, 17a-H), 2.81 (m, 2H, 6-$H_2$), 3.75 (s, 3H, $OCH_3$), 6.61 (s, 1H, 4-H), 6.69 (m, 1H, 2-H), 7.16 (d, $^3$J = 8.54 Hz; 1H, 1-H); nach Zugabe von Trichloracetylisocyanat (TAI): 0.80 (s, 3H, 18-$H_3$), 3.89 (q, 1H, 17α-H), 7.89 (1H, d, $^3$J = 8.57 Hz, 17β-NHCO), 8.67 (s, 1H, NH). Nach Umkristallisation aus Ether: F = 113-115 °C.

17α-Amino-3-methoxy-estra-1,3,5(10)-trien:

- 3-Methoxyestra-1,3,5(10)-trien-17β-ol (6 g, 0.01 mol) wird in 20 ml Pyridin gelöst und eine Lösung von p-Tolusulfonylchlorid (2.85 g, 0.015 mol) in Pyridin (10 ml) unter Eiskühlung zugetropft. Nach 24 h Stehen bei RT wird auf Eis/Wasser (500 g) gegossen und 10 ml konz. Schwefelsäure zugegeben. Man saugt ab, wäscht gründlich mit Wasser, kristallisiert aus Aceton/Wasser um und trocknet im Exhikkator über $P_2O_5$.
- Die so erhaltene 17β-Tosyloxy-Verbindung (5.16 g, 11.7 mmol) wird mit $NaN_3$ (5.68 g, 102.8 mmol) bei 100 °C in abs. HMPTA (70 ml) unter Zusatz von 18-Crone-6 (1.5 g) 20 h zur Reaktion gebracht. Nach dem Abkühlung wird das Reaktionsgemisch in Eis/Wasser gegeben und mit Methylenchlorid extrahiert. Die organische Phase wird abgetrennt und mehrmals mit Wasser gewaschen. Nach dem Einengen im Vakuum werden 3.5 g (96.1 %) des 17a-Azids in Form eines öligen Rohproduktes erhalten.
  $^1$H NMR ($CDCl_3$) 0.76 (s, 3H, 18-$H_3$), 3.57 (d, 1H, 17β-H).
- Das Azid (710 mg, 2.28 mmol) wird in abs. THF (10 ml) gelöst, $LiAlH_4$ (1 M Lösung in THF, Fluka, 10 ml) zugegeben und 2 h unter Rückfluß erhitzt. Nach Kühlen auf 0 °C wird vorsichtig eine Ether/$H_2O$-Mischung zugegeben, mit einer konz. wässrigen $NH_4Cl$-Lösung versetzt und filtriert. Der Filterrückstand wird mit Ether gewaschen und die wässrige Phase 2 x mit je 30 ml Ether gewaschen. Die vereinigten organischen Phasen werden getrocknet und dann trockenes

gasförmiges HCl eingeleitet. Das ausgefallene Hydrochlorid wird abgesaugt und aus Ethanol umkristallisiert. Zur Gewinnung des Amins wird eine konzentrierte Lösung des Hydrochlorides in Methanol zu einer Lösung von 1 g KOH in 10 ml Wasser gegeben. Nach Ether-Extraktion, waschen mit Wasser und Trocknung erhält man 322 mg (49.5 %) des Amins in Form eines wachsartigen Produktes.

$^1$H-NMR ($CDCl_3$): 0.71 (s, 3H, 18-$H_3$), 2.98 (d, 1H, 17β-H); nach Zugabe von TAI: 0.85 (s, 3H, 18-$H_3$), 3.97 (t, 1H, 17β-H), 7.88 (1H, d, $^3$J = 8.23 Hz, 17a-NHCO), 8.73 (s, 1H, NH).

**Beispiele zur Synthese von Steroidaminoalkoholen entsprechend Beispiel 3 (Schemata 3.1. und 3.2.)**

16β-Amino-3-methoxy-estra-1,3,5(10)-trien-17α-ol:

- 16β-Azido-3-methoxy-estra-1,3,5(10)-trien-17α-ol:

5.0 g 16α,17α-epoxy-3-methoxy-estra-1,3,5(10)-trien, 20 g Natriumazid, 100 ml abs. DMSO und 30 ml Eisessig werden bei 110 °C 7 h unter Argon gerührt. Nach Eingießen in Wasser wird ausgeethert und mit 1 N NaOH und dann mit Wasser gewaschen. Nach dem Trocknen und Abdestillieren des Lösungsmittels wird der Rückstand an 50 g Aluminiumoxid (neutral, Aktivitätsstufe 1) mit Benzol chromatographiert. Umkristallisation aus MethanollWasser ergibt 4.2 g (73 %) des Azids.
$C_{19}H_{25}N_3O_2$ (327,4), Ber. C, 69.70; H, 7.70; N, 12.83; Gef: C, 69.77; H, 752; N, 12.72.
F = 100-101 °C; $[\alpha]_D$ +86°. IR: 2092 ($N_3$), 3610 (OH);
$^1$H-NMR: 3.75 (16α-H, 17β-H, $OCH_3$), 17α-$OCONHCOCCl_3$-Verbindung: 4.85 (s, 17β-H).

- 16β-Amino-3-methoxy-estra-1,3,5(10)-trien-17α-ol:

1.0 g 16β-Azido-3-methoxy-estra-1,3,5(10)-trien-17α-ol, 10 ml Methanol, 2 ml 80 % Hydrazinhydrat und eine Spatelspitze Raney-Nickel werden 15 min auf dem Wasserbad erhitzt. Danach wird abfiltriert, das Raney-Nickel mit Methanol gewaschen, Waschflüssigkeit und Filtrat vereinigt und das Methanol i. Vak. abdestilliert. Der Rückstand wird aus Methanol/Ether umkristallisiert. Ausbeute: 0.8 g (87 %), F = 158-160 °C; $[\alpha]_D$ + 68° (Pyridin).
$C_{19}H_{27}NO_2$ (301.4), Ber: C, 75.71; H, 9,031 N, 4.65; Gef: C, 75.60; H, 8.99; N, 4.51. IR: 3365 cm (NH). 3605 (OH).

16α-Amino-3-methoxy-estra-1,3,5(10)-trien-17β-ol:

- 17α-Azido-3-methoxy-estra-1,3,5(10)-trien-16β-ol:

1.0 g 16β,17β-Epoxy-estra-1,3,5(10)-trien, 4.0 g Natriumazid, 20 ml abs. DMSO und 3 ml Eisessig werden 4 h bei 120 °C unter Argon gerührt. Die Aufarbeitung erfolgt wie oben beschrieben. Das trockene Rohprodukt wird durch präparative Schichtchromatographie an Kieselgel PF (Fa. Merck) mit Benzol/Ether 3:2 als Laufmittel in zwei Fraktionen getrennt. Falls notwendig, werden beide Fraktionen durch präparative Schichtchromatographie weiter gereinigt. Man erhält aus der polaren Fraktion 0.23 g 17α-Azido- 3-methoxy-estra-1,3,5(10)-trien-16β-ol (20%) als farbloses Öl.
$^1$H-NMR: 3.50 (s, 17β-H), 425 (m, 16α-H); 16β-$OCONHCOCCl_3$-Verbindung: 3.71 (s, 17β-H), 5.13 (m, 16α-H).

- 16a-Azido-3-methoxy-estra-1,3,5(10)-trien-17β-ol:

Variante 1: Aus der unpolareren Fraktion, die bei der Darstellung von 17α-Azido-3-methoxy-estra-1,3,5(10)-trien-16β-ols durch präparative Schichtchromatographie erhalten wird, werden durch Umkristallisation aus Methanol/Wasser 0.25 g 16α-Azido-3-methoxy-estra-1,3,5(10)-trien-17β-ol (22%) erhalten. F= 112-114 °C; $[\alpha]_D$ + 38°. IR: 2090 ($N_3$), 3612 (OH); NMR: ca. 3.68 (16β-H, 17α-H, $OCH_3$). 17β-$OCONHCOCCl_3$-Verbindung: 3.85 (m, 16β-H), 4.88 (d, 17α-H).
$C_{19}H_{25}N_3O_2$ (327.4), Ber: C. 69.70: H. 7.70: N. 12.83; Gef: C, 69.71; H, 7.53; N, 12.86.
Variante 2: 18.2 g 16β-Brom-3-methoxy-estra-1,3,5(10)-trien-17β-ol werden mit 10.8 g Natriumazid in 180 ml DMSO 40 min bei 90 °C gerührt. Nach dem Abkühlen wird Wasser zugetropft, das Produkt abgesaugt, mit Wasser gewaschen und getrocknet. Zur Abtrennung der Ketonfraktion wird das Rohprodukt in 90 ml Methanol und 6.8 ml Eisessig mit 6.1 g *Girards Reagens P* 1 h erhitzt. Danach wird in Wasser, das 5.7 g Natriumcarbonat (wasserfrei) enthält, eingegossen. Man extrahiert mit Ether, wäscht mit Wasser, trocknet und destilliert das Lösungsmittel ab. Der Rückstand wird aus Methanol/Wasser umkristallisiert. Ausbeute: 14.6 g (89 %), F= 112-114 °C.

- 16α-Amino-3-methoxy-estra-1,3,5(10)-trien-17β-ol:

5.0 g 16α-Azido-3-methoxy-estra-1,3,5(10)-trien-17β-ol, 100 ml Methanol, 10 ml 80 % Hydrazinhydrat und 4 Spatelspitzen Raney-Nickel werden 30 min auf dem Wasserbad erhitzt. Aufarbeitung und Umkristallisation erfolgen wie bei der Darstellung von 16α-Amino-3-methoxy-estra-1,3,5(10)-trien-17β-ol beschrieben. Ausbeute: 3.2 g (69 %). F = 158-161 °C; $[\alpha]_D$ + 41° (Pyridin). IR: 3367 (NH). 3601 (OH);
$C_{19}H_{27}NO_2$ (301.4), Ber: C, 75.71; H, 9.03; N, 4.65; Gef: C, 75.78; H, 9.02; N, 4.65.

16β-Amino-3-methoxy-estra-1,3,5(10)-trien-17β-ol:

1.0 g 16β-Azido-3-methoxy-estra-1,3,5(10)-trien-17β-ol werden mit 20 ml Methanol, 2 ml 80 % Hydrazinhydrat und einer Spatelspitze Raney-Nickel in der bei der Darstellung von 16betra-Amino-3-methoxy-estra-1,3,5(10)-trien-17α-ol beschriebenen Weise reduziert. Die Umkristallisation erfolgt aus Methanol/Ether. Ausbeute: 0.7 g (76 %). F= 152-157 °C; $[\alpha]_D$ + 59° (Pyridin). IR: 3300 (breit), 3417 (OH, NH), 3601 (OH).
$C_{19}H_{27}NO_2$ (301.4), Ber: C, 75.71; H, 9.03; N, 4.65; Gef: C, 75.55; H, 8.86; N, 4.56.

16α-Amino-3-methoxy-estra-1,3,5(10)-trien-17α-ol:

- 16a-Azido-3-methoxy-estra-1,3,5(10)-trien-17-on:

Zu einer Lösung von 20 g 16α-Azido-3-methoxy-estra-1,3,5(10)-trien-17β-ol in 1l Aceton werden bei 0 °C unter Rühren 40 ml einer 8 N Chromtrioxidlösung in konz. Schwefelsäure zugetropft. Nach 2 h werden 2 ml Isopropanol zugesetzt und das Reaktionsprodukt durch Zutropfen von Wasser in kristalliner Form ausgefällt. Man saugt ab, wäscht mit Wasser, trocknet und kristallisiert aus Aceton/Wasser um. Ausbeute: 16 g (81 %), F= 118-122 °C; $[\alpha]_D$ + 405°. IR: 1747cm (CO), 2095 ($N_3$). $C_{19}H_{23}N_3O_2$ (325.4); Ber: C, 70.13; H, 7.12; N, 12.91; Gef: C, 70.23; H, 6.97; N, 12.71.

- 16α-Azido-3-methoxy-estra-1,3,5(10)-trien-17α-ol:

Zu einer Suspension von 3.0 g16α-Azido-3-methoxy-estra-1,3,5(10)-trien-17-on in 75 ml abs. Ether werden bei 0 °C in einer Schutzgasatmosphäre unter Rühren 0.7 g Lithiumborhydrid portionsweise zugegeben. Nach 80 min setzt man wenig Aceton zu und nach weiteren 5 min. wird mit Wasser versetzt. Die Ether-Phase wird abgetrennt, nochmals mit Wasser gewaschen und getrocknet. Der nach dem Abdestillieren des Lösungsmittels verbleibende Rückstand wird an 75 g Kieselgel mit 2 l Benzol chromatographiert. Ausbeute nach Umkristallisation aus Methanol: 2.0 g (67 %). F= 97-99 °C; $[\alpha]_D$ 0°; IR: 2110 ($N_3$). 3540, 3610 (OH).
$C_{19}H_{25}N_3O_2$ (327.4), Ber: C, 69.70; H, 7.70; N, 12.83; Gef: C, 69.55; H, 7.74; N, 12.79.
Mit 150 ml Aceton werden abschließend noch 0.4 g 16α-Azido-3-methoxy-estra-1,3,5(10)-trien-17β-ol (13 %) eluiert.

- 16α-Amino-3-methoxy-estra-1,3,5(10)-trien-17a-ol:

2.0 g 16α-Azido-3-methoxy-estra-1,3,5(10)-trien-17α-ol werden mit 40 ml Methanol, 4 ml 80 % Hvdrazinhvdrat und 2 Spatelspitzen Raney-Nickel in der oben beschriebenen Weise reduziert. Die Umkristallisation erfolgt aus Methanol/Ether. Ausbeute: 1.3 g (71 %), F: bei 145 °C Umwandlung, bei 193-198 °C Schmelzen; $[\alpha]_D$ + 44 ° (Pyridin); IR: 3290 (breit), 3400 (NH, OH), 3618 (OH).
$C_{19}H_{27}NO_2$ (301.4); Ber: C, 75.71; H, 9.03; N, 4.65; Gef: C, 75.71; H, 9.13; N, 4.73.

**Aminocholane entsprechend Beispiel 4**

3α-Azido-5α-cholestan:

4 g (7.37 mmol) 3β-Tosyloxy-5α-cholestan und 0.99 g (15.23 mmol)
$NaN_3$ werden in 25 ml Hexamethylphosphorsäuretriamid (HMPT) 3 h auf 90 °C unter Rühren erhitzt. Die abgekühlte Reaktionsmischung wird in 100 ml Eiswasser gegeben, das ausgefallene Rohprodukt abfiltriert und mit Wasser gewaschen. Man löst in 60 ml Ether. Die organische Phase wird zweimal mit Wasser gewaschen, über $Na_2SO_4$ getrocknet und eingeengt. Das erhaltene wachsartige wird direkt weiterverarbeitet:
Ausbeute 95 %, F = 49-51 °C. IR: 2103 (vs, $N_3$). $C_{27}H_{47}N_3$ (413.7), Ber: C 78.39, H 11.45, N 10.16; Gef: C 78.11, H 11.40, N 9.98.

3α-amino-5α-cholestan:

Zu einer Lösung von 2.04 g (4.83 mmol) 3a-Azido-5a-cholestan in 50 ml THF werden vorsichtig 16 ml (16 mmol) einer 1 M Lösung von $LiAlH_4$ in Ether getropft. Man erhitzt 4 h unter Rückfluß, kühlt auf RT und gibt vorsichtig 5 ml einer gesättigten $NH_4Cl$-Lösung zu. Man filtriert, wäscht den Filterrückstand zweimal mit Ether, trocknet die etherische Phase über $Na_2SO_4$ und engt ein. Das Amin wird als weißer Festkörper erhalten und direkt weiterverarbeitet. Ausbeute 96 %, F = 95-97 °C. $C_{27}H_{49}N$ (387.7), Ber: C 83.65, H 12.74, N 3.61; Gef: C 83.30, H 12.42, N 3.65.

**Aminoalkohole des Cholans entsprechend Beispiel 4**

1α-Azido-cholestan-2β,3β-diol-3-acetat:

2 g 1,2β-Epoxy-cholestan-3β-ol-acetat werden mit 6 g Natriumazid in 75 ml Dimethylsulfoxyd in Gegenwart von einigen Tropfen konz. Schwefelsäure 20 h unter Rühren auf dem siedenden Wasserbad erhitzt. Es wird dann mit Eiswasser verdünnt, der Niederschlag in Ether aufgenommen, die etherische Lösung mehrmals mit Wasser gewaschen, getrocknet und das Lösungsmittel abdestilliert. Der Rückstand kristallisiert aus Hexan in farblosen Nadeln vom Schmp. 163 °C. Ausbeute 1.5 g. $C_{29}H_{49}N_3O_3$ (487.7); Ber: C 71.41, H 10.13, N 8.62; Gef: C 71.56, H 10.08, N 8.76. $[\alpha]_D$ +49 ° (c = 2).

1α-Azido-cholestan-2β,3β-diol

Variante 1: Werden 0.5 g 1α-Azido-cholestan-2β,3β-diol-3-acetat 30 min. mit 5-proz. methanolischem Kaliumhydroxyd unter Rückfluß erhitzt, fallen beim Verdünnen mit Wasser farblose Kristalle aus, die nach dem Umkristallisieren aus waßrigem Methanol bei 143-144 °C schmelzen. Ausbeute 0.41 g. $C_{27}H_{47}N_3O_2$ (445.7), Ber:. C72.75, H 10.64, N 9.43; Gef:. C 72.80, H 10.61, N 9.59. $[\alpha]_D$ + 27° (c = 2).
Variante 2: Werden 2.1 g 1,2β-Epoxy-cholestan-3β-ol mit $NaN_3$ in Dimethylsulfoxyd umgesetzt, so erhält man 1.7 g farblose Kristalle (aus Wasser/Methanol) vom Schmp. 143-144 °C.

1α-Amino-cholestan-2β,3β-diol-3-acetat:

Beim Erhitzen einer Lösung von 2.7 g 1α-Azido-cholestan-2β,3β-diol-3-acetat in 25 ml Ethanol mit 8 ml Hydrazinhydrat und 1 Spatelspitze Raney-Nickel kristallisieren nach ungefähr 1 min farblose Blättchen aus. Es wird so viel Ethanol zugegeben, bis sich der Niederschlag löst. Nach 5 min wird der Katalysator abfiltriert. Es kristallisieren beim Erkalten farblose Blättchen aus, die nach dem Umkristallisieren aus Ethanol bei 188-189 °C schmelzen. Ausbeute 1.1 g. $C_{29}H_{51}NO_3$ (461.7). $[\alpha]_D$ +30 °C (Pyridin).
Aus den vereinigten Mutterlaugen können noch 0.9 g der Zielverbindung vom Schmp. 186-189 °C gewonnen werden.

1α-Amino-cholestan-2β,3β-diol:

0.5 g des vorstehenden Acetats werden in 5-proz. methanolischem Kaliumhydroxyd gelöst, 20 min unter Rückfluß erhitzt, dann mit Wasser verdünnt und der ausgeschiedene Niederschlag aus Ethanol umkristallisiert. Man erhält 0.35 g farblose Blättchen vom Schmp. 188-189 °C.
$C_{27}H_{49}NO_2$ (419.7). Ber: C 77.27, H 11.77, N 3.34; Gef: C 77.13, H 12.07, N 3.40. $[\alpha]_D$ + 31 ° (Pyridin).

2β-Amino-cholestan-3-ol:

- 4 g 2β-Azido-cholestan-3-ol werden in 50 ml absol. Ether mit 0.2 g Lithiumaluminiumhydrid einen Tag bei Raumtemp. gerührt. Es wird dann vorsichtig mit Wasser zersetzt, mehrmals ausgeethert, die vereinigten etherischen Lösungen mit Wasser gewaschen, über Natriumsulfat getrocknet und das Lösungsmittel auf 50 ml eingeengt. Beim Einleiten von Chlorwasserstoff kristallisiert das Hydrochlorid in feinen farblosen Nadeln aus, die nach dem Umkristallisieren aus salzsäurehaltigem Methanol bei 254-256 °C schmelzen.
  $C_{27}H_{49}NO$-HCl (440.1); Ber: N 3.18, Gef: N 3.34.
- 0.2 g des Hydrochlorids werden in wenig heißem Methanol gelöst und bis zur Trübung verd. Natronlauge zugegeben. Es kristallisieren 0.15 g farblose Blättchen aus, die nach dem Umkristallisieren aus Methanol bei 206 -207" schmelzen.
  $C_{27}H_{49}NO$ (403.7), Ber: C 80.32, H 12.24, N 3.47: Gef: C 80.56, H 12.26, N 3.64; $[\alpha]_D$ + 34° (Pyridin).

3α-Amino-cholestan-2β-ol:

- Variante 1: Zur Lösung von 1 g 3α-Azido-cholestan-2β-ol in 10 ml Ethanol werden 3 ml 80-proz. Hydrazinhydrat und eine Spatelspitze Raney-Nickel gegeben und zum Sieden erhitzt. Die Reduktion erfolgt unter stürmischer Gasentwicklung, und nach ungefähr 1 min kristallisiert der Aminoalkohol aus. Man bringt ihn durch Zugabe von Ethanol in Lösung und erhitzt, bis die Gasentwicklung beendet ist. Der Katalysator wird dann abfiltriert und die auskristallisierte Base aus Ethanol umkristallisiert. Schmp. 203 -204 °C. Ausb. 0.65 g. $C_{27}H_{49}NO$ (403.7), Ber: C 80.32, H 12.24, N 3.47; Gef: C 80.30, H 12.21, N 3.54; $[\alpha]_D$ +38 ° (Pyridin). Ausb. 0.25 g.
- Variante 2: 0.5 g Azidoalkohol werden mit $LiALH_4$ reduziert, F = 202 °C, Ausbeute 0.25 g.

4β-Amino-cholestan-5α-ol:

1 g 4β-Azido-cholestan-5α-ol wird in Ethanol mit Hydrazinhydrat/Raney-Nickel reduziert und aus Methanol umkristallisiert. Schmp. 116 °C. Ausb. 0.5 g. Aus den Mutterlaugen können noch 0.3 g Aminoalkohol vom Schmp. 113 -116 °C gewonnen werden.
$C_{27}H_{49}NO$ (403.7), Ber: C 80.32, H 12.24, N 3.47; Gef: C 80.47, H 12.09, N 3.57; $[\alpha]_D$ + 42° (Pyridin).

5α-Amino-cholestan-3β,6β-diol:

Wird 1 g 5α-Azido-cholestan-3β,6β-diol-3-acetat mit $LiAlH_4$ reduziert, so kristallisieren aus Methanol 0.6 g farblose Nadeln vom Schmp. 242-243 °C. $C_{27}H_{49}N0_2$ (419.7); Ber: C 77.27, H 11.77, N 3.34; Gef: C 77.55, H 11.85, N 3.35. $[\alpha]_D$ -6 ° (Pyridin).

5α-Amino-cholestan-3β,6β-diol-3-acetat:

Werden 0.75 g 5-Azido-cholestan-3β,6β-diol-3-acetat nach der Hydrazinhydrat-Methode reduziert, so erhält man aus Methanol 0.6 g farblose Nadeln vom Schmp. 179 °C. $C_{29}H_{51}N0_3$ (461.7), Ber: C 75.44, H 11.13, N 3.03; Gef: C 75.67, H 11.33, N 3.25.
$[\alpha]_D$ - 22° (Pyridin).

6β-Amino-cholestan-3β,5α-dio3-acetat:

Die Reduktion von 3.5 g 6β-Azido-cholestan-3β,5α-diol-3-acetat mit Hydrazinhydrat/Raney-Nickel gibt 2.8 g farblose Blättchen vom Schmp. 190-191 °C (aus Ethanol).
$C_{29}H_{51}N0_3$ (461.7); Ber: C 75.44, H 11.13, N 3.03; Gef: C 75.13, H 11.10, N 3.22. $[\alpha]_D$ - 17 ° (Pyridin).

6β-Amino-cholestan-3β,5α-diol:

Werden 1.25 g 6β-Amino-cholestan-3β,5α-dio-3-acetat mit 8 ml 5-proz. methanolischer Kaliumhydroxidlösung 30 min unter Rückfluß erhitzt, so kristallisieren beim Abkühlen farblose Nadeln aus, die nach dem Umkristallisieren aus Methanol bei 199-200 °C schmelzen. Ausbeute 0.95 g.
$C_{27}H_{49}N0_2$ (410.7), Ber: C 77.27, H 11.77, N 3.34; Gef: C 77.01, H 11.61, N 3.60. $[\alpha]_D$ + 1 ° (Pyridin).

6β-Amino-cholestan-3β,7α-diol:

1.25 g 6ß-Azido-cholestan-3ß,7a-diol-3-acetat werden, wie oben beschrieben, mit $LiAlH_4$ reduziert, dann mit Ether verdünnt und langsam 2N $H_2S0_4$ bis zur deutlich sauren Reaktion zugegeben. Der kristalline Niederschlag wird abgesaugt, mit Ether gewaschen und aus Methanol umkristallisiert. Schmp. 257-258 °C (Zers.). Durch Lösen des Sulfats in heißem Methanol und Zugeben von verd. Natronlauge erhält man farblose Nadeln, die nach dem Umkristallisieren aus Benzol/Methanol (1:3) und dann aus Methanol bei 191-192 °C schmelzen. Ausbeute 0.8 g.
$C_{27}H_{49}N0_2$ (419.7), Ber: C 77.26, H 11.77, N 3.34; Gef: C 77.19, H 11.73, N 3.41. $[\alpha]_D$ +21 ° (Pyridin).

6β-Amino-cholestan-3β, 7α-diol-3-acetat:

Werden 0.8 g 6β-Azido-cholestan-3β,7α-diol-3-acetat in Ethanol mit Hydrazinhydrat/Raney-Nickel reduziert, so erhält man nach dem Umkristallisieren aus Methanol 0.5 g farblose Nadeln vom Schmelzpunkt 136 °C.

$C_{29}H_{51}NO_3$ (461.7), Ber: C 75.44, H 11.13, N 3.03; Gef: C75.20, H 11.41, N 3.31. $[\alpha]_D$ - 1 ° (Pyridin).

7α-Amino-cholestan-3β,6β-dial:

Reduziert man 1.5 g 7a-Azido-cholestan-3β,6β-diol-3-acetat mit $LiAlH_4$ in absol. Ether und zersetzt mit verd. Salzsäure, so erhalt man nach dem Umkristallisieren aus Methanol 1.2 g des Hydrochlorids vom Schmp. 303-304 °C. $C_{27}H_{56}NO_2$-HCl (456.2), Ber: N 3.07; Gef: N 3.10.
0.8 g des Hydrochlorids werden in heißem Methanol gelöst und bis zur Trübung verd. Natronlauge zugegeben. Es fallen farblose Nadeln aus, die nach dem Umkristallisieren aus Methanol bei 217-218 °C schmelzen. Ausb. 0.51 g. $C_{27}H_{49}N0_2$ (419.7), Ber: C 77.26, H 11.77, N 3.34; Gef: C 77.30, H 11.50, N 3.38. $[\alpha]_D$ + 14 ° (Pyridin).

7α-Amino-cholesfan-3β,6β-diol-3-acetat:

1.25 g 7α-Azido-cholestan-3β,6β-diol-3-acetat werden mit Hydrazinhydraf/Raney-Nickel in Athanol reduziert. Aus Ethanol erhält man 0.75 g
farblose Kristalle vom Schmp. 212 °C. $C_{29}H_{51}N0_3$ (461.7), Ber: C 75.44, H 11.13, N 3.03; Gef: C75.34, H11.01, N 3.16. $[\alpha]_D$ -13 ° (Pyridin).

Beispiel 9: Testen von Wirkstoffkombinationen

Um das Potential der erfindungsgemäßen Verbindungen in Kombinationstherapien mit anderen, klinisch bereits etablierten Antimalariamitteln einschätzen zu können, wurden die Substanzen 2, 18 und 41 in Kombinationsassays mit Chloroquin, Artemisinin, Artesunate, Artemeter, Mefloquin sowie Methylenblau getestet. Auffällig war ein deutlicher Synergieeffekt aller drei Substanzen mit Artemisinin und seinem Derivat Artesunate. Dies spiegelte sich in "fractionary inhibitory concentration ($FIC_{50}$ und $FIC_{90}$) Werten" wider, die kleiner als 1 sind.
Methode: Zur Bestimmung der Synergieeffekte wurden Isotopen-basierte Sensitivitäts-Assays auf der Basis der halbautomatisierten Mikroverdünnungsmethode eingesetzt. Die Methode basiert auf der Inkorporation von [3H]-Hypoxanthin, das von den Parasiten als Prekursor der Purindeoxynukleotide für die DNA-Synthese aufgenommen wird. In 96-Well Mikrotiterplatten (Nunc) wurde eine zweifache serielle Verdünnungsreihe der Startkonzentration jeder getesteten Substanz angesetzt. Die beiden Substanzen, die in Kombination getestet werden sollten, wurden jeweils alleine sowie in einem festgelegten Konzentrationsverhältnis von 1:1, 1:3 und 3:1 eingesetzt.
Die Parasiten *(Plasmodium falciparum,* Stamm 3D7) wurden bei einer Parasitämie von 0,125% (ca. 70% Ringstadien) und einem Hämatokrit von 1,25% in Hypoxanthinfreiem Medium inkubiert. Nach 48 Stunden wurde 0,5 Ci [3H]-Hypoxanthin zu jeder Vertiefung zugesetzt und die Platten wurden weitere 24 h inkubiert. Die Zellen jeder Vertiefung (jedes Wells) wurden auf einem Plexiglas-Filter (Perkin-Elmer, Rodgau-Jügesheim, Germany) geerntet, dann gewaschen und getrocknet. Ihre Radioaktivität in "counts per minute" wird bei dieser Methode als proportional zum Wachstum der Parasiten angenommen. Die $IC_{50}$-Werte sowie die $IC_{90}$-Werte, also die Wirkstoffkonzentrationen, die 50% bzw. 90% Reduktion der [3H]-Hypoxanthin-Aufnahme bewirken, wurden berechnet. Die "fractional inhibitory concentrations (FIC)" der jeweiligen Wirkstoffe wurden wir folgt berechnet:

$$FIC_{50}\ A = [IC_{50}\ (A + B)]/IC_{50}\ A;$$

$$FIC_{50}\ B = [IC_{50}\ (B + A)]/IC_{50}\ B;$$

und

$$FIC_{50} = FIC_{50}\ A + FIC_{50}\ B.$$

Gleiche Berechnungen wurden auch mit den jeweiligen $IC_{90}$-Werten durchgeführt. FIC-Werte größer als 1 zeigen sich in konvexen Isobologrammen und indizieren Antagonismus von Wirkstoffen. Die fünf Messwerte, d.h. jeweils die IC-Werte der Einzelsubstanzen sowie diejenigen der fixen Wirkstoffkombinationen 1:1, 1:3 und 3:1, liegen oberhalb der gestrichelten Linie.
FIC-Werte von 1 weisen auf additive Wirkung von Substanzen hin. Die Messwerte liegen auf der gestrichelten Linie.
FIC-Werte kleiner als 1 weisen auf Synergieeffekte der beiden kombinierten Substanzen hin. Die Messwerte liegen unterhalb der gestrichelten Linie und die Isobologramme sind konkav.

Für methodische Details s. Akoachere M, Buchholz K, Fischer E, Burhenne J, Haefeli WE, Schirmer RH, Becker K (2005) In vitro assessment of methylene blue on chloroquine-sensitive and -resistant Plasmodium falciparum strains reveals synergistic action with artemisinins. Antimicrob Agents Chemother 49: 4592-4597.

In Figur 3 und 4 sind beispielhaft aufgeführt die Isobologramme für Substanz 18 mit Artemisinin und Artesunate. Für die Diagramme wurden jeweils $FIC_{90}$-Werte verwendet. Des weiteren sind die Isobologramme gezeigt für Substanz 2 ($FIC_{90}$-Werte) und Substanz 41 ($FIC_{50}$-Werte) mit Artemisinin. Alle Versuche wurden mit dem *Plasmodium falciparum* Stamm 3D7 durchgeführt. Alle Isobologramme zeigen einen deutlichen synergistischen Effekt der jeweils getesteten Wirkstoffe.

**Beispiel 10: *In vitro* -Behandlung von adulten S. *mansoni* Pärchen mit den Verbindungen 41, 18 und 26 für 24 Stunden**

Die drei Verbindungen 2, 18 und 41 wurden von Prof. Dr. Grevelding, Parasitologie, Veterinärmedizin, Universität Gießen, auch an dem Parasiten *Schistosoma mansoni* getestet. Alle drei Substanzen zeigten eine deutliche Wirkung auf die Parasiten, jedoch mit deutlichen Unterschieden hinsichtlich der getesteten Verbindung, der eingesetzten Konzentrationen, sowie der makroskopisch erkennbaren phänotypischen Auswirkungen. Es wurde jeweils mit 3 Gruppen a 10 Würmern gearbeitet. Auffällig war, dass es unterschiedliche Effekte auf die Würmer hinsichtlich morphologischer Veränderungen gab. Verbindung 41 führte zu dorsalen Einkrümmungen, wohingegen Verbindung 18 zu ventralen Einkrümmungen führte. Bei Verbindung 2 erschienen die Würmer ventral bis Korkenzieher-artig verbogen zu sein. Beispielhaft sind in Figur 1 und 2 eine Aufnahme eines Kontrollversuchs und eine Aufnahme von Saugwürmern nach 48 h Inkubation mit Verbindung 18 gezeigt. Je drei Gruppen (eine Gruppe je well) mit je 10 Pärchen von adulten S. *mansoni* wurden mit der zu testenden Substanz 24h lang inkubiert. Als Kontrolle dienten drei Gruppen, die mit Medium (M199) und drei Gruppen die mit Medium und DMSO als Lösungsmittel der Testsubstanzen inkubiert wurden. Die Auswertung erfolgte visuell mittels Mikroskop.

Ergebnisse:

Die Pärchen der Kontrollgruppen (nur Medium oder DMSO-Kontrolle) waren nach 24 Stunden alle angesaugt, sahen vital und normal aus und hatten eine gestreckte Form. Auch die Darmperistaltik und Saugnapfbewegung war vorhanden. Die Eiproduktion war ebenfalls normal.

Nach einer 24-stündigen Inkubation mit 20 μM der erfindungsgemäßen Verbindung 41 war lediglich in zwei der Gruppen ein Pärchen zu erkennen. Die Saugwürmer hatten sich alle abgelöst. Die Darmperistaltik war vermindert und eine Saugnapfbewegung war nicht mehr zu erkennen. Die Männchen zeigten kaum Bewegungen, nur Zuckungen, wohigegen die Weibchen mehr zuckende Bewegungen als die Männchen durchführten. Die Männchen waren zur dorsalen Seite hin gekrümmt. Es waren nahezu keine Eier zu erkennen.

Nach einer 24-stündigen Inkubation mit 100 μM der erfindungsgemäßen Verbindung 41 war lediglich in einer der Gruppen ein Pärchen zu erkennen. Die Saugwürmer hatten sich alle abgelöst. Die Darmperistaltik und Saugnapfbewegung war nicht mehr zu erkennen. Die Männchen zeigten kaum Bewegungen, nur seltene, spontane Zuckungen konnten beobachtet werden. Auch die Weibchen zeigten nur noch spontane, vereinzelte und zuckende Bewegungen. Die Männchen waren zur dorsalen Seite hin gekrümmt. Es waren nahezu keine Eier zu erkennen.

Nach einer 24-stündigen Inkubation mit 20 μM der erfindungsgemäßen Verbindung 18 war kein Pärchen mehr zu erkennen. Die Saugwürmer hatten sich alle abgelöst. Die Darmperistaltik oder eine Saugnapfbewegung war nicht mehr zu erkennen. Die Männchen zeigten kaum Bewegungen, nur Zuckungen, wohigegen die Weibchen mehr zuckende Bewegungen als die Männchen durchführten. Die Saugwürmer hatten eine gestreckte bis leicht ventral gekrümmte Form. Es waren nahezu keine Eier zu erkennen.

Nach einer 24-stündigen Inkubation mit 100 μM der erfindungsgemäßen Verbindung 18 war kein Pärchen mehr zu erkennen. Die Saugwürmer hatten sich alle abgelöst. Die Darmperistaltik und eine Saugnapfbewegung war nicht mehr zu erkennen. Die Saugwürmer zeigten nur sehr leichte, seltene Bewegungen. Die Männchen sowie die Weibchen sind stark ventral gekrümt. Es waren nahezu keine Eier zu erkennen.

Nach einer 24-stündigen Inkubation mit 20 μM der erfindungsgemäßen Verbindung 2 waren je Gruppe 4 - 6 Pärchen vorhanden. Die Saugwürmer hatten sich alle abgelöst. Die Darmperistaltik war vermindert und eine Saugnapfbewegung war nicht mehr zu erkennen. Die Saugwürmer bewegten sich nur leicht und zuckend. Es waren nahezu keine Eier zu erkennen.

Nach einer 24-stündigen Inkubation mit 100 μM der erfindungsgemäßen Verbindung 2 war in zwei Gruppen je ein Pärchen zu erkennen. Die Saugwürmer hatten sich alle abgelöst. Die Darmperistaltik und eine Saugnapfbewegung waren nicht mehr zu erkennen. Die Männchen und Weibchen waren sehr gekrümmt und in sich verwunden. Die Männchen sowie die Weibchen hatten Aufblähungen/Blasen im Darm-Bereich. Die Saugwürmer bewegten sich nur leicht und zuckend.Es waren nahezu keine Eier zu erkennen.

**Beispiel 11: *In vitro* -Behandlung von adulten S. *mansoni* Pärchen mit den Substanzen 41, 18 und 26 für 48 Stunden**

Es wurden in einem zweiten Ansatz nochmals je drei Gruppen (eine Gruppe je well) mit je 8 Pärchen von adulten S. *mansoni* mit der zu testenden Substanz inkubiert, diesmal jedoch 48 h lang. Als Kontrolle dienten drei Gruppen, die mit Medium (M199) oder mit Medium und DMSO als Lösungsmittel der Testsubstanzen inkubiert wurden. Die Auswertung erfolgte visuell mittels Mikroskop.

Ergebnisse:

Die Pärchen der Kontrollgruppen (nur Medium und DMSO-Kontrolle) waren nach 48 Stunden alle gepaart und angesaugt; sahen vital und normal aus und hatten eine gestreckte Form. Auch die Darmperistaltik und Saugnapfbewegung war vorhanden. Die Eiproduktion war ebenfalls normal. Siehe Figur 1.

Nach einer 48-stündigen Inkubation mit 20 μM der erfindungsgemäßen Verbindung 41 war in keiner der Gruppen ein Pärchen zu erkennen. Die Saugwürmer hatten sich alle abgelöst. Die Darmperistaltik war vermindert und eine Saugnapfbewegung war nicht mehr zu erkennen. Die Saugwürmer führten vereinzelt nur noch leichte Zuckungen aus, sonst waren sie unbeweglich. Viele Männchen waren zur dorsalen Seite hin gekrümmt. Es wurden keine Eier gebildet.

Nach einer 48-stündigen Inkubation mit 100 μM der erfindungsgemäßen Verbindung 41 war kein Pärchen mehr zu erkennen. Einige der Saugwürmer waren bereits tot, andere fast tot. Einzelne Würmer hatten leichte, spontane Zuckungen. Die Würmer waren meist gestreckt. Es waren keine Eier zu erkennen.

Nach einer 48-stündigen Inkubation mit 20 μM der erfindungsgemäßen Verbindung 18 war kein Pärchen mehr zu erkennen und alle Würmer tot. Die Weibchen waren stark verwunden und gekrümmt und die Männchen hatten eine ventral gekrümmte Form. Es waren keine Eier zu erkennen. Siehe Figur 2.

Nach einer 48-stündigen Inkubation mit 100 μM der erfindungsgemäßen Verbindung 18 war kein Pärchen mehr zu erkennen und alle Würmer tot. Die Weibchen waren stark verwunden und gekrümmt und die Männchen waren ventral gekrümmt und teilweise ganz eingerollt. Es waren keine Eier zu erkennen. Siehe Figur 2.

Nach einer 48-stündigen Inkubation mit 20 μM der erfindungsgemäßen Verbindung 2 war in keiner der Gruppen ein Pärchen zu erkennen. Die Saugwürmer hatten sich alle abgelöst. Die Darmperistaltik war vermindert und eine Saugnapfbewegung war nicht mehr zu erkennen. Die Saugwürmer führten kaum noch Bewegungen aus. Alle Saugwürmer waren ventral eingerollt. Es wurden keine Eier gebildet.

Nach einer 48-stündigen Inkubation mit 100 μM der erfindungsgemäßen Verbindung 2 war in keiner der Gruppen ein Pärchen zu erkennen. Die Saugwürmer waren alle tot. Die Weibchen waren sehr gekrümmt und in sich verwunden, wohingegen die Männchen nach ventral eingerollt waren. Es wurden keine Eier gebildet.

**Beispiel 12:** Aktivität der Verbindungen gegen *Trypanosoma brucei*

Die Verbindungen 18 und 41 wurden an der London School of Hygiene and Tropical Medicine in vitro auf ihre Wirkung gegen den Parasiten *Trypanosoma brucei brucei* S427 getestet. Verbindung 18 zeigte einen $IC_{50}$ von 0.93 μM, Verbindung 41 zeigte einen $IC_{50}$ von 3.58 μM. Dieses Beispiel belegt, dass die erfindungsgemäßen Verbindungen ein potentiell breites Anwendungsspektrum haben.

## Patentansprüche

**1.** Verbindungen der allgemeinen Formel

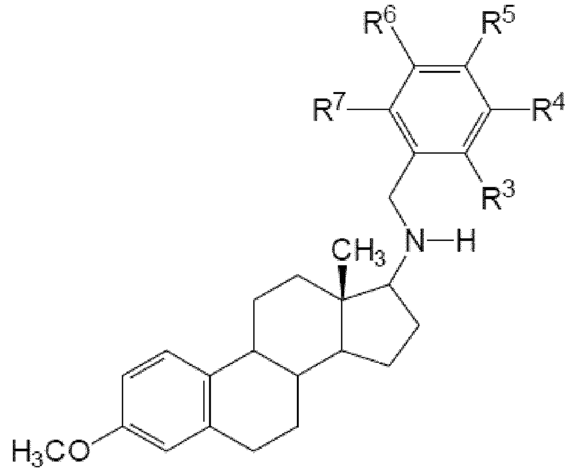

worin

$R^3$, $R^4$, $R^5$, $R^6$ und $R^7$ unabhängig voneinander folgende Reste bedeuten: -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, -O-CO-Ph, -O-CO-$CH_3$, -O-CO-$C_2H_5$, -O-CO-$C_3H_7$, -O-CO-cyclo-$C_3H_5$, -O-CO-$CH(CH_3)_2$, -O-CO-$C(CH_3)_3$, -O-CO-para-$C_6H_4$-$OR^9$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, -NHCO-$CH(CH_3)_2$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OCH(CH_3)_2$, -NHCO-$OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3$-cyclo-$C_3H_5$, $-SO_3CH(CH_3)_2$, $-SO_3C(CH_3)_3$, $-SO_2NH_2$, $-OCF_3$, $-OC_2F_5$, $-O-COOCH_3$, $-O-COOC_2H_5$, $-O-COOC_3H_7$, -O-COO-cyclo-$C_3H_5$, -O-$COOCH(CH_3)_2$, -O-$COOC(CH_3)_3$, -NH-CO-$NH_2$, -NH-CO-$NHCH_3$, -NH-CO-$NHC_2H_5$, -NH-CO-$NHC_3H_7$, -NH-CO-NH-cyclo-$C_3H_5$, -NH-CO-$NH[CH(CH_3)_2]$, -NH-CO-$NH[C(CH_3)_3]$, -NH-CO-$N(CH_3)_2$, -NH-CO-$N(C_2H_5)_2$, -NH-CO-$N(C_3H_7)_2$, -NH-CO-N(cyclo-$C_3H_5)_2$, -NH-CO-$N[CH(CH_3)_2]_2$, -NH-CO-$N[C(CH_3)_3]_2$, -NH-CS-$NH_2$, -NH-CS-$NHCH_3$, -NH-CS-$NHC_2H_5$, -NH-CS-$NHC_3H_7$, -NH-CS-NH-cyclo-$C_3H_5$, -NH-CS-$NH[CH(CH_3)_2]$, -NH-CS-$NH[C(CH_3)_3]$, -NH-CS-$N(CH_3)_2$, -NH-CS-$N(C_2H_5)_2$, -NH-CS-$N(C_3H_7)_2$, -NH-CS-N(cyclo-$C_3H_5)_2$, -NH-CS-$N[CH(CH_3)_2]_2$, -NH-CS-$N[C(CH_3)_3]_2$, -NH-C(=NH)-$NH_2$, -NH-C(=NH)-$NHCH_3$, -NH-C(=NH)-$NHC_2H_5$, -NH-C(=NH)-$NHC_3H_7$, -NH-C(=NH)-NH-cyclo-$C_3H_5$, -NH-C(=NH)-$NH[CH(CH_3)_2]$, -NH-C(=NH)-$NH[C(CH_3)_3]$, -NH-C(=NH)-$N(CH_3)_2$, -NH-C(=NH)-$N(C_2H_5)_2$, -NH-C(=NH)-$N(C_3H_7)_2$, -NH-C(=NH)-N(cyclo-$C_3H_5)_2$, -O-CO-$NH_2$, -NH-C(=NH)-$N[CH(CH_3)_2]_2$, -NH-C(=NH)-$N[C(CH_3)_3]_2$, -O-CO-$NHCH_3$, -O-CO-$NHC_2H_5$, -O-CO-$NHC_3H_7$, -O-CO-NH-cyclo-$C_3H_5$, -O-CO-$NH[CH(CH_3)_2]$, -O-CO-$NH[C(CH_3)_3]$, -O-CO-$N(CH_3)_2$, -O-CO-$N(C_2H_5)_2$, -O-CO-$N(C_3H_7)_2$, -O-CO-N(cyclo-$C_3H_5)_2$, -O-CO-$N[CH(CH_3)_2]_2$, -O-CO-$N[C(CH_3)_3]_2$, -O-CO-$OCH_3$, -O-CO-$OC_2H_5$, -O-CO-$OC_3H_7$, -O-CO-O-cyclo-$C_3H_5$, -O-CO-$OCH(CH_3)_2$, -O-CO-$OC(CH_3)_3$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_3H_5$, cyclo-$C_4H_7$, cyclo-$C_5H_9$, cyclo-$C_6H_{11}$, cyclo-$C_7H_{13}$, cyclo-$C_8H_{15}$, -Ph, $-CH_2$-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$,

$-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-CH(CH_3)-C_2H_4-CH=CH_2$, $-C_2H_4-CH=C(CH_3)_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-CH(CH_3)-CH_2-C(CH_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[C(CH_3)_3]=CH_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-CH=CH-C_2H_4-CH=CH_2$, $-CH_2-CH=CH-CH=CH-CH_3$, $-CH=CH-CH_2-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH=CH-CH=CH_2$, $-CH=CH-CH_2-C(CH_3)=CH_2$, $-CH=CH-CH(CH_3)-CH=CH_2$, $-CH=C(CH_3)-CH_2-CH=CH_2$, $-C(CH_3)=CH-CH_2-CH=CH_2$, $-CH=CH-CH=C(CH_3)_2$, $-CH=CH-C(CH_3)=CH-CH_3$, $-CH=C(CH_3)-CH=CH-CH_3$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-CH_2-CH(CH_3)-C\equiv CH$, $-CH(CH_3)-CH_2-C\equiv CH$, $-CH(CH_3)-C\equiv C-CH_3$, $-C_4H_8-C\equiv CH$, $-C_3H_6-C\equiv C-CH_3$, $-C_2H_4-C\equiv C-C_2H_5$, $-CH_2-C\equiv C-C_3H_7$, $-C\equiv C-C_4H_9$, $-C_2H_4-CH(CH_3)-C\equiv CH$, $-CH_2-CH(CH_3)-CH_2-C\equiv CH$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-C\equiv C-C(CH_3)_3$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-C=C-C=CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C\equiv C-CH(CH_3)-C\equiv CH$, $-CH(CH_3)-C\equiv C-C\equiv CH$, $-CH(C\equiv CH)-CH_2-C\equiv CH$, $-C(C\equiv CH)_2-CH_3$, $-CH_2-CH(C\equiv CH)_2$, $-CH(C\equiv CH)-C\equiv C-CH_3$;

wobei jeweils zwei benachbarte Substituenten $R^3$ und $R^4$ oder $R^4$ und $R^5$ oder $R^5$ und $R^6$ oder $R^6$ und $R^7$ über $-CH=CH-CH=CH-$ Gruppe miteinander verbunden sind, um einen kondensierten aromatischen Ring zu bilden;

sowie deren Metallkomplexe, Salze, Enantiomere, Enantiomerengemische, Diastereomere, Diastereomerengemische, Tautomere, Hydrate, Solvate, Racemateder vorgenannten Verbindungen.

**2.** Verbindungen gemäß Anspruch 1 , worin $R^3$ $-OH$, $-OCH_3$, $-OC_2H_5$, $-OC_3H_7$, $-O$-cyclo-$C_3H_5$, $-OCH(CH_3)_2$, $-OC(CH_3)_3$, $-OC_4H_9$, $-OPh$, $-OCH_2-Ph$, $-NH_2$, $-NHCH_3$, $-NHC_2H_5$, $-NHC_3H_7$, $-NH$-cyclo-$C_3H_5$, $-NHCH(CH_3)_2$, $-NHC(CH_3)_3$, $-N(CH_3)_2$, $-N(C_2H_5)_2$, $-N(C_3H_7)_2$, $-N$(cyclo-$C_3H_5)_2$, $-N[CH(CH_3)_2]_2$ oder $-N[C(CH_3)_3]_2$ bedeutet.

**3.** Verbindungen gemäß Anspruch 1:

17α-[(2-Hydroxynaphth-1-yl)-methylamino]-3-methoxy-estra-1,3,5(10)-trien,
17β-[(3-Hydroxynaphth-2-yl)-methylamino]-3-methoxy-estra-1,3,5(10)-trien,
17β-[(2-Hydroxynaphth-1-yl)-methylamino]-3-methoxy-estra-1,3,5(10)-trien Hydroperchlorat,
und
17α-[(3-Hydroxy-naphth-2-yl)-methylamino]-3-methoxy-estra-1,3,5(10)-trien.

**4.** Verbindungen gemäß eines der vorherigen Ansprüche zur Verwendung in der Medizin.

**5.** Verbindungen gemäß eines der vorherigen Ansprüche zur Verwendung für die Behandlung und/oder Prophylaxe parasitärer Infektionen und Erkrankungen.

**6.** Verbindungen zur Verwendung gemäß Anspruch 5, wobei die parasitäre Infektionen oder Erkrankung ausgewählt wird aus der Gruppe bestehend aus: Trypanosomiasis, Chagas-Krankheiten, Amoebiasis, Ancylostomiasis, Babesiose, Balantidiasis, Blastocystis Infektion, Schistosomiasis, Bilharziose, Kokzidiosen, Kryptosporidiose, Dientamoebiasis, Mikrosporidiose, Zoonosen, Giardiasis, Isosporiasis, Leishmaniose, *Naegleria* Infektion, Malaria, Sarcosporidiose, Piroplasmose, Trichinose, Toxoplasmose, Trichomoniasis, Ascariasis, Filariose, Taeniasis, Echinokokkose, Onchozerkose, Capillariasis, Elephantiasis, Enterobiasis, Strongyloidiasis, Trichuriasis und Zystizerkose.

**7.** Pharmazeutische Zusammensetzung enthaltend mindestens eine Verbindung wie in Anspruch 1 definiert und mindestens einen pharmakologisch verträglichen Träger, Hilfsstoff und/oder Lösungsmittel.

**8.** Pharmazeutische Zusammensetzung gemäß Anspruch 7 in Form von Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen.

**9.** Pharmazeutische Zusammensetzung gemäß Anspruch 7 oder 8 geeignet zur Inhalation oder zur intravenösen, intraperitonealen, intramuskulären, subkutanen, mucokutanen, oralen, rektalen, transdermalen, topikalen, bukkal, intradermalen, intragastralen, intrakutanen, intranasalen, intrabuccalen, perkutanen oder sublingualen Applikation.

**10.** Pharmazeutische Zusammensetzung gemäß Anspruch 7, 8 oder 9 des weiteren enthaltend einen antiparasitären, antibakteriellen, antimykotischen und/oder antiviralen Wirkstoff.

**11.** Pharmazeutische Zusammensetzung gemäß Anspruch 10, wobei der antiparasitäre Wirkstoff Artemisinin oder Artesunat ist.

## Claims

**1.** Compounds of general formula

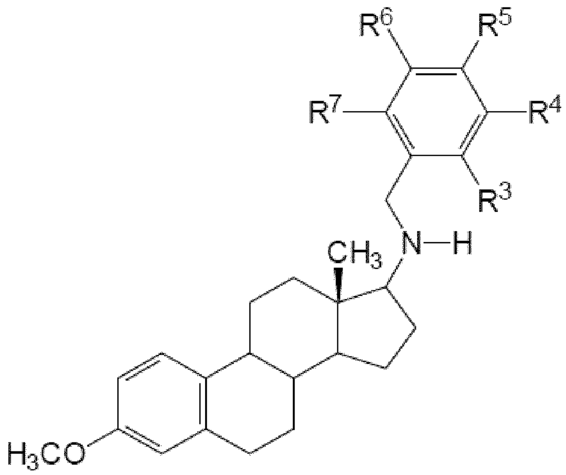

wherein
$R^3$, $R^4$, $R^5$, $R^6$ and $R^7$ independently of each other represent one of the following residues:
-H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, -O-CO-Ph, -O-CO-$CH_3$, -O-CO-$C_2H_5$, -O-CO-$C_3H_7$, -O-CO-cyclo-$C_3H_5$, -O-CO-$CH(CH_3)_2$, -O-CO-$C(CH_3)_3$, -O-CO-para-$C_6H_4$-$OR^9$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, -NHCO-$CH(CH_3)_2$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OCH(CH_3)_2$, -NHCO-$OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3$-cyclo-$C_3H_5$, $-SO_3CH(CH_3)_2$, $-SO_3C(CH_3)_3$, $-SO_2NH_2$, $-OCF_3$, $-OC_2F_5$, -O-$COOCH_3$, -O-$COOC_2H_5$, -O-$COOC_3H_7$, -O-COO-cyclo-$C_3H_5$, -O-$COOCH(CH_3)_2$, -O-$COOC(CH_3)_3$, -NH-CO-$NH_2$, -NH-CO-$NHCH_3$, -NH-CO-$NHC_2H_5$, -NH-CO-$NHC_3H_7$, -NH-CO-NH-cyclo-$C_3H_5$, -NH-CO-$NH[CH(CH_3)_2]$, -NH-CO-$NH[C(CH_3)_3]$, -NH-CO-$N(CH_3)_2$, -NH-CO-$N(C_2H_5)_2$, -NH-CO-$N(C_3H_7)_2$, -NH-CO-N(cyclo-$C_3H_5)_2$, -NH-CO-$N[CH(CH_3)_2]_2$, -NH-CO-$N[C(CH_3)_3]_2$, -NH-CS-$NH_2$, -NH-CS-$NHCH_3$, -NH-CS-$NHC_2H_5$, -NH-CS-$NHC_3H_7$, -NH-CS-NH-cyclo-$C_3H_5$, -NH-CS-$NH[CH(CH_3)_2]$, -NH-CS-$NH[C(CH_3)_3]$, -NH-CS-$N(CH_3)_2$, -NH-CS-$N(C_2H_5)_2$, -NH-CS-$N(C_3H_7)_2$, -NH-CS-N(cyclo-$C_3H_5)_2$, -NH-CS-$N[CH(CH_3)_2]_2$, -NH-CS-$N[C(CH_3)_3]_2$, -NH-

C(=NH)-NH$_2$, -NH-C(=NH)-NHCH$_3$, -NH-C(=NH)-NHC$_2$H$_5$, -NH-C(=NH)-NHC$_3$H$_7$, -NH-C(=NH)-NH-cyclo-C$_3$H$_5$, -NH-C(=NH)-NH[CH(CH$_3$)$_2$], -NH-C(=NH)-NH[C(CH$_3$)$_3$], -NH-C(=NH)-N(CH$_3$)$_2$, -NH-C(=NH)-N(C$_2$H$_5$)$_2$, -NH-C(=NH)-N(C$_3$H$_7$)$_2$, -NH-C(=NH)-N(cyclo-C$_3$H$_5$)$_2$, -O-CO-NH$_2$, -NH-C(=NH)-N[CH(CH$_3$)$_2$]$_2$, -NH-C(=NH)-N[C(CH$_3$)$_3$]$_2$, -O-CO-NHCH$_3$, -O-CO-NHC$_2$H$_5$, -O-CO-NHC$_3$H$_7$, -O-CO-NH-cyclo-C$_3$H$_5$, -O-CO-NH[CH(CH$_3$)$_2$], -O-CO-NH[C(CH$_3$)$_3$], -O-CO-N(CH$_3$)$_2$, -O-CO-N(C$_2$H$_5$)$_2$, -O-CO-N(C$_3$H$_7$)$_2$, -O-CO-N(cyclo-C$_3$H$_5$)$_2$, -O-CO-N[CH(CH$_3$)$_2$]$_2$, -O-CO-N[C(CH$_3$)$_3$]$_2$, -O-CO-OCH$_3$, -O-CO-OC$_2$H$_5$, -O-CO-OC$_3$H$_7$, -O-CO-O-cyclo-C$_3$H$_5$, -O-CO-OCH(CH$_3$)$_2$, -O-CO-OC(CH$_3$)$_3$, -CH$_2$F, -CHF$_2$, -CF$_3$, -CH$_2$Cl, -CH$_2$Br, -CH$_2$I, -CH$_2$-CH$_2$F, -CH$_2$-CHF$_2$, -CH$_2$-CF$_3$, -CH$_2$-CH$_2$Cl, -CH$_2$-CH$_2$Br, -CH$_2$-CH$_2$I, cyclo-C$_3$H$_5$, cyclo-C$_4$H$_7$, cyclo-C$_5$H$_9$, cyclo-C$_6$H$_{11}$, cyclo-C$_7$H$_{13}$, cyclo-C$_8$H$_{15}$, -Ph, -CH$_2$-Ph, -CPh$_3$, -CH$_3$, -C$_2$H$_5$, -C$_3$H$_7$, -CH(CH$_3$)$_2$, -C$_4$H$_9$, -CH$_2$-CH(CH$_3$)$_2$, -CH(CH$_3$)-C$_2$H$_5$, -C(CH$_3$)$_3$, -C$_5$H$_{11}$, -CH(CH$_3$)-C$_3$H$_7$, -CH$_2$-CH(CH$_3$)-C$_2$H$_5$, -CH(CH$_3$)-CH(CH$_3$)$_2$, -C(CH$_3$)$_2$-C$_2$H$_5$, -CH$_2$-C(CH$_3$)$_3$, -CH(C$_2$H$_5$)$_2$, -C$_2$H$_4$-CH(CH$_3$)$_2$, -C$_6$H$_{13}$, -C$_7$H$_{15}$, -C$_8$H$_{17}$, -C$_3$H$_6$-CH(CH$_3$)$_2$, -C$_2$H$_4$-CH(CH$_3$)-C$_2$H$_5$, -CH(CH$_3$)-C$_4$H$_9$, -CH$_2$-CH(CH$_3$)-C$_3$H$_7$, -CH(CH$_3$)-CH$_2$-CH(CH$_3$)$_2$, -CH(CH$_3$)-CH(CH$_3$)-C$_2$H$_5$, -CH$_2$-CH(CH$_3$)-CH(CH$_3$)$_2$, -CH$_2$-C(CH$_3$)$_2$-C$_2$H$_5$, -C(CH$_3$)$_2$-C$_3$H$_7$, -C(CH$_3$)$_2$-CH(CH$_3$)$_2$, -C$_2$H$_4$-C(CH$_3$)$_3$, -CH(CH$_3$)-C(CH$_3$)$_3$, -CH=CH$_2$, -CH$_2$-CH=CH$_2$, -C(CH$_3$)=CH$_2$, -CH=CH-CH$_3$, -C$_2$H$_4$-CH=CH$_2$, -CH$_2$-CH=CH-CH$_3$, -CH=CH-C$_2$H$_5$, -CH$_2$-C(CH$_3$)=CH$_2$, -CH(CH$_3$)-CH=CH, -CH=C(CH$_3$)$_2$, -C(CH$_3$)=CH-CH$_3$, -CH=CH-CH=CH$_2$, -C$_3$H$_6$-CH=CH$_2$, -C$_2$H$_4$-CH=CH-CH$_3$, -CH$_2$-CH=CH-C$_2$H$_5$, -CH=CH-C$_3$H$_7$, -CH$_2$-CH=CH-CH=CH$_2$, -CH=CH-CH=CH-CH$_3$, -CH=CH-CH$_2$-CH=CH$_2$, -C(CH$_3$)=CH-CH=CH$_2$, -CH=C(CH$_3$)-CH=CH$_2$, -CH=CH-C(CH$_3$)=CH$_2$, -C$_2$H$_4$-C(CH$_3$)=CH$_2$, -CH$_2$-CH(CH$_3$)-CH=CH$_2$, -CH(CH$_3$)-CH$_2$-CH=CH$_2$, -CH$_2$-CH=C(CH$_3$)$_2$, -CH$_2$-C(CH$_3$)=CH-CH$_3$, -CH(CH$_3$)-CH=CH-CH$_3$, -CH=CH-CH(CH$_3$)$_2$, -CH=C(CH$_3$)-C$_2$H$_5$, -C(CH$_3$)=CH-C$_2$H$_5$, -C(CH$_3$)=C(CH$_3$)$_2$, -C(CH$_3$)$_2$-CH=CH$_2$, -CH(CH$_3$)-C(CH$_3$)=CH$_2$, -C(CH$_3$)=CH-CH=CH$_2$, -CH=C(CH$_3$)-CH=CH$_2$, -CH=CH-C(CH$_3$)=CH$_2$, -C$_4$H$_8$-CH=CH$_2$, -C$_3$H$_6$-CH=CH-CH$_3$, -C$_2$H$_4$-CH=CH-C$_2$H$_5$, -CH$_2$-CH=CH-C$_3$H$_7$, -CH=CH-C$_4$H$_9$, -C$_3$H$_6$-C(CH$_3$)=CH$_2$, -C$_2$H$_4$-CH(CH$_3$)-CH=CH$_2$, -CH$_2$-CH(CH$_3$)-CH$_2$-CH=CH$_2$, -CH(CH$_3$)-C$_2$H$_4$-CH=CH$_2$, -C$_2$H$_4$-CH=C(CH$_3$)$_2$, -C$_2$H$_4$-C(CH$_3$)=CH-CH$_3$, -CH$_2$-CH(CH$_3$)-CH=CH-CH$_3$, -CH(CH$_3$)-CH$_2$-CH=CH-CH$_3$, -CH$_2$-CH=CH-CH(CH$_3$)$_2$, -CH$_2$-CH=C(CH$_3$)-C$_2$H$_5$, -CH$_2$-C(CH$_3$)=CH-C$_2$H$_5$, -CH(CH$_3$)-CH=CH-C$_2$H$_5$, -CH=CH-CH$_2$-CH(CH$_3$)$_2$, -CH=CH-CH(CH$_3$)-C$_2$H$_5$, -CH=C(CH$_3$)-C$_3$H$_7$, -C(CH$_3$)=CH-C$_3$H$_7$, -CH$_2$-CH(CH$_3$)-C(CH$_3$)=CH$_2$, -CH(CH$_3$)-CH$_2$-C(CH$_3$)=CH$_2$, -CH(CH$_3$)-CH(CH$_3$)-CH=CH$_2$, -CH$_2$-C(CH$_3$)$_2$-CH=CH$_2$, -C(CH$_3$)$_2$-CH$_2$-CH=CH$_2$, -CH$_2$-C(CH$_3$)=C(CH$_3$)$_2$, -CH(CH$_3$)-CH=C(CH$_3$)$_2$, -C(CH$_3$)$_2$-CH=CH-CH$_3$, -CH(CH$_3$)-C(CH$_3$)=CH-CH$_3$, -CH=C(CH$_3$)-CH(CH$_3$)$_2$, -C(CH$_3$)=CH-CH(CH$_3$)$_2$, -C(CH$_3$)=C(CH$_3$)-C$_2$H$_5$, -CH=CH-C(CH$_3$)$_3$, -C(CH$_3$)$_2$-C(CH$_3$)=CH$_2$, -CH(C$_2$H$_5$)-C(CH$_3$)=CH$_2$, -C(CH$_3$)(C$_2$H$_5$)-CH=CH$_2$, -CH(CH$_3$)-C(C$_2$H$_5$)=CH$_2$, -CH$_2$-C(C$_3$H$_7$)=CH$_2$, -CH$_2$-C(C$_2$H$_5$)=CH-CH$_3$, -CH(C$_2$H$_5$)-CH=CH-CH$_3$, -C(C$_4$H$_9$)=CH$_2$, -C(C$_3$H$_7$)=CH-CH$_3$, -C(C$_2$H$_5$)=CH-C$_2$H$_5$, -C(C$_2$H$_5$)=C(CH$_3$)$_2$, -C[C(CH$_3$)$_3$]=CH$_2$, -C[CH(CH$_3$)(C$_2$H$_5$)]=CH$_2$, -C[CH$_2$-CH(CH$_3$)$_2$]=CH$_2$, -C$_2$H$_4$-CH=CH-CH=CH$_2$, -CH$_2$-CH=CH-CH$_2$-CH=CH$_2$, -CH=CH-C$_2$H$_4$-CH=CH$_2$, -CH$_2$-CH=CH-CH=CH-CH$_3$, -CH=CH-CH$_2$-CH=CH-CH$_3$, -CH=CH-CH=CH-C$_2$H$_5$, -CH$_2$-CH=CH-C(CH$_3$)=CH$_2$, -CH$_2$-CH=C(CH$_3$)-CH=CH$_2$, -CH$_2$-C(CH$_3$)=CH-CH=CH$_2$, -CH(CH$_3$)-CH=CH-CH=CH$_2$, -CH=CH-CH$_2$-C(CH$_3$)=CH$_2$, -CH=CH-CH(CH$_3$)-CH=CH$_2$, -CH=C(CH$_3$)-CH$_2$-CH=CH$_2$, -C(CH$_3$)=CH-CH$_2$-CH=CH$_2$, -CH=CH-CH=C(CH$_3$)$_2$, -CH=CH-C(CH$_3$)=CH-CH$_3$, -CH=C(CH$_3$)-CH=CH-CH$_3$, -C(CH$_3$)=CH-CH=CH-CH$_3$, -CH=C(CH$_3$)-C(CH$_3$)=CH$_2$, -C(CH$_3$)=CH-C(CH$_3$)=CH$_2$, -C(CH$_3$)=C(CH$_3$)-CH=CH$_2$, -CH=CH-CH=CH-CH=CH$_2$, -C≡CH, -C≡C-CH$_3$, -CH$_2$-C≡CH, -C$_2$H$_4$-C≡CH, -CH$_2$-C≡C-CH$_3$, -C≡C-C$_2$H$_5$, -C$_3$H$_6$-C≡CH, -C$_2$H$_4$-C≡C-CH$_3$, -CH$_2$-C≡C-C$_2$H$_5$, -C≡C-C$_3$H$_7$, -CH(CH$_3$)-C≡CH, -CH$_2$-CH(CH$_3$)-C≡CH, -CH(CH$_3$)-CH$_2$-C≡CH, -CH(CH$_3$)-C≡C-CH$_3$, -C$_4$H$_8$-C≡CH, -C$_3$H$_6$-C≡C-CH$_3$, -C$_2$H$_4$-C≡C-C$_2$H$_5$, -CH$_2$-C≡C-C$_3$H$_7$, -C≡C-C$_4$H$_9$, -C$_2$H$_4$-CH(CH$_3$)-C≡CH, -CH$_2$-CH(CH$_3$)-CH$_2$-C≡CH, -CH(CH$_3$)-C$_2$H$_4$-C≡CH, -CH$_2$-CH(CH$_3$)-C≡C-CH$_3$, -CH(CH$_3$)-CH$_2$-C≡C-CH$_3$, -CH(CH$_3$)-C≡C-C$_2$H$_5$, -CH$_2$-C≡C-CH(CH$_3$)$_2$, -C≡C-CH(CH$_3$)-C$_2$H$_5$, -C≡C-CH$_2$-CH(CH$_3$)$_2$, -C≡C-C(CH$_3$)$_3$, -CH(C$_2$H$_5$)-C≡C-CH$_3$, -C(CH$_3$)$_2$-C≡C-CH$_3$, -CH(C$_2$H$_5$)-CH$_2$-C≡CH, -CH$_2$-CH(C$_2$H$_5$)-C≡CH, -C(CH$_3$)$_2$-CH$_2$-C≡CH, -CH$_2$-C(CH$_3$)$_2$-C≡CH, -CH(CH$_3$)-CH(CH$_3$)-C≡CH, -CH(C$_3$H$_7$)-C≡CH, -C(CH$_3$)(C$_2$H$_5$)-C≡CH, -C=C-C=CH, -CH$_2$-C≡C-C≡CH, -C≡C-C≡C-CH$_3$, -CH(C≡CH)$_2$, -C$_2$H$_4$-C≡C-C≡CH, -CH$_2$-C≡C-CH$_2$-C≡CH, -C≡C-C$_2$H$_4$-C≡CH, -CH$_2$-C≡C-C≡C-CH$_3$, -C≡C-CH$_2$-C≡C-CH$_3$, -C≡C-C≡C-C$_2$H$_5$, -C≡C-CH(CH$_3$)-C≡CH, -CH(CH$_3$)-C≡C-C≡CH, -CH(C≡CH)-CH$_2$-C≡CH, -C(C≡CH)$_2$-CH$_3$, -CH$_2$-CH(C≡CH)$_2$, -CH(C≡CH)-C≡C-CH$_3$;

wherein two adjacent substituents $R^3$ and $R^4$ or $R^4$ and $R^5$ or $R^5$ and $R^6$ or $R^6$ and $R^7$ are connected via a -CH=CH-CH=CH-group respectively to form a condensed aromatic ring;

as well as their metal complexes, salts, enantiomers, enantiomer mixtures, diastereomers, diastereomer mixtures, tautomers, hydrates, solvates, racemates of the aforementioned compounds.

**2.** Compounds according to claim 1, wherein $R^3$ represents -OH, -OCH$_3$, -OC$_2$H$_5$, -OC$_3$H$_7$, -O-cyclo-C$_3$H$_5$, -OCH(CH$_3$)$_2$, -OC(CH$_3$)$_3$, -OC$_4$H$_9$, -OPh, -OCH$_2$-Ph, -NH$_2$, -NHCH$_3$, -NHC$_2$H$_5$, -NHC$_3$H$_7$, -NH-cyclo-C$_3$H$_5$, -NHCH(CH$_3$)$_2$, -NHC(CH$_3$)$_3$, -N(CH$_3$)$_2$, -N(C$_2$H$_5$)$_2$, -N(C$_3$H$_7$)$_2$, -N(cyclo-C$_3$H$_5$)$_2$, -N[CH(CH$_3$)$_2$]$_2$ or -N[C(CH$_3$)$_3$]$_2$.

**3.** Compounds according to claim 1:

17α-[(2-hydroxynaphth-1-yl)-methylamino]-3-methoxy-estra-1,3,5(10)-triene,
17β-[(3-hydroxynaphth-2-yl)-methylamino]-3-methoxy-estra-1,3,5(10)-triene,
17β-[(2-hydroxynaphth-1-yl)-methylamino]-3-methoxy-estra-1,3,5(10)-triene hydroperchlorate,
and
17α-[(3-hydroxy-naphth-2-yl)-methylamino]-3-methoxy-estra-1,3,5(10)-triene.

**4.** Compounds according to any one of the previous claims for use in medicine.

**5.** Compounds according to any one of the previous claims for use in the treatment and/or prophylaxis of parasitic infections and diseases.

**6.** Use according to claim 5, wherein the parasitic infections or diseases are selected from the group consisting of: trypanosomiasis, Chagas diseases, echinococcosis, amoebiasis, ancylostomiasis, babesiosis, balantidiasis, blastocystis infection, schistosomiasis, bilharzia, coccidioses, cryptosporidiosis, dientamoebiasis, microsporidiosis, zoonoses, giardiasis, isosporiasis, leishmaniasis, *Naegleria* infection, malaria, sarcosporidiosis, piroplasmosis, trichinosis, toxoplasmosis, trichomoniasis, ascariasis, filariasis, taeniasis, onchocerciasis, capillariasis, elephantiasis, enterobiasis, strongyloidiasis, trichuriasis and cysticercosis.

**7.** Pharmaceutical composition containing at least one compound as defined in claim 1 and at least one pharmacologically acceptable carrier, excipient and/or solvent.

**8.** Pharmaceutical composition according to claim 7 in form of droplets, oral sprays, nasal sprays, pills, tablets, film-coated tablets, layered tablets, uvulas, gels, ointments, syrups, inhalation powders, granulates, suppositories, emulsions, dispersions, microcapsules, capsules, powders or injection solutions.

**9.** Pharmaceutical composition according to claim 7 or 8 suitable for inhalation or for intravenous, intraperitoneal, intramuscular, subcutaneous, mucocutaneous, oral, rectal, transdermal, topical, buccal, intradermal, intragastral, intracutaneous, intranasal, intrabuccal, percutaneous or sublingual administration.

**10.** Pharmaceutical composition according to claim 7, 8 or 9, furthermore containing an antiparasitic, antibacterial, antimycotic and/or antiviral active agent.

**11.** Pharmaceutical composition according to claim 10, wherein the antiparasitic active agent is artemisinin or artesunate.

## Revendications

**1.** Composés de formule générale

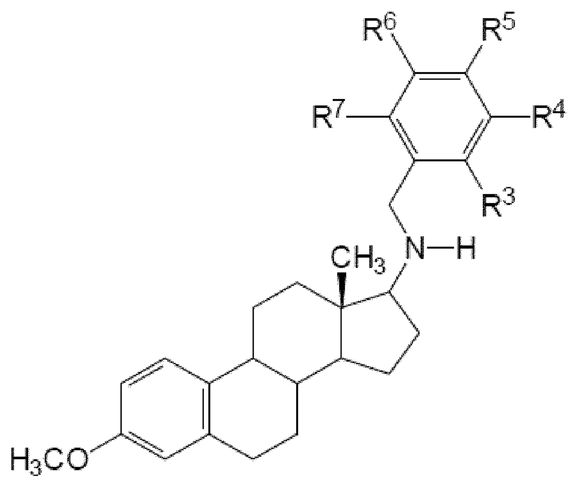

où
$R^3$, $R^4$, $R^5$, $R^6$ et $R^7$ représentent, de façon mutuellement indépendante, l'un des résidus suivants :

-H, -OH, $-OCH_3$, $-OC_2H_5$, $-OC_3H_7$, $-O\text{-cyclo-}C_3H_5$, $-OCH(CH_3)_2$, $-OC(CH_3)_3$, $-OC_4H_9$, -OPh, $-OCH_2\text{-Ph}$, $-OCPh_3$, -SH, $-SCH_3$, $-SC_2H_5$, $-SC_3H_7$, $-S\text{-cyclo-}C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, $-CO\text{-cyclo-}C_3H_5$, $-COCH(CH_3)_2$, $-COC(CH_3)_3$, -COOH, -COCN, $-COOCH_3$, $-COOC_2H_5$, $-COOC_3H_7$, $-COO\text{-cyclo-}C_3H_5$, $-COOCH(CH_3)_2$, $-COOC(CH_3)_3$, -O-CO-Ph, $-O\text{-CO-}CH_3$, $-O\text{-CO-}C_2H_5$, $-O\text{-CO-}C_3H_7$, $-O\text{-CO-cyclo-}C_3H_5$, $-O\text{-CO-}CH(CH_3)_2$, $-O\text{-CO-}C(CH_3)_3$, $-O\text{-CO-para-}C_6H_4\text{-}OR^9$, $-CONH_2$, $-CONHCH_3$, $-CONHC_2H_5$, $-CONHC_3H_7$, $-CONH\text{-cyclo-}C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, $-CON(\text{cyclo-}C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, $-NHCO\text{-cyclo-}C_3H_5$, $-NHCO\text{-}CH(CH_3)_2$, $-NHCO\text{-}C(CH_3)_3$, $-NHCO\text{-}OCH_3$, $-NHCO\text{-}OC_2H_5$, $-NHCO\text{-}OC_3H_7$, $-NHCO\text{-O-cyclo-}C_3H_5$, $-NHCO\text{-}OCH(CH_3)_2$, $-NHCO\text{-}OC(CH_3)_3$, $-NH_2$, $-NHCH_3$, $-NHC_2H_5$, $-NHC_3H_7$, $-NH\text{-cyclo-}C_3H_5$, $-NHCH(CH_3)_2$, $-NHC(CH_3)_3$, $-N(CH_3)_2$, $-N(C_2H_5)_2$, $-N(C_3H_7)_2$, $-N(\text{cyclo-}C_3H_5)_2$, $-N[CH(CH_3)_2]_2$, $-N[C(CH_3)_3]_2$, $-SOCH_3$, $-SOC_2H_5$, $-SOC_3H_7$, $-SO\text{-cyclo-}C_3H_5$, $-SOCH(CH_3)_2$, $-SOC(CH_3)_3$, $-SO_2CH_3$, $-SO_2C_2H_5$, $-SO_2C_3H_7$, $-SO_2\text{-cyclo-}C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3\text{-cyclo-}C_3H_5$, $-SO_3CH(CH_3)_2$, $-SO_3C(CH_3)_3$, $-SO_2NH_2$, $-OCF_3$, $-OC_2F_5$, $-O\text{-}COOCH_3$, $-O\text{-}COOC_2H_5$, $-O\text{-}COOC_3H_7$, $-O\text{-COO-cyclo-}C_3H_5$, $-O\text{-}COOCH(CH_3)_2$, $-O\text{-}COOC(CH_3)_3$, $-NH\text{-CO-}NH_2$, $-NH\text{-CO-}NHCH_3$, $-NH\text{-CO-}NHC_2H_5$, $-NH\text{-CO-}NHC_3H_7$, $-NH\text{-CO-NH-cyclo-}C_3H_5$, $-NH\text{-CO-}NH[CH(CH_3)_2]$, $-NH\text{-CO-}NH[C(CH_3)_3]$, $-NH\text{-CO-}N(CH_3)_2$, $-NH\text{-CO-}N(C_2H_5)_2$, $-NH\text{-CO-}N(C_3H_7)_2$, $-NH\text{-CO-}N(\text{cyclo-}C_3H_5)_2$, $-NH\text{-CO-}N[CH(CH_3)_2]_2$, $-NH\text{-CO-}N[C(CH_3)_3]_2$, $-NH\text{-CS-}NH_2$, $-NH\text{-CS-}NHCH_3$, $-NH\text{-CS-}NHC_2H_5$, $-NH\text{-CS-}NHC_3H_7$, $-NH\text{-CS-NH-cyclo-}C_3H_5$, $-NH\text{-CS-}NH[CH(CH_3)_2]$, $-NH\text{-CS-}NH[C(CH_3)_3]$, $-NH\text{-CS-}N(CH_3)_2$, $-NH\text{-CS-}N(C_2H_5)_2$, $-NH\text{-CS-}N(C_3H_7)_2$, $-NH\text{-CS-}N(\text{cyclo-}C_3H_5)_2$, $-NH\text{-CS-}N[CH(CH_3)_2]_2$, $-NH\text{-CS-}N[C(CH_3)_3]_2$, $-NH\text{-}C(=NH)\text{-}NH_2$, $-NH\text{-}C(=NH)\text{-}NHCH_3$, $-NH\text{-}C(=NH)\text{-}NHC_2H_5$, $-NH\text{-}C(=NH)\text{-}NHC_3H_7$, $-NH\text{-}C(=NH)\text{-NH-cyclo-}C_3H_5$, $-NH\text{-}C(=NH)\text{-}NH[CH(CH_3)_2]$, $-NH\text{-}C(=NH)\text{-}NH[C(CH_3)_3]$, $-NH\text{-}C(=NH)\text{-}N(CH_3)_2$, $-NH\text{-}C(=NH)\text{-}N(C_2H_5)_2$, $-NH\text{-}C(=NH)\text{-}N(C_3H_7)_2$, $-NH\text{-}C(=NH)\text{-}N(\text{cyclo-}C_3H_5)_2$, $-O\text{-CO-}NH_2$, $-NH\text{-}C(=NH)\text{-}N[CH(CH_3)_2]_2$, $-NH\text{-}C(=NH)\text{-}N[C(CH_3)_3]_2$, $-O\text{-CO-}NHCH_3$, $-O\text{-CO-}NHC_2H_5$, $-O\text{-CO-}NHC_3H_7$, $-O\text{-CO-NH-cyclo-}C_3H_5$, $-O\text{-CO-}NH[CH(CH_3)_2]$, $-O\text{-CO-}NH[C(CH_3)_3]$, $-O\text{-CO-}N(CH_3)_2$, $-O\text{-CO-}N(C_2H_5)_2$, $-O\text{-CO-}N(C_3H_7)_2$, $-O\text{-CO-}N(\text{cyclo-}C_3H_5)_2$, $-O\text{-CO-}N[CH(CH_3)_2]_2$, $-O\text{-CO-}N[C(CH_3)_3]_2$, $-O\text{-CO-}OCH_3$, $-O\text{-CO-}OC_2H_5$, $-O\text{-CO-}OC_3H_7$, $-O\text{-CO-O-cyclo-}C_3H_5$, $-O\text{-CO-}OCH(CH_3)_2$, $-O\text{-CO-}OC(CH_3)_3$, $-CH_2F$, $-CHF_2$, $-CF_3$, $-CH_2Cl$, $-CH_2Br$, $-CH_2I$, $-CH_2\text{-}CH_2F$, $-CH_2\text{-}CHF_2$, $-CH_2\text{-}CF_3$, $-CH_2\text{-}CH_2Cl$, $-CH_2\text{-}CH_2Br$, $-CH_2\text{-}CH_2I$, $\text{cyclo-}C_3H_5$, $\text{cyclo-}C_4H_7$, $\text{cyclo-}C_5H_9$, $\text{cyclo-}C_6H_{11}$, $\text{cyclo-}C_7H_{13}$, $\text{cyclo-}C_8H_{15}$, -Ph, $-CH_2\text{-Ph}$, $-CPh_3$, $-CH_3$, $-C_2H_5$, $-C_3H_7$, $-CH(CH_3)_2$, $-C_4H_9$, $-CH_2\text{-}CH(CH_3)_2$, $-CH(CH_3)\text{-}C_2H_5$, $-C(CH_3)_3$, $-C_5H_{11}$, $-CH(CH_3)\text{-}C_3H_7$, $-CH_2\text{-}CH(CH_3)\text{-}C_2H_5$, $-CH(CH_3)\text{-}CH(CH_3)_2$, $-C(CH_3)_2\text{-}C_2H_5$, $-CH_2\text{-}C(CH_3)_3$, $-CH(C_2H_5)_2$, $-C_2H_4\text{-}CH(CH_3)_2$, $-C_6H_{13}$, $-C_7H_{15}$, $-C_8H_{17}$, $-C_3H_6\text{-}CH(CH_3)_2$, $-C_2H_4\text{-}CH(CH_3)\text{-}C_2H_5$, $-CH(CH_3)\text{-}C_4H_9$, $-CH_2\text{-}CH(CH_3)\text{-}C_3H_7$, $-CH(CH_3)\text{-}CH_2\text{-}CH(CH_3)_2$, $-CH(CH_3)\text{-}CH(CH_3)\text{-}C_2H_5$, $-CH_2\text{-}CH(CH_3)\text{-}CH(CH_3)_2$, $-CH_2\text{-}C(CH_3)_2\text{-}C_2H_5$, $-C(CH_3)_2\text{-}C_3H_7$, $-C(CH_3)_2\text{-}CH(CH_3)_2$, $-C_2H_4\text{-}C(CH_3)_3$, $-CH(CH_3)\text{-}C(CH_3)_3$, $-CH=CH_2$, $-CH_2\text{-}CH=CH_2$, $-C(CH_3)=CH_2$, $-CH=CH\text{-}CH_3$, $-C_2H_4\text{-}CH=CH_2$, $-CH_2\text{-}CH=CH\text{-}CH_3$, $-CH=CH\text{-}C_2H_5$, $-CH_2\text{-}C(CH_3)=CH_2$, $-CH(CH_3)\text{-}CH=CH$, $-CH=C(CH_3)_2$, $-C(CH_3)=CH\text{-}CH_3$, $-CH=CH\text{-}CH=CH_2$, $-C_3H_6\text{-}CH=CH_2$, $-C_2H_4\text{-}CH=CH\text{-}CH_3$, $-CH_2\text{-}CH=CH\text{-}C_2H_5$, $-CH=CH\text{-}C_3H_7$, $-CH_2\text{-}CH=CH\text{-}CH=CH_2$, $-CH=CH\text{-}CH=CH\text{-}CH_3$, $-CH=CH\text{-}CH_2\text{-}CH=CH_2$, $-C(CH_3)=CH\text{-}CH=CH_2$, $-CH=C(CH_3)\text{-}CH=CH_2$, $-CH=CH\text{-}C(CH_3)=CH_2$, $-C_2H_4\text{-}C(CH_3)=CH_2$, $-CH_2\text{-}CH(CH_3)\text{-}CH=CH_2$, $-CH(CH_3)\text{-}CH_2\text{-}CH=CH_2$, $-CH_2\text{-}CH=C(CH_3)_2$, $-CH_2\text{-}C(CH_3)=CH\text{-}CH_3$, $-CH(CH_3)\text{-}CH=CH\text{-}CH_3$, $-CH=CH\text{-}CH(CH_3)_2$, $-CH=C(CH_3)\text{-}C_2H_5$, $-C(CH_3)=CH\text{-}C_2H_5$, $-C(CH_3)=C(CH_3)_2$, $-C(CH_3)_2\text{-}CH=CH_2$, $-CH(CH_3)\text{-}C(CH_3)=CH_2$, $-C(CH_3)=CH\text{-}CH=CH_2$, $-CH=C(CH_3)\text{-}CH=CH_2$, $-CH=CH\text{-}C(CH_3)=CH_2$, $-C_4H_8\text{-}CH=CH_2$, $-C_3H_6\text{-}CH=CH\text{-}CH_3$, $-C_2H_4\text{-}CH=CH\text{-}C_2H_5$, $-CH_2\text{-}CH=CH\text{-}C_3H_7$, $-CH=CH\text{-}C_4H_9$, $-C_3H_6\text{-}C(CH_3)=CH_2$, $-C_2H_4\text{-}CH(CH_3)\text{-}CH=CH_2$, $-CH_2\text{-}CH(CH_3)\text{-}CH_2\text{-}CH=CH_2$, $-CH(CH_3)\text{-}C_2H_4\text{-}CH=CH_2$, $-C_2H_4\text{-}CH=C(CH_3)_2$, $-C_2H_4\text{-}C(CH_3)=CH\text{-}CH_3$, $-CH_2\text{-}CH(CH_3)\text{-}CH=CH\text{-}CH_3$, $-CH(CH_3)\text{-}CH_2\text{-}CH=CH\text{-}CH_3$, $-CH_2\text{-}CH=CH\text{-}CH(CH_3)_2$, $-CH_2\text{-}CH=C(CH_3)\text{-}C_2H_5$, $-CH_2\text{-}C(CH_3)=CH\text{-}C_2H_5$, $-CH(CH_3)\text{-}CH=CH\text{-}C_2H_5$, $-CH=CH\text{-}CH_2\text{-}CH(CH_3)_2$, $-CH=CH\text{-}CH(CH_3)\text{-}C_2H_5$, $-CH=C(CH_3)\text{-}C_3H_7$, $-C(CH_3)=CH\text{-}C_3H_7$, $-CH_2\text{-}CH(CH_3)\text{-}C(CH_3)=CH_2$, $-CH(CH_3)\text{-}CH_2\text{-}C(CH_3)=CH_2$, $-CH(CH_3)\text{-}CH(CH_3)\text{-}CH=CH_2$, $-CH_2\text{-}C(CH_3)_2\text{-}CH=CH_2$, $-C(CH_3)_2\text{-}CH_2\text{-}CH=CH_2$, $-CH_2\text{-}C(CH_3)=C(CH_3)_2$, $-CH(CH_3)\text{-}CH=C(CH_3)_2$, $-C(CH_3)_2\text{-}CH=CH\text{-}CH_3$, $-CH(CH_3)\text{-}C(CH_3)=CH\text{-}CH_3$, $-CH=C(CH_3)\text{-}CH(CH_3)_2$, $-C(CH_3)=CH\text{-}CH(CH_3)_2$, $-C(CH_3)=C(CH_3)\text{-}C_2H_5$, $-CH=CH\text{-}C(CH_3)_3$, $-C(CH_3)_2\text{-}C(CH_3)=CH_2$, $-CH(C_2H_5)\text{-}C(CH_3)=CH_2$, $-C(CH_3)(C_2H_5)\text{-}CH=CH_2$, $-CH(CH_3)\text{-}C(C_2H_5)=CH_2$, $-CH_2\text{-}C(C_3H_7)=CH_2$, $-CH_2\text{-}C(C_2H_5)=CH\text{-}CH_3$, $-CH(C_2H_5)\text{-}CH=CH\text{-}CH_3$, $-C(C_4H_9)=CH_2$, $-C(C_3H_7)=CH\text{-}CH_3$, $-C(C_2H_5)=CH\text{-}C_2H_5$, $-C(C_2H_5)=C(CH_3)_2$, $-C[C(CH_3)_3]=CH_2$, $-C[CH(CH_3)(C_2H_5)]=CH_2$, $-C[CH_2\text{-}CH(CH_3)_2]=CH_2$, $-C_2H_4\text{-}CH=CH\text{-}CH=CH_2$, $-CH_2\text{-}CH=CH\text{-}CH_2\text{-}CH=CH_2$, $-CH=CH\text{-}C_2H_4\text{-}CH=CH_2$, $-CH_2\text{-}CH=CH\text{-}CH=CH\text{-}CH_3$, $-CH=CH\text{-}CH_2\text{-}CH=CH\text{-}CH_3$, $-CH=CH\text{-}CH=CH\text{-}C_2H_5$, $-CH_2\text{-}CH=CH\text{-}C(CH_3)=CH_2$, $-CH_2\text{-}CH=C(CH_3)\text{-}CH=CH_2$, $-CH_2\text{-}C(CH_3)=CH\text{-}CH=CH_2$, $-CH(CH_3)\text{-}CH=CH\text{-}CH=CH_2$, $-CH=CH\text{-}CH_2\text{-}C(CH_3)=CH_2$, $-CH=CH\text{-}CH(CH_3)\text{-}CH=CH_2$, $-CH=C(CH_3)\text{-}CH_2\text{-}CH=CH_2$, $-C(CH_3)=CH\text{-}CH_2\text{-}CH=CH_2$, $-CH=CH\text{-}CH=C(CH_3)_2$, $-CH=CH\text{-}C(CH_3)=CH\text{-}CH_3$, $-CH=C(CH_3)\text{-}CH=CH\text{-}CH_3$, $-C(CH_3)=CH\text{-}CH=CH\text{-}CH_3$, $-CH=C(CH_3)\text{-}C(CH_3)=CH_2$, $-C(CH_3)=CH\text{-}C(CH_3)=CH_2$, $-C(CH_3)=C(CH_3)\text{-}CH=CH_2$, $-CH=CH\text{-}CH=CH\text{-}CH=CH_2$, $-C\equiv CH$, $-C\equiv C\text{-}CH_3$, $-CH_2\text{-}C\equiv CH$, $-C_2H_4\text{-}C\equiv CH$, $-CH_2\text{-}C\equiv C\text{-}CH_3$, $-C\equiv C\text{-}C_2H_5$, $-C_3H_6\text{-}C\equiv CH$, $-C_2H_4\text{-}C\equiv C\text{-}CH_3$, $-CH_2\text{-}C\equiv C\text{-}C_2H_5$, $-C\equiv C\text{-}C_3H_7$,

$-CH(CH_3)-C\equiv CH$, $-CH_2-CH(CH_3)-C\equiv CH$, $-CH(CH_3)-CH_2-C\equiv CH$, $-CH(CH_3)-C\equiv C-CH_3$, $-C_4H_8-C\equiv CH$, $-C_3H_6-C\equiv C-CH_3$, $-C_2H_4-C\equiv C-C_2H_5$, $-CH_2-C\equiv C-C_3H_7$, $-C\equiv C-C_4H_9$, $-C_2H_4-CH(CH_3)-C\equiv CH$, $-CH_2-CH(CH_3)-CH_2-C\equiv CH$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-C\equiv C-C(CH_3)_3$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C\equiv C-CH(CH_3)-C\equiv CH$, $-CH(CH_3)-C\equiv C-C\equiv CH$, $-CH(C\equiv CH)-CH_2-C\equiv CH$, $-C(C\equiv CH)_2-CH_3$, $-CH_2-CH(C\equiv CH)_2$, $-CH(C\equiv CH)-C\equiv C-CH_3$;
où deux substituants adjacents $R^3$ et $R^4$ ou $R^4$ et $R^5$ ou $R^5$ et $R^6$ ou $R^6$ et $R^7$ sont connectés par un groupe -CH=CH-CH=CH pour constituer respectivement un cycle aromatique ;
ainsi que leurs complexes métalliques, sels, énantiomères, mélanges d'énantiomères, diastéréomères, mélanges de diastéréomères, tautomères, hydrates, solvates, racémiques des composés susmentionnés.

**2.** Composés conformes à la revendication 1, où $R^3$ représente -OH, $-OCH_3$, $-OC_2H_5$, $-OC_3H_7$, $-O-cyclo-C_3H_5$, $-OCH(CH_3)_2$, $-OC(CH_3)_3$, $-OC_4H_9$, -OPh, $-OCH_2-Ph$, $-NH_2$, $-NHCH_3$, $-NHC_2H_5$, $-NHC_3H_7$, $-NH-cyclo-C_3H_5$, $-NHCH(CH_3)_2$, $-NHC(CH_3)_3$, $-N(CH_3)_2$, $-N(C_2H_5)_2$, $-N(C_3H_7)_2$, $-N(cyclo-C_3H_5)_2$, $-N[CH(CH_3)_2]_2$ ou $-N[C(CH_3)_3]_2$.

**3.** Composés conformes à la revendication 1 :

17a-[(2-hydroxynaphth-1-yl)-méthylamino]-3-méthoxy-estra-1,3,5(10)-triène,
17β-[(3-hydroxynaphth-2-yl)-méthylamino]-3-méthoxy-estra-1,3,5(10)-triène,
17β-[(2-hydroxynaphth-1-yl)-méthylamino]-3-méthoxy-estra-1,3,5(10)-triène hydroperchlorate,
et
17α-[(3-hydroxy-naphth-2-yl)-méthylamino]-3-méthoxy-estra-1,3,5(10)-triène.

**4.** Composés conformes à l'une des revendications ci-dessus, destinés au domaine médical.

**5.** Composés conformes à l'une des revendications ci-dessus, destinés au traitement et/ou à la prophylaxie d'infections parasitiques et de maladies.

**6.** Utilisation conforme à la revendication 5, où les infections parasitiques ou maladies font partie d'un groupe constitué de : trypanosomiase, maladie de Chagas, échinococcose, amibiase, ankylostomiase, babésiose, balantidiose, infection par blastocyste, schistosomiase, bilharziose, coccidiose, cryptosporidiose, infection à dientamoeba, microsporidiose, zoonose, giardiase, leishmaniose, infection par *Naegleria,* paludisme, sarcosporidiose, piroplasmose, trichinose, toxoplasmose, trichomonase, ascaridiase, filariose, taeniasis, onchocercose, capillariose, éléphantiasis, oxyurose, strongyloïdose, trichocéphalose et cysticercose.

**7.** Composition pharmaceutique comprenant au moins un composé défini dans la revendication 1 et au moins un porteur, excipient et/ou solvant acceptable en pharmacologie.

**8.** Composition pharmaceutique conforme à la revendication 7 sous forme de gouttelettes, de spray oral, de spray nasal, de comprimés, de pilules, de gélules, de comprimés sous film, de comprimés enduits, de produits à gargariser, de gels, d'onguents, de sirops, de poudres à inhaler, de granulés, de suppositoires, d'émulsions, de dispersions, de microcapsules, de capsules, de poudres ou de solutions à injecter.

**9.** Composition pharmaceutique conforme à la revendication 7 ou 8, adaptée à l'inhalation ou à une administration par voie intranerveuse, intrapéritonéale, intramusculaire, sous-cutanée, cutanéo-muqueuse, orale, rectale, transdermale, topique, buccale, intradermale, intragastrale, intracutanée, intranasale, intrabuccale, percutanée ou sublinguale.

**10.** Composition pharmaceutique conforme à la revendication 7, 8 ou 9 comprenant également un principe actif antiparasite, antibactérien, antifongique et/ou antiviral.

**11.** Composition pharmaceutique conforme à la revendication 10, où le principe actif antiparasite est l'artémisinine ou l'artésunate.

# FIGUREN

Figur 1

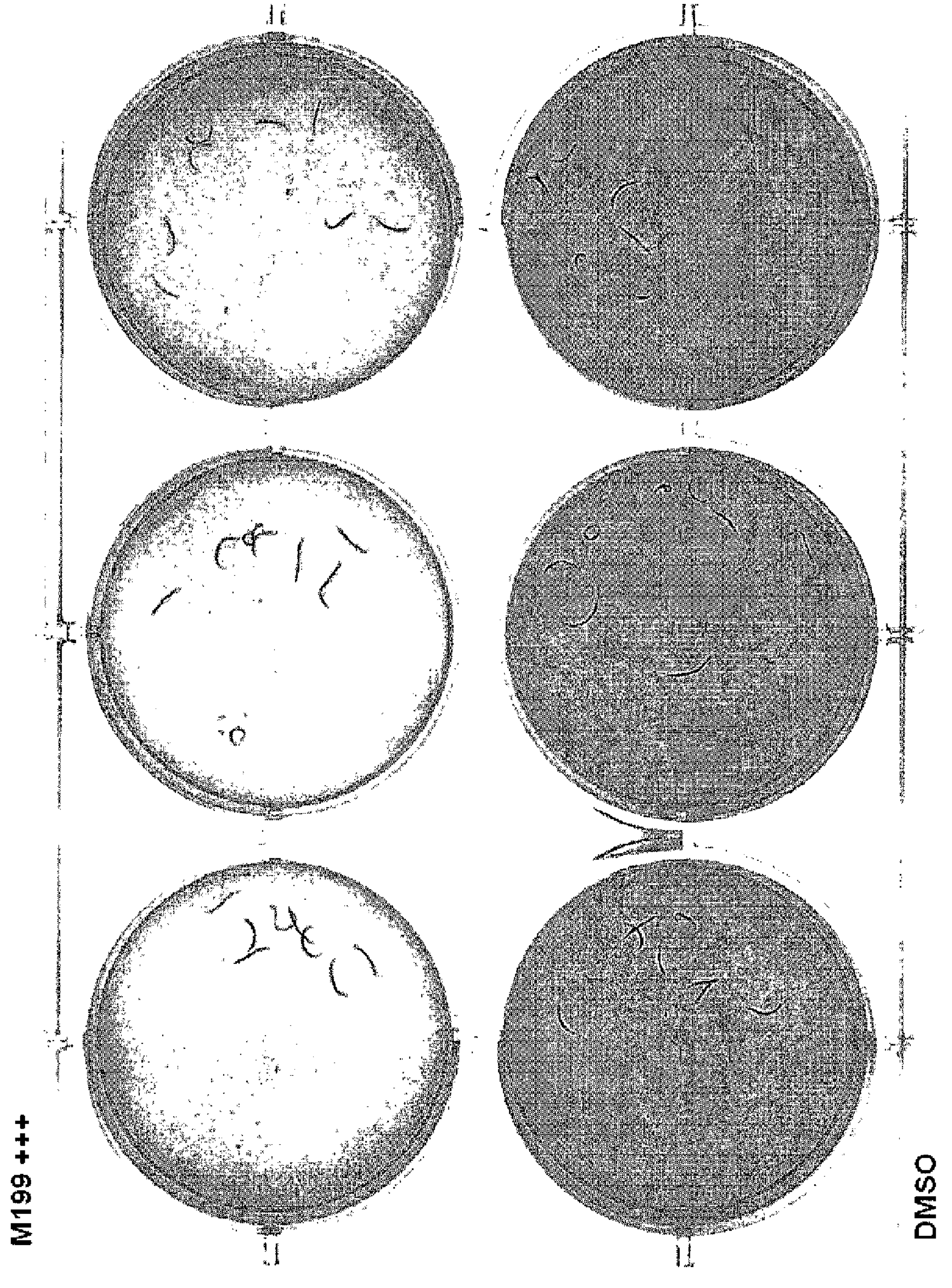

Figur 2

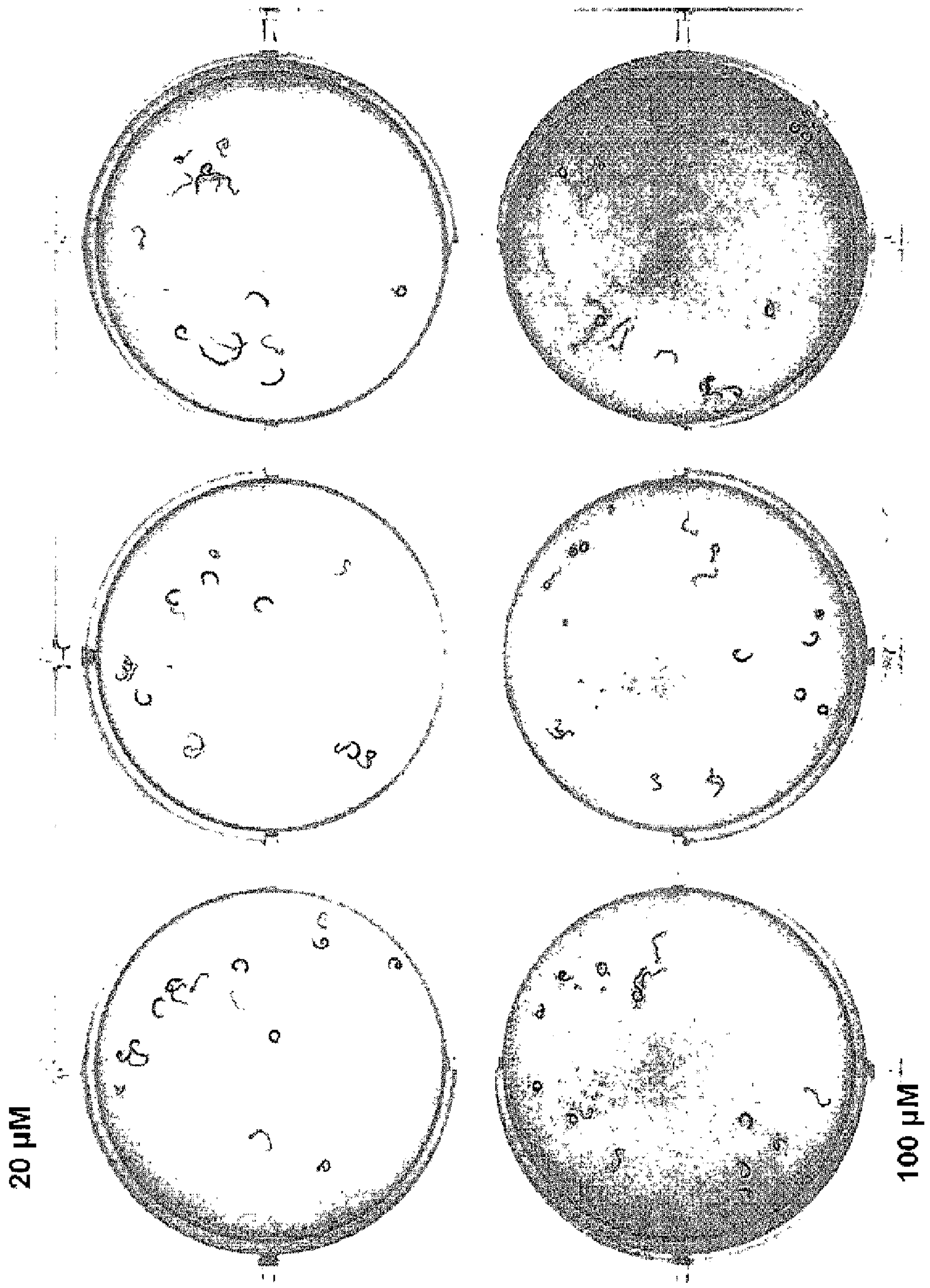

Figur 3

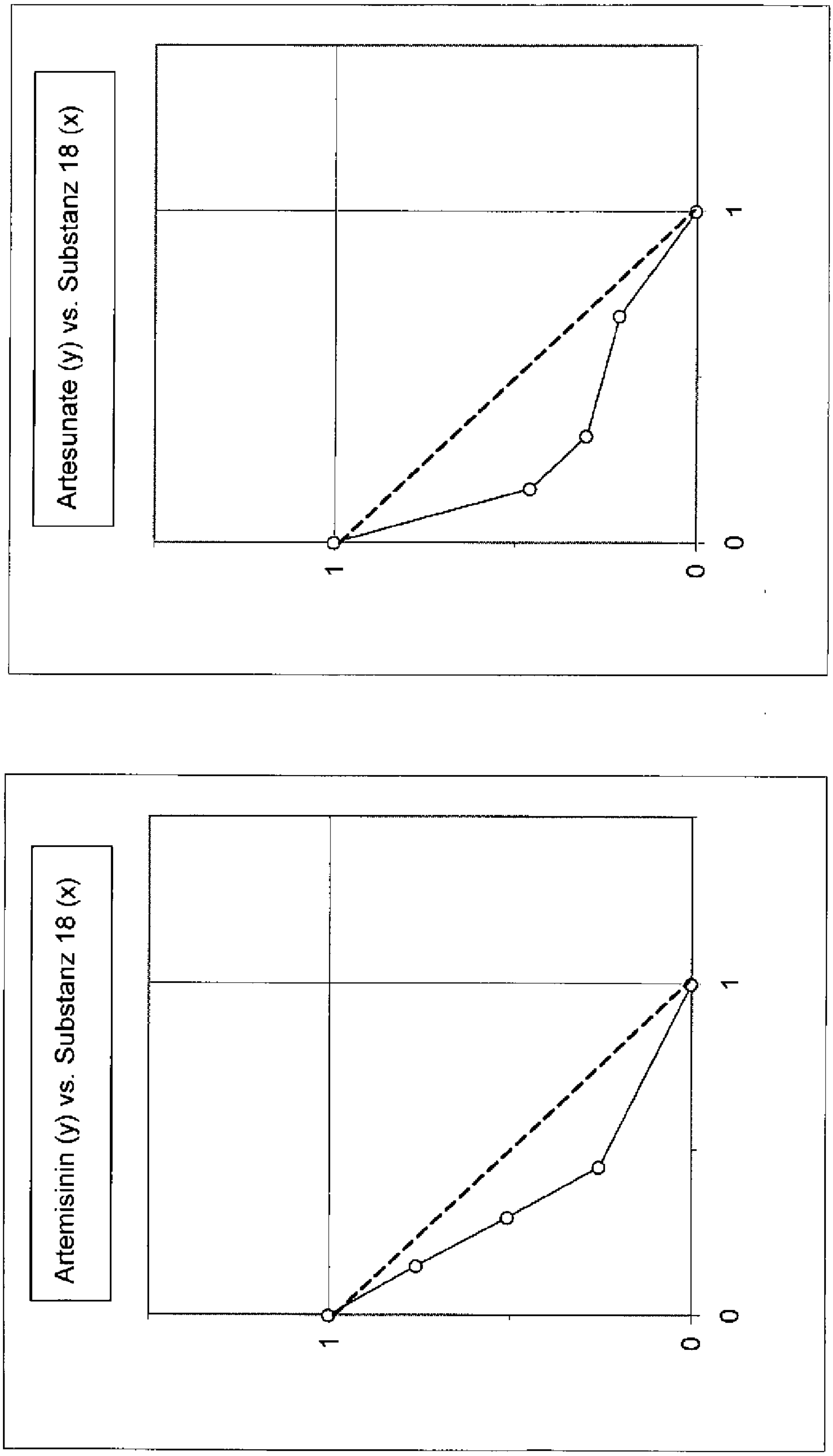
Artesunate (y) vs. Substanz 18 (x)
1
0
0
1
Artemisinin (y) vs. Substanz 18 (x)
1
0
0
1

Figur 4

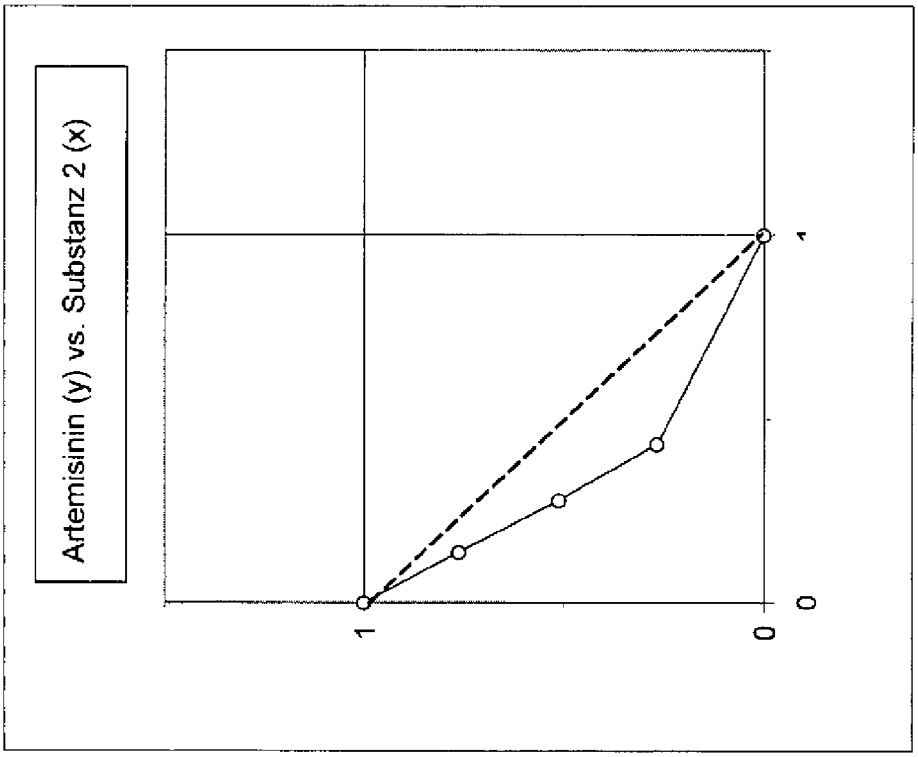
Artemisinin (y) vs. Substanz 2 (x)
1
0
0
1

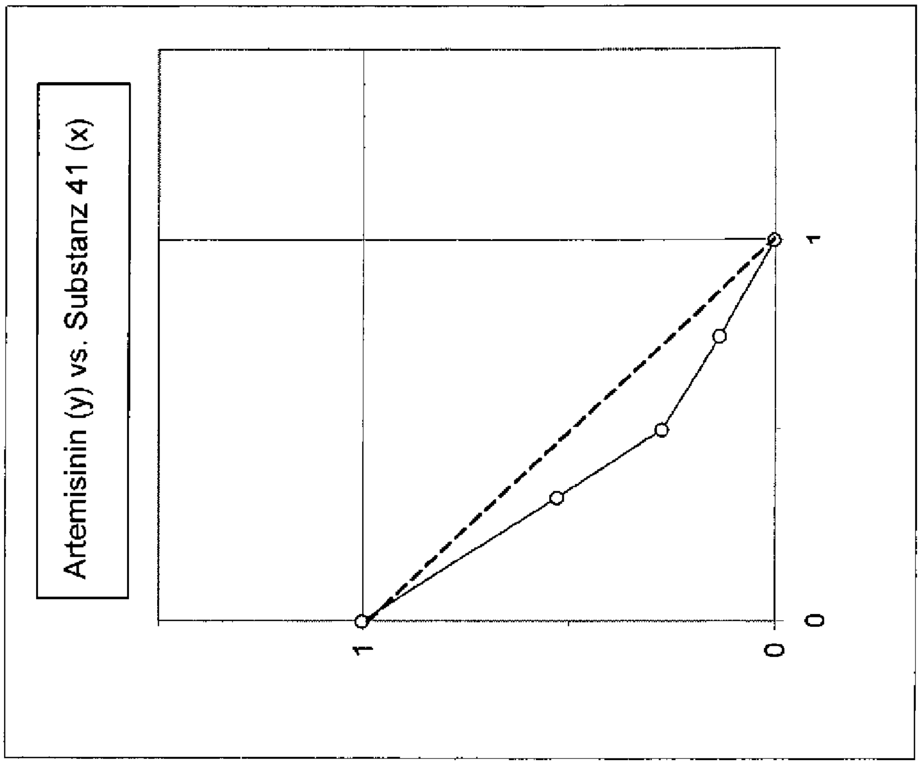
Artemisinin (y) vs. Substanz 41 (x)
1
0
0
1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19633206

### In der Beschreibung aufgeführte Nicht-Patentliteratur


- **AKOACHERE et al.** In Vitro Assessment of Methylene Blue on Chloroquine-Sensitive and-Resistant Plasmodium falciparum Strains Reveals Synergistic Action with Artemisinins. *Antimicrobial Agents and Chemotherapy,* 2005, vol. 49, 4592-4597
- **PETERS ; ROBINSON.** Handbook of Animal Models in Infection. Academic Press, 1999
- **STURM et al.** *Compounds Structurally Related to Ellagic Acid Show Improved Antiplasmodial Activity, Antimicrobial Agents and Chemotherapy,* 2009, vol. 53, 622-630
- **AKOACHERE M ; BUCHHOLZ K ; FISCHER E ; BURHENNE J ; HAEFELI WE ; SCHIRMER RH ; BECKER K.** In vitro assessment of methylene blue on chloroquine-sensitive and -resistant Plasmodium falciparum strains reveals synergistic action with artemisinins. *Antimicrob Agents Chemother,* 2005, vol. 49, 4592-4597
- **PROF. DR. GREVELDING.** Parasitologie. Universität Gießen